# EUROPEAN PATENT APPLICATION

(11) **EP 1 637 068 A1**
(43) Date of publication of application: **22.03.2006**
(21) Application number: 04746414.4
(22) Date of filing: 18.06.2004
(51) Int. Cl.: A61B 5/00, A61B 5/11, A47K 13/30, A47K 13/24

(54) **VIBRATION DETECTING APPARATUS AND TOILET SEAT APPARATUS**

(30) Priority: 20.06.2003 JP 2003176677; 06.10.2003 JP 2003346815; 06.10.2003 JP 2003346816; 07.10.2003 JP 2003348200
(71) Applicant: MATSUSHITA ELECTRIC INDUSTRIAL CO., LTD., Kadoma-shi, Osaka 571-8501 (JP)
(72) Inventor: UEDA, Shigeki, . (JP); OGINO, Hiroyuki, . (JP); YOSHINO, Koji, . (JP); MATSUDA, Masato, . (JP)
(74) Representative: Grünecker, Kinkeldey, Stockmair & Schwanhäusser Anwaltssozietät
(86) International application number: PCT/JP2004/008945
(87) International publication number: WO 2004/112600

(57) **Abstract**

A problem of the invention is to provide a vibration detecting apparatus for accurately detecting vibration transmitted to a rigid body, particularly provide a toilet seat apparatus for accurately detecting body movement of a user transmitted to a toilet seat.

There are provided pressing means (amplifying means) (27) for amplifying vibration by body movement of a user transmitted to an upper lid (rigid body) (6) of a toilet seat apparatus (5), and a piezoelectric sensor (vibration detecting sensor) (9) for detecting the amplified vibration and therefore, even when the toilet seat is hardly deformed by the body movement of the user transmitted to the toilet seat, the body movement of the user transmitted to the toilet seat is detected by the piezoelectric sensor (vibration detecting sensor) (9) by amplifying the body movement by the pressing means (amplifying means) (27) and therefore, the body movement of the user transmitted to the toilet seat can accurately be detected.

## Description

### <Technical Field>

The present invention relates to a toilet seat apparatus capable of detecting a heart rate or the like by including a pressure sensitive sensor, particularly relates to a technique of mounting a piezoelectric sensor in a cord-like shape.

Further, the invention relates to a vibration detecting apparatus for detecting vibration transmitted to a rigid body, particularly relates to an apparatus for accurately detecting body movement of heart beat or the like of a user by mounting a vibration detecting sensor of a piezoelectric sensor or the like having a flexibility to a rigid body of a toilet seat or the like and amplifying to detect vibration transmitted to the rigid body.

Further, the invention relates to a vibration detecting apparatus for detecting a plurality of pieces of information from vibration transmitted to a rigid body by a piezoelectric sensor having a flexibility, particularly relates to an apparatus mounted to a rigid body of a toilet seat or the like for accurately detecting biologic information or motion information of a user.

### <Background Art>

In a background art, there is a toilet seat apparatus for detecting presence/absence of the human body and even a heart rate thereof by attaching a pressure sensitive sensor to a toilet seat (refer to, for example Patent Reference 1).

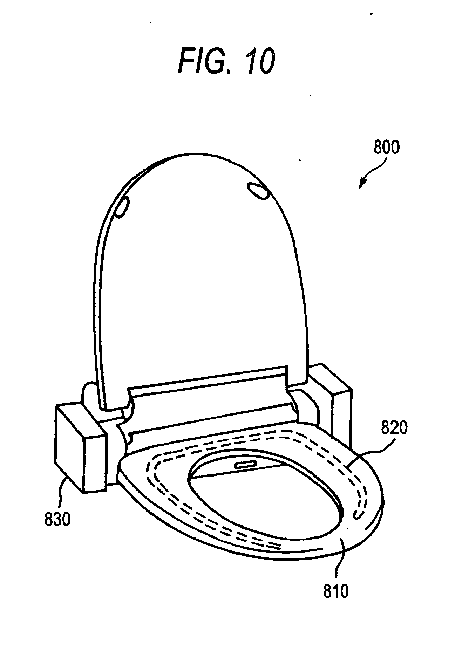

Fig.10 shows a toilet seat apparatus described in Patent Reference 1, mentioned above.

According to the toilet seat apparatus 800, as shown by the drawing, when the human body is seated, by body movement of the human body, a piezoelectric sensor 820 in a cord-like shape which is a pressure sensitive sensor included in a toilet seat 810 is deformed, and when a signal in accordance with the deformation is generated, a signal processing unit 830 processes an output signal thereof to thereby detect presence/absence, a heart rate or the like of the human body.

(Patent Reference 1) JP-A-05-091955

According to the toilet seat apparatus 800, the piezoelectric sensor 820 is contained in the toilet seat 810 which is a hard resin mold member and is difficult to receive deformation/vibration by the toilet seat 810 and therefore, an output from the piezoelectric sensor 820 is extremely small and it is difficult to provide an output having a desired signal intensity.

The invention has been carried out in view of the above-described situation and it is an object thereof to provide a toilet seat apparatus capable of firmly providing a sensor output to thereby promote detection of a heart rate or the like.

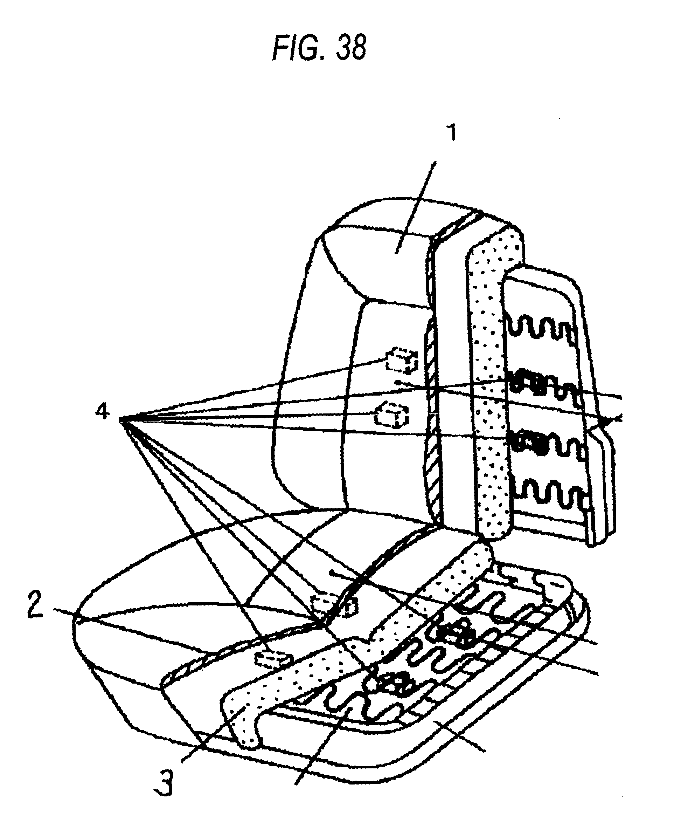

Further, as a representative vibration detecting apparatus of a background art, there is a human body detection apparatus mounting a piezoelectric sensor at a seat as a vibration detecting sensor (refer to, for example, Patent Reference 2). Fig.38 shows a human body detection apparatus described in Patent Reference 2, mentioned above, a piezoelectric sensor 4 is mounted below a skin 2, urethane foam 3 in a seat 1, body movement of the human body seated on the seat 1 vibrates the skin 2 and urethane foam 3 which are extremely highly elastic while partially deforming the skin 2 and urethane foam 3, as a result, also the piezoelectric sensor can be deformed. Based on an output generated by the piezoelectric sensor in accordance with the deformation, presence/absence, a heart rate or the like of a user is detected.

(Patent Reference 2) JP-A-08-282358

The above-described background art constitution can be adopted easily for a seat having a cushion such as a seat of a vehicle or the like. On the other hand, in the case of a rigid body which is not provided with a cushioning performance (for example, toilet seat or the like), even when a person is seated thereon, the rigid body is not deformed in compliance with a body shape of the person, partial deformation by body movement is hardly present and therefore, a piezoelectric sensor cannot be deformed and vibration cannot be detected as accurately as being able to detect presence/absence or heart beat of a user.

Further, the above-described background art constitution can easily be adopted for a seat having a cushion such as a seat of a vehicle or the like. On the other hand, in the case of a rigid body which is not provided with cushioning performance (for example, toilet seat or the like), even when a person is seated thereon, the rigid body is not deformed in compliance with a body shape of the person, partial deformation by body movement is hardly present and therefore, a piezoelectric sensor cannot be deformed and a large output is difficult to be provided. Further, in the case of a rigid body, vibration of body movement or the like is propagated to a total of the rigid body and therefore, also a sensor mounted to the rigid body is similarly vibrated, and vibration of the rigid body relative to the sensor cannot be provided. Although in order to detect vibration, vibration needs to be provided to a sensor inherently maintained in an unvibrated state, the sensor cannot be brought into the unvibrated state. Therefore, it is difficult to detect vibration as accurate as being able to detect presence/absence or heart beat of a user.

The invention is for resolving the problems and it is an object thereof to provide a vibration detecting apparatus for accurately detecting vibration transmitted to a rigid body.

Further, the invention is for resolving the problem and it is an object thereof to provide a vibration detecting apparatus for accurately detecting vibration transmitted to a rigid body by bringing at least a portion of a vibration detecting sensor to be proximate to an unvibrated state.

Further, it is an object thereof to provide a toilet seat apparatus for accurately detecting body movement of a user transmitted to a toilet seat.

Further, according to a representative vibration detecting apparatus of a background art, there is a toilet seat apparatus for determining motion information based on a motion in which a person stands up or is seated (presence/absence detection) and biologic information (heart rate) by a magnitude of an output of a piezoelectric sensor mounted to a toilet seat (refer to Patent Reference 3). According thereto, there is constructed a constitution commonly filtering and amplifying particularly an output of a piezoelectric sensor and separately having operating means only for determining a heart rate.

(Patent Reference 3) Japanese Patent Publication No.2734832

According to the above-described background art constitution, although it is only described that a plurality of pieces of information of motion information, biologic information and the like can be determined from body movement of a person by a piezoelectric sensor, there is not specifically described with regard to a constitution of determining means necessary for determining a plurality of pieces of information from the single piezeoelectric sensor and the respective pieces of information cannot be outputted accurately and efficiently.

The invention is for resolving the problems and it is an object thereof to provide a vibration detecting apparatus capable of determining motion information and biologic information accurately and efficiently from vibration detected by a piezoelectric sensor.

Further, it is an object thereof to provide a toilet seat apparatus capable of determining motion information and biologic information accurately and efficiently from body movement of a user transmitted to a toilet seat.

### <Disclosure of the Invention>

In order to resolve the problem of the background art, a vibration detecting apparatus of the invention is constructed by a constitution including first amplifying means for amplifying a vibration transmitted a rigid body and a vibration detecting sensor for detecting the amplified vibration.

Further, the vibration detecting apparatus of the invention is constructed by a constitution arranging a vibration detecting sensor to a case comprising an upper lid and a base plate, the upper lid comprising a rigid body, and including amplifying means for amplifying the vibration transmitted to the rigid body and the vibration detecting sensor for detecting the amplified vibration.

Thereby, even when the rigid body is hardly deformed by the vibration transmitted to the rigid body, the vibration transmitted to the rigid body is amplified by the amplifying means, the amplified vibration is detected by the vibration detecting sensor and therefore, the vibration transmitted to the rigid body can accurately be detected.

Further, a toilet seat apparatus of the invention is constructed by a constitution in which the rigid body is an upper lid of a toilet seat and a body movement of a user transmitted to the toilet seat is detected by the vibration detecting apparatus.

Thereby, even when the toilet seat is hardly deformed by the body movement of the user transmitted to the toilet seat, the body movement transmitted to the toilet is amplified by the amplifying means, the amplified vibration is detected by the vibration detecting sensor and therefore, the body movement of the user transmitted to the toilet seat can accurately be detected.

Further, in order to resolve the problem of the background art, the vibration detecting apparatus of the invention is constructed by a constitution of supporting the vibration detecting sensor for detecting the vibration transmitted to the rigid body at a vicinity of a fixing portion for fixing the rigid body.

Further, the vibration detecting apparatus of the invention is constructed by a constitution of arranging the vibration detecting sensor in a case comprising the rigid body, providing a leg portion as a fixing portion at a bottom face of the case and supporting the vibration detecting sensor at a vicinity of the leg portion.

Thereby, the vibrating rigid body is difficult to be vibrated the most at a vicinity of the fixing portion and therefore, the vibration detecting sensor supported by the vicinity of the fixing portion can be prevented from being vibrated at at least a supported portion. Therefore, the vibration transmitted to the rigid body can accurately be detected in an environment in which the vibration detecting sensor per se is not vibrated.

Further, the toilet seat apparatus of the invention is constructed by a constitution constituting the rigid body by a toilet seat, constituting the fixing portion by a pad attached to a lower face of the toilet seat and capable of fixing the toilet seat by being brought into contact with an upper face of a toilet and detecting the body movement of the user transmitted to the toilet seat by the vibration detecting apparatus.

Thereby, the toilet seat is difficult to be vibrated the most by being fixed to the toilet at a vicinity of the pad and therefore, the vibration detecting sensor supported by the vicinity of the pad can be prevented from being vibrated at at least a supported portion. Therefore, the body movement of the user transmitted to the toilet seat can accurately be detected in an environment in which the vibration detecting sensor per se is not vibrated.

In order to resolve the problem of the background art, the vibration detecting apparatus of the invention is constructed by a constitution including a piezoelectric sensor having a flexibility for detecting a vibration and determining means for determining biologic information after determining motion information based on an output of the piezoelectric sensor.

Thereby, it is not necessary to await for the biologic information until determining the motion information and therefore, consumption of power necessary for awaiting for the biologic information can be prevented to achieve efficient formation, electric noise generated by useless current can be prevented similarly and accuracy of determination is promoted.

Further, the toilet seat apparatus of the invention is constructed by a constitution of detecting the body movement of the user transmitted to the toilet seat by the vibration detecting apparatus to determine motion information and biologic information.

Thereby, until determining motion information in which a user lifts up the lid or is seated on the toilet seat, it is not necessary to await for biologic information of the heart rate, respiration or the like of the seated user and therefore, the effect of efficient formation and promotion of accuracy is similarly achieved.

Further, in order to achieve the above-described object, the toilet seat apparatus according to the invention is characterized in a toilet seat apparatus arranging a vibration detecting sensor of the vibration detecting apparatus of the invention to a case comprising an upper lid and a base plate, wherein the vibration detecting sensor is a piezoelectric sensor in a cord-like shape.

According to the constitution, the piezoelectric sensor in the cord-like shape can generate a large output and is provided with a flexibility and difficult to be destructed even when impact continues applying thereto and outputs a detected signal for facilitating to differentiate a person and an article and therefore, detection of seating or the like can firmly be executed.

Further, the toilet seat apparatus according to the invention is characterized in that the piezoelectric sensor in the cord-like shape outputs an electric signal in accordance with acceleration of vibration when the vibration is applied thereto.

According to the constitution, the piezoelectric sensor in the cord-like shape outputs the electric signal in accordance with acceleration of vibration. Therefore, the piezoelectric sensor can easily detect even slight motion of the human body.

Further, the toilet seat apparatus according to the invention is characterized in that the piezoelectric sensor in the cord-like shape is attached to one of the upper lid and the base plate, further including pressing means for generating an output by being brought into contact with the piezoelectric sensor in the cord-like shape when the toilet seat apparatus is seated at the case.

According to the constitution, there is provided the pressing means brought into contact with the piezoelectric sensor in the cord-like shape by the seating of the human body or the like and therefore, the piezoelectric sensor can firmly output the electric signal by reacting with the slight vibration.

Further, the toilet seat apparatus according to the invention is characterized in that the pressing means is constituted by a projection projected from an inner face of the case to the piezoelectric sensor in the cord-like shape arranged in the case.

According to the constitution, by constituting the pressing means by the projection, a structure of operating the piezoelectric sensor can be simplified.

Further, the toilet seat apparatus according to the invention is characterized in that the projection is constituted by a pad for absorbing an impact attached to a lower face of the base plate and brought into elastic contact with an upper face of the toilet main body and the pad is provided to be able to be brought into contact with the piezoelectric sensor in the cord-like shape by penetrating a through hole of the base plate.

According to the constitution, the pad for absorbing an impact is applicable by only changing a shape of a background art product slightly and therefore, the pad can be produced without executing large capital investment.

Further, the toilet seat apparatus according to the invention is characterized in that the piezoelectric sensor in the cord-like shape is supported in a state of being separated from the inner face of the case and the projections are alternately arranged to the upper lid and the base plate along a cable longitudinal direction.

According to the constitution, the respective projections press the piezoelectric sensor alternately from an up and down direction and therefore, detection of presence/absence or the like of the human body can be ensured by increasing the output of the piezoelectric sensor.

Further, the toilet seat apparatus according to the invention is characterized in that an outer face of the upper lid is recessed with a peripheral groove, the pressing means is formed by an elastic body fitted into the peripheral groove and the piezoelectric sensor in the cord-like shape is arranged to be contained in an elastic member.

According to the constitution, when the elastic body is pressed, a deformation thereof is directly operated to the piezoelectric sensor to easily bend the piezoelectric sensor and therefore, the piezoelectric sensor can output the electric signal by detecting even a slight vibration.

Further, the toilet seat apparatus according to the invention is characterized in that the electric signal is used for controlling a temperature of hot water of cleaning means, a water pressure, a temperature of a heater in the toilet seat or detecting a heart rate or the like.

According to the invention, when there is constructed a constitution in which by utilizing the electric signal detected by the piezoelectric sensor in controlling a temperature controller for controlling the hot water temperature, a water pressure controller for controlling the water pressure, a heater controller for governing a heater temperature in the toilet seat and the like, operation of the apparatus is stopped when not used by using, for example, a timer or the like, power can be saved efficiently. Further, the toilet seat apparatus can contribute to health control when a heart rate or the like is detected based on the electric signal detected by the piezoelectric sensor.

Further, the toilet seat apparatus according to the invention is characterized in that the electric signal is used by being outputted to an outside monitor via communicating means.

According to the constitution, for example, the heart rate or the like can be detected via the outside monitor and therefore, even a behavior in a rest room which cannot be diagnosed directly in a hospital or the like can always be monitored.

### <Brief Description of the Drawings>

Fig. 1 is a disassembled perspective view of a toilet apparatus according to the invention.
Fig. 2 is a perspective view of an outlook of a piezoelectric sensor in a cord-like shape used in a toilet seat apparatus.
Fig.3 is a perspective view of a total of a toilet to which a toilet seat apparatus is applied.
Fig.4 is a sectional view of an essential portion of a toilet seat apparatus.
Fig. 5 is a side view of a state of using the toilet shown in Fig.3.
Fig.6 is a block constitution diagram of a control unit in a toilet seat apparatus.
Fig. 7 is a time chart of a sensor output and motion in a toilet seat apparatus.
Fig. 8 is a sectional view of an essential portion of a toilet seat apparatus according to a second embodiment of the invention.
Fig.9 is a partially broken perspective view of a toilet seat apparatus according to a third embodiment of the invention.
Fig.10 is a perspective view of an outlook of a toilet seat apparatus of a background art.
Figs.11 (a) and 11(b) illustrate sectional views of an essential portion of a toilet seat apparatus.
Figs.12(a) to 12(c) illustrate sectional views of an essential portion of other mode of a toilet seat apparatus.
Figs.13(a) to 13(c) illustrate sectional views of an essential portion of other mode of a toilet seat apparatus.
Figs.14(a) and 14(b) illustrate sectional views of an essential portion of other mode of a toilet seat apparatus.
Figs.15(a) to 15(c) illustrate constitution views of a section of a bath tub apparatus according to the invention.
Fig.16 is a sectional view of an essential portion of a bath tub apparatus.
Figs.17(a) and 17(b) illustrate sectional views of an essential portion of other mode of a bath tub apparatus.
Figs.18(a) and 18(c) illustrate constitution views of a section of a shower apparatus according to the invention.
Fig.19 is a sectional view of an essential portion of a shower apparatus.
Fig.20 is a sectional view of other essential portion of a shower apparatus.
Figs. 21 (a) to 21 (c) illustrate sectional views of an essential portion of other mode of a toilet seat apparatus.
Figs.22(a) to 22(c) illustrate sectional views of an essential portion of other mode of a toilet seat apparatus.
Figs.23(a) and 23(b) illustrates sectional views of an essential portion of other mode of a toilet seat apparatus.
Fig.24 is a sectional view of an essential portion of a bath tub apparatus.
Fig.25 is a sectional view of an essential portion of a shower apparatus.
Fig.26 is a sectional view of an essential portion of other mode of a shower apparatus.
Figs.27(a) and 27(b) illustrate constitution views of a section of an essential portion of a toilet seat apparatus according to the invention.
Figs.28(a) and 28(b) illustrate constitution views of a section of an essential portion of other mode of a toilet seat apparatus.
Fig.29 is a block constitution diagram of controlling means in a toilet seat apparatus.
Fig.30 is a characteristic diagram of a piezoelectric sensor in still time in a toilet seat apparatus.
Fig.31 is a characteristic diagram of a filter output in a toilet seat apparatus.
Fig.32 is a characteristic diagram of an autocorrelation coefficient in a toilet seat apparatus.
Fig.33 is a flowchart diagram for calculating a period of heart beat in a toilet seat apparatus.
Fig.34 is a block constitution diagram of controlling means in a bath tub apparatus.
Fig. 35 is a constitution view of a child seat according to the invention.
Fig.36 is a constitution view of a car seat according to the invention.
Fig.37 is a constitution view of bedclothes according to the invention.
Fig.38 is a constitution view of a section of a human body detection apparatus of a background art.

Further, in the drawings, notation 5 designates a toilet seat apparatus, notation 6 designates an upper lid (rigid body), notation 7 designates a base plate, notation 8 designates a case, notations 9, 45, 53 designate piezoelectric sensors (vibration detecting sensors), notations 27, 35, 36, 37, 39, 40, 41, 42, 54 designate pressing means (amplifying means), notations 28, 46, 49 designate projections (amplifying means), notations 34, 52 designate vibration detecting apparatus, notation 43 designates a bath tub (rigid body), notation 50 designates a seat (rigid body), notation 58 designates first pressing means (amplifying means), notation 59 designates second pressing means, notations 213, 251 designate controlling means, notations 229, 252 designate first determining means (determining means), notations 230, 253 designate second determining means (determining means), notations 232, 238, 255, 262 designate amplifying means, notations 236, 259, 266 designate power supplying means, notations 242, 270 designate displaying means, notations 244, 269 designate informing means, notation 245 designates a vibration detecting apparatus, notation 268 designates a hot water supply apparatus (water supplying and discharging means), notation 500 designates upper cloth, notation 501 designates a user, and notation 502 designates a buzzer.

### <Best Mode for Carrying Out the Invention>

Embodiment of the invention will be explained in details in reference to the drawings as follows.

### (First Embodiment)

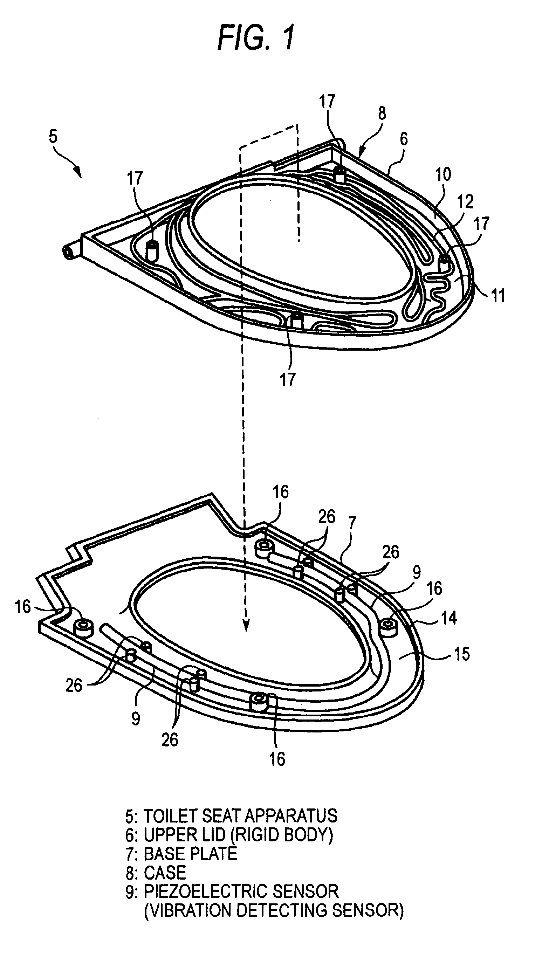

As shown by Fig.1, the toilet seat apparatus 5 according to an embodiment of the invention is constituted by arranging the piezoelectric sensor 9 in a cord-like shape constituting a pressure sensitive sensor to the base plate 7 of the case 8 comprising the upper lid 6 and the base plate 7 molded by resin.

The upper lid 6 forms an upper portion of the case 8 by including a main body portion 10 in a semicircular shape in a sectional view thereof. A lower face of a ceiling plate 11 of the main body portion 10 is attached with a heater 12 for heating. Further, the heater 12 is connected to a control unit 13, mentioned later, and is set to a desired temperature by manual operation.

The base plate 7 forms a lower portion of the case 8 by including a main body portion 14 in a channel-like shape in a sectional view thereof. An upper face of a bottom plate 15 of the main body portion 14 is attached with the piezoelectric sensor 9.

The upper lid 6 and the base plate 7 are integrally integrated by forming a space above the sensor 14 by fitting a screw, not illustrated, (not illustrated) from a through hole 16 formed at the base plate 7 to a locking portion 17 formed at the upper lid 6. Further, the piezoelectric sensor 9 is connected to the control unit 13 similar to the heater 12.

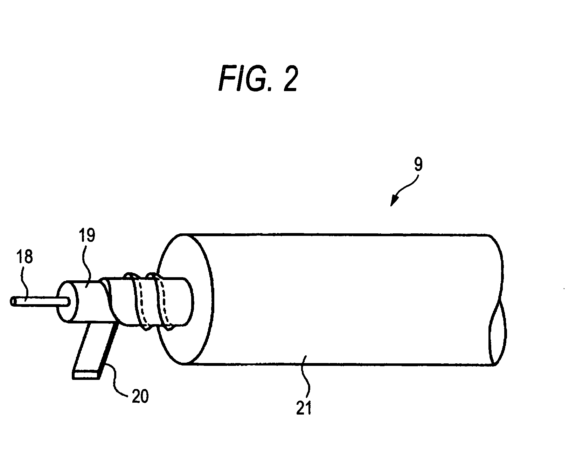

Here, simply describing the piezoelectric sensor 9 in the cord-like shape used in the embodiment, as shown by Fig.2, the sensor 14 is a sensor in a cable-like shape using a piezoelectric element material and is constituted by a core line (center electrode) 18 arranged at a center in an axial direction, a piezoelectric element material 19 covered at a surrounding of the core line 18, an outer side electrode 20 arranged at a surrounding of the piezoelectric element material 19, and PVC (polyvinyl chloride resin) 21 for covering an outermost periphery.

The piezoelectric sensor 9 uses the piezoelectric element material 19 having heat resistance capable of withstanding a surrounding temperature of about 120 °C, further, is provided with a flexibility comparable to that of a normal vinyl cord by using the piezoelectric element material 19 constituted by a resin having a flexibility and a piezoelectric ceramic and the flexible electrodes. Further, the piezoelectric sensor 9 is provided with a high sensitivity comparable to that of a polymer piezoelectric element and achieves a particularly sensitivity in a low frequency region for detecting a heart rate of the human body (10 Hz or lower). This is because a reduction in the sensitivity is small even in the low frequency region since a specific inductive capacity (about 55) of the piezoelectric element material 19 is larger than a specific inductive capacity (about 10) of the polymer piezoelectric element material.

The piezoelectric sensor 9 in the cord-like shape provided in this way as molded with the piezoelectric element material 19 is not provided with a piezoelectric function and therefore, it is necessary to carry out a processing for providing the piezoelectric function (polarizing processing) for the piezoelectric element material 19 by applying a direct current high voltage of several KV/mm to the piezoelectric element material 19. The polarizing processing is carried out by applying a direct current voltage between the two electrodes 18, 20 after forming the core line 18 and the outer side electrode 20 to the piezoelectric electric element material 19.

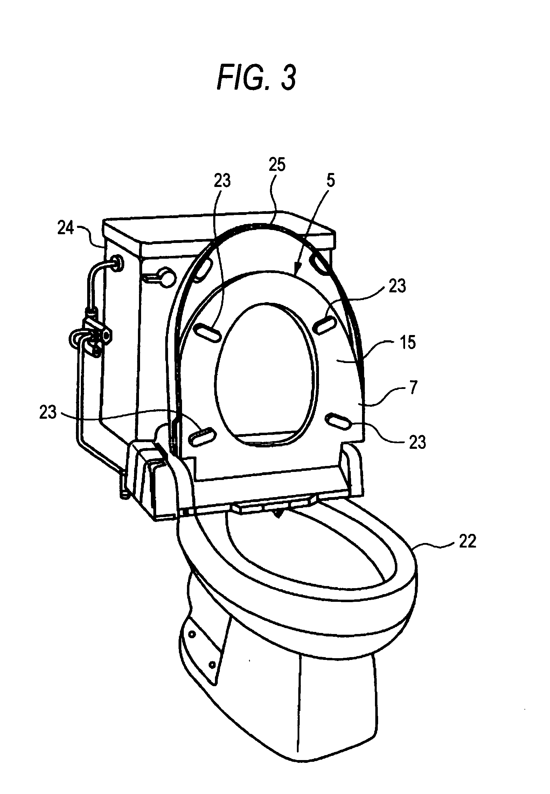

As shown by Fig.3, the base plate 7 is mounted with 4 pieces of pads 23 for absorbing impact attached to a rear face of the bottom plate 15 and having an elastic force for absorbing impact between the base plate 7 and a toilet main body 22 by being disposed between the toilet seat apparatus 5 and the toilet main body 22 when the toilet seat is used similar to the background art apparatus. Further, the toilet seat apparatus 5 is mounted with a lid member 25 lifted up to a side of a water tank 24 along with the toilet seat apparatus 5.

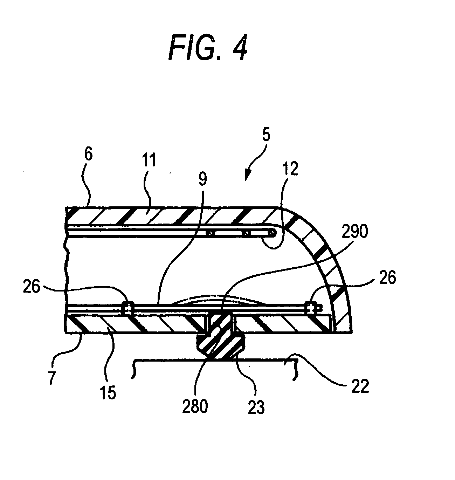

According to the embodiment, the piezoelectric sensor 9 in the cord-like shape is fixedly arranged on the base plate 7 by being supported by a plurality of holders 26 arranged separately from each other. As shown by Fig.4, the pad 23 is provided with a projected portion 290 penetrating a through hole 280 formed at the base plate 7 to be able to be brought into contact with the piezoelectric sensor 9 between the holders 26 to constitute pressing means for deforming the piezoelectric sensor 9 in the cord-like shape. Further, an inner face of the upper lid 12 is fixedly arranged with the heater 12 as mentioned above.

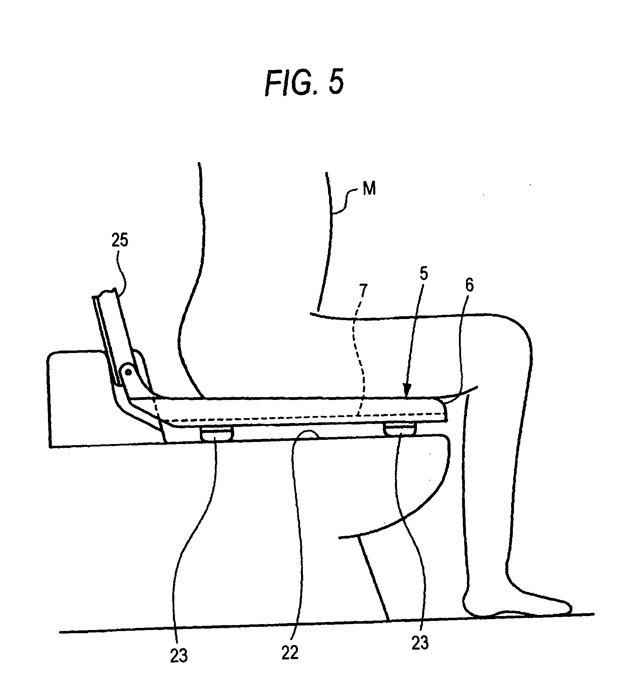

As shown by Fig. 5, in using the toilet seat apparatus 5 having the above-described constitution, when the toilet seat apparatus 5 is arranged above the toilet main body 22 and is applied with a weight of the human body M seated thereon, simultaneously with compressing the pad 23 by receiving a pressing force from the toile main body 22, the projected portion 290 is projected upward from the through hole 280 and presses the piezoelectric sensor 9 as indicated by a two-dotted chain line in Fig.4. As a result, in comparison with the background art apparatus which is not provided with pressing means, the piezoelectric sensor 14 is applied with vibration in accordance with movement of the human body M via the pad 23 and firmly outputs an electric signal.

The electric signal provided in accordance with acceleration of vibration is supplied to the control unit 13.

However, according to the toilet seat apparatus 5, the electric signal outputted from the piezoelectric sensor 9 is masked by the control unit 13 such that vibration generated by driving a cleaning nozzle, operating a blower, or flushing water or the like in using the toilet seat apparatus 5 does not constitute a noise for detecting presence/absence, a heart rate or the like of the human body.

In this way, the piezoelectric sensor 9 generates the electric signal by pressing the pad 23 and therefore, the piezoelectric sensor 9 can generate a signal larger than that of the background art apparatus for detecting deformation of the toilet seat apparatus per se.

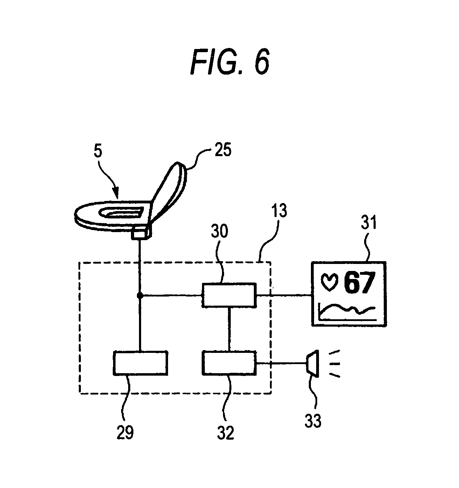

As shown by Fig.6, the control unit 13 is provided whit a filter circuit, amplifying means, smoothing means, determining means and the like, not illustrated, in a control portion 29 and is mounted with heart rate calculating means 30 for calculating a heart rate based on an output signal of the smoothing means, displaying means 31 for displaying an output signal of the heart rate calculating means 30, comparing means 32 for comparing a calculated output of the heart rate calculating means 30 and a set value, and alarm issuing means 33 for issuing an alarm based on a result of the comparison and is inputted with a detected signal of the piezoelectric sensor 9.

When the piezoelectric sensor 9 detects body movement of the human body M and outputs an electric signal, the control unit 13 filters the electric signal by the filter circuit, thereafter, amplifies the electric signal by the amplifying means and smoothes the electric signal by smoothing means.

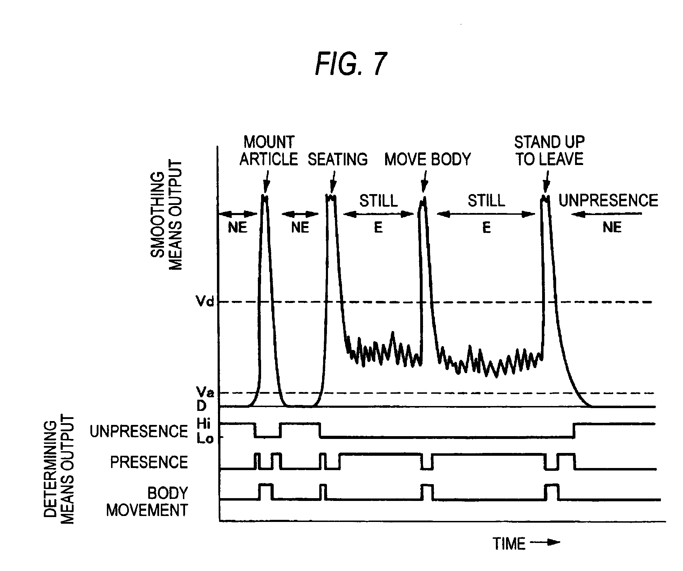

As shown by Fig. 7, a large output waveform is outputted from the smoothing means at an instance at which the human body M is seated on the toilet seat apparatus 5, when an article is mounted thereon, or the body is moved. On the other hand, when the human body M is seated thereon to be brought into a still state thereafter, an output waveform of a comparatively low level is outputted and the smoothing means by small body movement of the body propagated by action of the heart and respiratory action. In contrast thereto, when the human body M is not present, or when an article is mounted thereon, an output waveform is not shown in a constant period of time by the smoothing means after outputting a large output waveform.

Hence, the determining means compares an output V of the smoothing means and previously determined two set values Va, Vb to determine. That is, when V < Va, it is determined that the human body M or an article is not present (unpresent output H:). When Va ≦ V < Vb, it is determined that the human body M is present in a still state (present output H:). Further, when Vb < V, it is determined that the human body M generates body movement (body movement output H:). When an article is mounted thereon in place of the human body M, it is temporarily determined that the body is seated thereon or the body is moved, the human body M is determined not to be present as a state of placing the article thereon since vibration of a low level propagated by action of the heart or the respiratory action as in the human body M does not appear.

When the determining means determines that a person is seated thereon, deodorizing means and heating means start to be operated. The operation is stopped when a person is determined not to be present. Further, the heating means governs a temperature control of the heater 12.

Further, a small signal propagated by action of the heart is outputted from the smoothing means when the human body M is seated thereon in the still state. Based on the signal, the heart rate calculating means 30 calculates to output a heart rate. A result of the calculation is displayed by the displaying means 31 which is an outside monitor. As shown by Fig.6, the control unit 13 is mounted with the comparing means 32 for comparing the output signal of the heart rate calculating means 30 and the previously determining set value and the alarm issuing means 33 for issuing the alarm by the output of the comparing means 32 and can issue the alarm when the heart rate becomes equal to or larger than the set value. Particularly, when a person strains in evacuation, the heart rate is increased and there is a concern of bringing about occurrence of the cerebral hemorrhage, however, the control unit 13 can contribute to health control by foreseeing the attack. At this occasion, when the control unit 13 is connected by a network, in, for example, a hospital or the like, not only the toile seat apparatus used in the hospital can centrally be monitored summarizingly but also a behavior in a rest room which cannot be diagnosed directly can always be monitored.

Further, the signal to the displaying means 31 can be transmitted via communicating means by wired means or wireless means.

According to the above-described toilet seat apparatus 5, the piezoelectric sensor 9 can achieve high reliability by easily detecting even slight motion of the human body by firmly supplying the electric signal in accordance with acceleration of vibration to the control unit 13. Further, the piezoelectric sensor 9 is provided with a flexibility and is difficult to be destructed even when impact continues applying thereto, further, the electric signal easily differentiating a person and an article is outputted and therefore, detection of seating or the like can be ensured.

Further, according to the toilet seat apparatus 5, the projected portion 290 is provided to be able to be brought into contact with the piezoelectric sensor 9 by being projected from the bottom plate 15 of the base plate 7 and therefore, the piezoelectric electric sensor 9 can firmly output the electric signal in accordance with the acceleration of vibration reacting with even small vibration from the human body.

Further, the pad 23 for absorbing impact can be applied also as pressing means by slightly changing a shape of a background art product and therefore, the pads can be produced without carrying out large capital investment.

Further, although according to the above-described embodiment, presence/absence, the heart rate or the like is determined by smoothing the output signal from the piezoelectric sensor 9, there may be constructed a constitution in which the output signal from the piezoelectric sensor 9 is amplified as necessary, thereafter, converted into digital data by AD conversion by a microcomputer or the like, presence/absence is determined based on a value constituted by subjecting the digital data to a moving average in a microcomputer, or the heart rate or the like is calculated by calculating an autocorrelation coefficient of the digital data.

### (Second Embodiment)

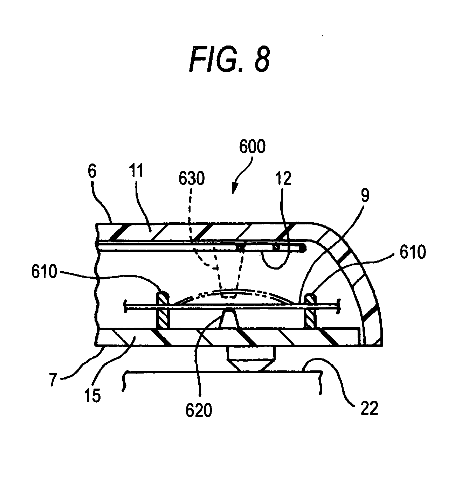

Next, a second embodiment of the toilet seat apparatus according to the invention will be explained in details in reference to Fig.8 as follows.

Fig.8 is a sectional view of an essential portion showing the second embodiment of the toilet seat apparatus according to the invention. Further, members having constitution and operation similar to those of respective embodiments of the second embodiment and thereafter which have already been explained are attached with the same notations or corresponding notations to thereby simplify or omit the explanation.

According to a toilet seat apparatus 600 of the embodiment, as shown by Fig. 8, the piezoelectric sensor 9 in the cord-like shape is supported by a holder 610 in a state of being more or less separated upward from the bottom plate 15 of the base plate 7 and a projection 620 constituting pressing means is projected to be able to be brought into contact with the piezoelectric sensor 9 above the bottom plate 15 of the base plate 7.

Also in the above-described constitution, according to the toilet seat apparatus 600, when the human body M is seated thereon, the base plate 7 is bent to a side of the upper lid 6 by receiving a pressing force from the toilet main body 22 and simultaneously therewith, the projection 620 is brought into contact with the piezoelectric sensor 9 to deform the piezoelectric sensor as indicated by a two-dotted chain line in Fig.8.

In this case, as indicated by a broken line in Fig.8, a projection 630 constituting pressing means can be arranged at the ceiling plate 11 of the upper lid 6 in place of the base plate 13. As a result, when the human body M is seated on the upper lid 6, the projection 630 pressed down to a side of the base plate 7 by more or less deforming the upper lid 6 is strongly brought into contact with the piezoelectric sensor 9 approaching the side of the upper lid 6 and the electric signal is further firmly outputted from the piezoelectric sensor 9.

Further, although according to the above-described embodiment, the projection 620, 630 is arranged at the upper lid 6 or the base plate 7, by constructing a constitution of alternately arranging the respective projections 620, 630 to respectives of the upper lid 6 and the base plate 7 along a longitudinal direction of the piezoelectric sensor 9 in the cord-like shape, the output of the piezoelectric sensor 9 can further be ensured by increasing deformation of the piezoelectric sensor 9.

### (Third Embodiment)

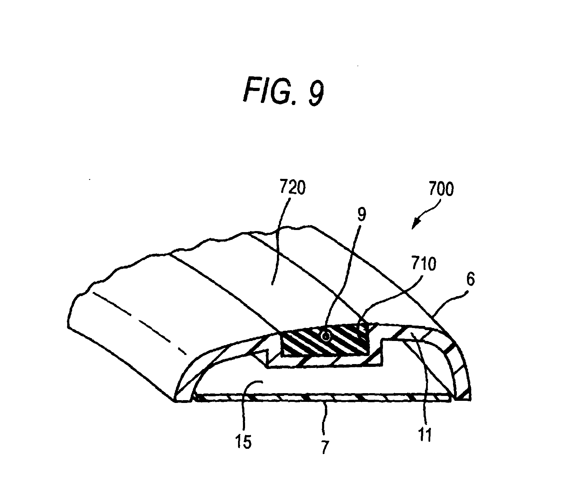

Next, a third embodiment of the toilet seat apparatus according to the invention will be explained in details in reference to Fig.9 as follows.

Fig.9 is a partially broken perspective view showing a third embodiment of the toilet seat apparatus according to the invention. According to a toilet seat apparatus 700 of the embodiment, as shown by Fig.9, a peripheral groove 710 in a recess shape is formed at an outer face of the upper lid 6, and an elastic body 720 arranged to contain with the piezoelectric sensor 9 in the cord-like at inside thereof is fitted into the peripheral groove 710. That is, the elastic body 720 is operated as pressing means for bending the piezoelectric sensor 9 by being deformed by the human body seated on the seat.

By constituting in this way, the elastic body 720 constitutes pressing means for easily bending the piezoelectric sensor 9 by being brought into direct contact with the human body and therefore, the piezoelectric sensor 9 delicately detects even small movement of the human body and can further firmly output the electric signal.

Further, when the elastic member 720 is constituted by a color different from that of the upper lid 6, aesthetic sense can also be promoted in view of design.

The invention is not limited to the above-described respective embodiments but can pertinently be modified or improved. Further, the materials, the shapes, the mode of arranging the respective constituent elements in the above-described respective embodiments are arbitrary so far as the invention can be achieved thereby and are not limited.

For example, although there is constructed the constitution of installing the piezoelectric sensor on the side of the base plate different from the face of installing the heater, since the piezoelectric sensor used in the respective embodiments is provided with the heat resistance as described above and therefore, the piezoelectric sensor can also be arranged on the face the same as that of the heater.

### (Fourth Embodiment)

According to the embodiment, an explanation will be given particularly of an example of using pressing means which is provided with an elasticity and in which a shape of a face thereof opposed to a vibration detecting sensor differs for amplifying means.

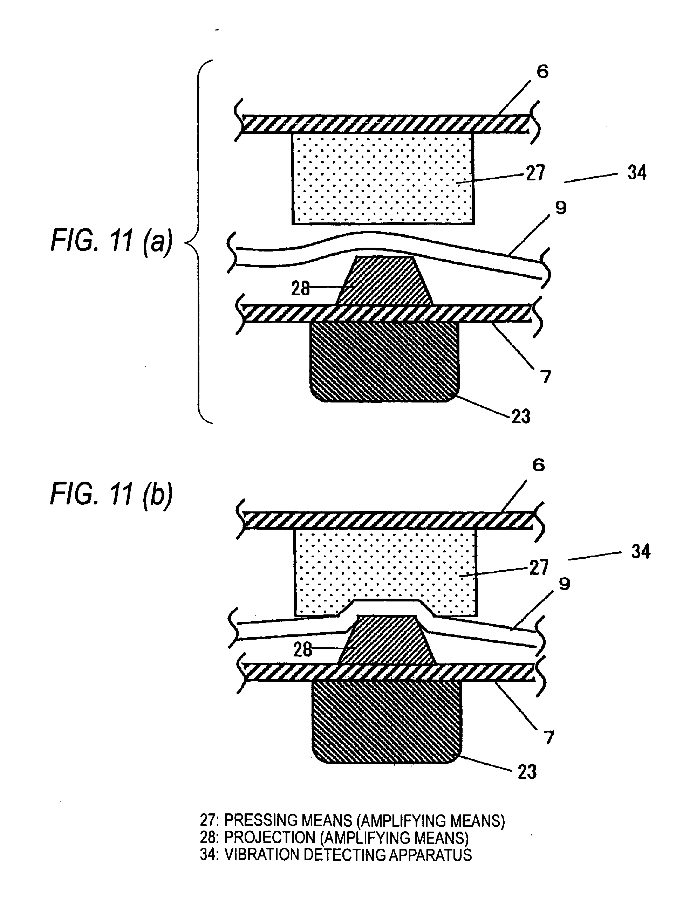

Fig. 1 is a disassembled perspective view of a toilet seat apparatus showing a fourth embodiment according to the invention, Fig.2 is a perspective view of an outlook of a vibration detecting sensor used in the toilet seat apparatus of Fig.1, Fig.3 is a perspective view of a total of a toilet to which the toilet seat apparatus shown in Fig.1 is applied, Fig.11 illustrates sectional views of the toilet seat apparatus of Fig.1, Fig.5 is a side view of a state of using the toilet shown in Fig.3, Fig.6 is a block constitution diagram of a control unit in the toilet seat apparatus, and Fig. 7 is a time chart of a sensor output and operation in the toilet seat apparatus.

As shown by Fig.1, the toilet seat apparatus 5 of the embodiment according to the invention is constituted by arranging the piezoelectric sensor 9 in the cord-like shape having a flexibility as a vibration detecting sensor at the base plate 7 of the case 8 comprising the upper lid 6 of the rigid body and the base plate 7 molded by resin.

The upper lid 6 forms the upper portion of the case 8 by including the main body portion 10 in the semicircular shape in a sectional view thereof. The lower face of the ceiling plate 11 of the main body portion 10 is attached with the heater 12 for heating. Further, the heater 12 is connected to the control unit 13, mentioned later, and is set to a desired temperature by manual operation.

The base plate 7 forms the lower portion of the case 8 by including the main body portion 14 in the channel-like shape in a sectional view thereof. The piezoelectric sensor 9 is attached to the upper face of the bottom plate 15 of the main body portion 14.

The upper lid 6 and the base plate 7 are integrally integrated by fitting a screw (not illustrated) from the through hole 16 formed at the base plate 7 to the locking portion 17 formed at the upper lid 6. Further, the piezoelectric sensor 9 is connected to the control unit 13 similar to the heater 12.

Here, simply describing of the piezoelectric sensor 9 in the cord-like shape used in the embodiment, as shown by Fig.2, the sensor 9 is a sensor in the cable-like shape using the piezoelectric element material and is constituted by the core line (center electrode) 18 arranged at the center in the axial direction, the piezoelectric element material 19 covered at the surrounding of the core line 18, the outer side electrode 20 arranged at the surrounding of the piezoelectric element material 19 and PVC (polyvinyl chloride resin) 21 for covering the outermost periphery.

The piezoelectric sensor 9 uses the piezoelectric element material 19 having a heat resistance capable of withstanding a surround temperature of about 120 °C and is provided with a flexibility (flexible performance) comparable to that of a normal vinyl cord by using the piezoelectric element material 19 constituted by resin having a flexibility (flexible performance) and piezoelectric ceramic and the flexible electrodes.

Further, the piezoelectric sensor 9 is provided with a high sensitivity comparable to that of a polymer piezoelectric element material and achieves a particularly high sensitivity in a low frequency region (10 Hz or lower) for detecting the heart rate of the human body. This is because a reduction in the sensitivity is small even in the low frequency region since the specific inductive constant (about 55) of the piezoelectric element material 19 is larger than the specific inductive contact (about 10) of the polymer piezoelectric element material.

The piezoelectric sensor 9 in the cord-like shape provided in this way as being molded with the piezoelectric element material 19 is not provided with the piezoelectric function and therefore, it is necessary to carry out the processing (polarizing processing) for providing the piezoelectric function to the piezoelectric element material 19 by applying the direct current high voltage of several KV/mm to the piezoelectric material 19. The polarizing processing is carried out by applying the direct current voltage between the two electrodes 18, 20 after forming the core line 18 and the outer side electrodes 20 to the piezoelectric element material 19.

As shown by Fig.3, the base plate 7 is mounted with 4 pieces of the pads 23 for absorbing impact attached to the rear face of the bottom plate 15 and having the elastic force for absorbing impact with the toilet main body 22 by being disposed between the toilet seat apparatus 5 and the toilet main body 22 when the toilet seat is used similar to the background art apparatus. Further, the toilet seat apparatus 5 is mounted with the lid member 25 lifted up to the side of the water tank 24 along with the toilet seat apparatus 5.

According to the embodiment, the piezoelectric sensor 9 in the cord-like shape is mounted onto the base plate 7 by being positioned and supported by the plurality of holders 26 arranged separately from each other.

Enlarging above the pad 23, as shown by Fig.11, there is constructed a constitution of including the pressing means (amplifying means) 27 having an elasticity attached to an inner face of the upper lid 6 as a rigid body, and the projection (amplifying means) 28 of the rigid body attached onto the base plate 7 and pinching the piezoelectric sensor 9 by the pressing means 27 and the projection 28. Fig. 11(a) shows a state before integrating the upper lid 6 and the base plate 7, and Fig.11 (b) shows a state of screwing to integrate the upper lid 6 and the base plate 7. When the user is seated on the toilet seat apparatus 5, naturally, a state of Fig.11(b) is brought about, the upper lid 6 and the base plate 7 are constituted by rigid bodies such that the toilet seat apparatus 5 is not destructed even when the body weight is applied thereon.

Here, the rigid body is defined as a member which is not deformed to exceed a strength thereof even when at least the body weight of the person applies thereon, particularly, a member by which the person does not feel deformation such that the hip portion sinks when the person is seated thereon, that is, a member by which the user does not feel anxiety for the strength. A material therefor is not particularly limited but may be an insulating member of resin, or ceramic or the like, or may be a conductor of a metal or the like, however, it is added that the toilet seat of the background art is generally made of resin.

Now, although in a state in which the user is seated on the toilet seat, all the body weight of the user is applied on the toilet seat, however, the upper lid 6 is hardly deformed partially since the upper lid 6 is constituted by the rigid body. However, it is conceivable that the total of the toilet seat is vibrated by body movement of the user although the vibration is slight. A detailed explanation will be given as follows of how the vibration of the toilet seat is amplified and transmitted to the piezoelectric sensor 9.

First, the pressing means 27 is disposed between the upper lid 6 and the piezoelectric sensor 9 and therefore, the pressing means 27 is vibrated by vibrating the upper lid 6, however, a behavior of the vibration is considerably complicated. Whereas the upper lid 6 is constituted by the rigid body, the pressing means 27 is a member having an elasticity and therefore, whereas an upper portion of the pressing means 27 (a vicinity of a portion thereof connected to the upper lid 6) is vibrated similar to the upper lid 6, a lower portion of the pressing means 27 (vicinity of a portion thereof connected to the piezoelectric sensor 9) is vibrated slightly delayedly to repeat vibration different from that of the upper lid 6. Therefore, the piezoelectric sensor 9 is transmitted not only with vibration of the upper lid 6 and the base plate 7 as the rigid bodies but also different vibration by the pressing means 27 and the pressing means 27 carries out so-to-speak amplification of the vibration of the upper lid 6. Therefrom, the pressing means 27 can be regarded as a kind of amplifying means.

Next, the pressing means 27 and the piezoelectric sensor 9 are opposed to each other by shapes different from each other. There is constructed a constitution in which the piezoelectric sensor 9 is longer in a left and right direction of Fig.11 and the pressing means 27 is longer in a depth direction of Fig. 11, further, whereas the piezoelectric sensor 9 is opposed thereto by a curved face owing to the cable-like shape, the pressing means 27 is opposed thereto by a plane. Therefrom, the piezoelectric sensor 9 is provided with a portion which is not brought into contact with the pressing means 27 and a portion which is brought into contact with the pressing means 27 and in the portion brought into contact with the pressing means 27, there are present various portions having different states of pressing from a portion which is strongly pressed thereby to a portion which is not pressed considerably thereby. Therefore, the piezoelectric sensor 9 receives vibration which differs by the portions such that at the portion which is not brought into contact with the pressing means 27, the piezoelectric sensor 9 mainly receives vibration as a rigid body and at the portion which is strongly pressed to the pressing means 27, the piezoelectric sensor 9 receives vibration different from that of the rigid body by the pressing means 27. That the total of the piezoelectric sensor does not receive the same vibration but receives vibration which differs by the portions is equal to that the piezoelectric sensor 9 is partially deformed and therefore, the output of the piezoelectric sensor can be increased. As a result, the piezoelectric sensor 9 is deformed from the vibration of the rigid body which is hardly deformed and therefore, vibration of the rigid body is amplified. Therefrom, a difference between the shapes of the faces of the pressing means 27 and the piezoelectric sensor 9 opposed to each other can be regarded as a kind of amplifying means.

Next, the projection 28 and the piezoelectric sensor 9 are opposed to each other by shapes different from each other. Therefrom, the piezoelectric sensor 9 is provided with a portion which is not brought into contact with the projection 28 and a portion which is brought into contact with the projection 28. Therefore, relatively, the piezoelectric sensor 9 receives vibration or does not receive vibration depending on portions thereof such that at the portion which is not brought into contact with the projection 28, vibration as a rigid body is not received so much and at the portion brought into contact with the projection 28, the piezoelectric sensor 9 receives vibration as the rigid body by the projection 28 similar to that of the base plate 7 or the upper lid (rigid body) 6. That the total of the piezoelectric sensor does not receive the same vibration but the total of the piezoelectric sensor receives vibration or does not receive vibration depending on the portions is equal to that the piezoelectric sensor 9 is partially deformed and therefore, the output of the piezoelectric sensor can be increased. As a result, the piezoelectric sensor 9 can be deformed although the deformation is slight from vibration of the rigid body which is hardly deformed and therefore, vibration of the rigid body is amplified. Therefrom, a difference of shapes of faces of the projections 28 and the piezoelectric sensor 9 opposed to each other can be regarded as a kind of amplifying means. However, an effect of amplification by the projection 28 is smaller than an effect of amplification of the pressing means 27, mentioned above.

Next, the pressing means 27 and the projection 28 are opposed to each other by shapes different from each other and particularly, an area of the pressing means 27 is larger. Since the projection 28 is constituted by the rigid body, only the center portion of the pressing means 27 is compressed and at the compressed center portion, the elasiticity of the pressing means 27 is hampered and vibration near to that of the rigid body is executed at the center portion. Further, a force is exerted to the piezoelectric sensor 9 by the pressing means 27 and the projection 28 and the piezoelectric sensor 9 is fixed to some degree. A magnitude of the force is changed also by the elasticity of the pressing means 27 and a distance between the upper lid 6 and the base plate 7 when integrated. The piezoelectric sensor 9 is further exerted with the force and is firmly fixed when the elasticity of the pressing means 27 is low, or when the distance between the upper lid 6 and the base plate 7 is short. On the other hand, a side of a surrounding of the pressing means 27 (a portion thereof to which the projection 28 is not opposed) can be vibrated differently from that of the rigid body since the elasticity is maintained. Therefore, a center portion of the pressing means 27 is fixed to execute vibration near to that of the rigid body and a side of surrounding of the pressing means 27 (portion to which the projection 28 is not opposed) executes vibration different from that of the rigid body by only being pressed by the pressing means 27. At this occasion, a point which requires caution is that the portion executing vibration near to that of the rigid body and the portion which executes vibration different from that of the rigid body are disposed at the positions extremely proximate to each other in the piezoelectric sensor 9. That vibrations different from each other are received at the positions extremely proximate to each other is equal to that the piezoelectric sensor 9 is locally deformed and therefore, the output of the piezoelectric sensor can be increased. As a result, the piezoelectric sensor 9 can be deformed although the deformation is small from vibration of the rigid body which is hardly deformed and therefore, vibration of the rigid body is amplified. Therefrom, the difference between the shapes of the faces of the pressing means 27 and the projection 28 can be regarded as a kind of amplifying means. Here, the projection 28 may not be the rigid body. Because even when the projection 28 is constituted by an elastic body, elasticity can be hampered by strongly compressing the pressing means 27 at the center portion by increasing the height of the pressing means 27 or the projection 28 or shortening the distance between the upper lid 6 and the base plate 7.

Here, with regard to the elasticity of the pressing means 27, the elasticity may be higher than that of the upper lid and the above-described effect as amplifying means is achieved. For example, a representative cushion material may be used therefor and the pressing means 27 may be constituted by rubber or sponge.

As shown by Fig. 5, in using the toilet seat apparatus 5 having the above-described constitution, when the toilet seat apparatus 5 is arranged on the toilet main body 22 and is applied with a weight of the human body M by seating, as described above, the piezoelectric sensor 9 is pressed. As a result, vibration in accordance with motion of the human body M is amplified to apply on the piezoelectric sensor 9 to firmly output an electric signal.

The electric signal provided in accordance with acceleration of vibration is supplied to the control unit 13.

However, the electric signal outputted from the piezoelectric sensor 9 is masked by the control unit 13 such that in using the toilet seat apparatus 5, vibration generated by driving a cleaning nozzle, operating a blower or flushing water or the like does not constitute noise for detecting presence/absence, a heart rate or the like of the human body.

As shown by Fig.6, the control unit 13 is provided with the filter circuit, the amplifying means, the smoothing means, the determining means and the like, not illustrated, at inside of the control portion 29 and is mounted with the heart rate calculating means 30 for calculating the heart rate based on the output signal of the smoothing means, the displaying means 31 for displaying the output signal of the output calculating means 30, the comparing means 32 for comparing the calculated output of the heart rate calculating means and the set value and the alarm issuing means 33 for issuing the alarm based on the result of the comparison and is inputted with the detected signal of the piezoelectric sensor 9.

When the piezoelectric sensor 9 detects body movement of the human body M and outputs the electric signal, the control unit 13 filters the electric signal by the filter circuit, thereafter, amplifies the electric signal by the amplifying means, further, smoothes the electric signal by the smoothing means.

As shown by Fig. 7, a large output waveform is outputted from the smoothing means at the instance at which the human body M is seated on the toilet seat apparatus 5, when an article is mounted thereon, or when the body is moved. On the other hand, when the still state is brought about after the human body M is seated thereon, the output waveform of the comparatively low level is outputted from the smoothing means by small body movement of the body propagated by action of the heart or respiratory action.

In contrast thereto, when the human body M is not present or when an article is mounted thereon, an output waveform is not indicated in a constant period of time by the smoothing means after outputting a large output waveform.

Hence, the determining means compares an output V of the smoothing means and previously determines two set values Va, Vb as follows to determine. That is, when V < Va, it is determined that the human body M and the article is not present (unpresent output Hi). When Va ≦ V < Vb, it is determined that the human body M is present in the still state (present output Hi). Further, when Vb < V, it is determined that body movement is brought about in the human body M. When the article is mounted thereon in place of the human body M, although determination of seating or body movement is temporarily executed, it is determined that the human body M is not present by determining a state of placing the article since the low level vibration propagated by action of the heart and the respiratory action as in the human body M does not appear.

When the determining means determines seating of the person, the deodorizing means and the heating means are started to operate. The operation is stopped when it is determined that the person is not present.

Further, the heating means governs temperature control of the heater 12.

Further, a small signal propagated by action of the heart is outputted from the smoothing means when the human body M is seated thereon in the still state. Based on the signal, the heart rate is calculated to output by the heart rate calculating means 30. The result of calculation is displayed by the displaying means 31 which is the outside monitor. Further, as shown by Fig. 6, the control unit 13 is mounted with the comparing means 32 for comparing the output signal of the heart rate calculating means 30 and the predetermined set value and the alarm issuing means 33 for issuing the alarm by the output of the comparing means 32 and can issue the alarm when the heart rate is equal to or larger than the set value. Particularly, when the person strains in evacuation, the heart rate is increased and there is the concern of bringing about occurrence of the cerebral hemorrhage, however, the control unit 13 can contribute to health control by foreseeing the attack. At this occasion, when the control unit 13 is connected by a network, in, for example, a hospital or the like, not only the toilet seat apparatus 5 used in the hospital can centrally be monitored summarizingly but also behavior in the rest room which cannot be diagnosed directly can always be monitored.

Further, the signal to the displaying means 31 can be transmitted via communicating means by wired means or wireless means.

According to the above-described toilet seat apparatus 5, the piezoelectric sensor 9 achieves high reliability by easily detecting even slight movement of the human body by firmly supplying the electric signal in accordance with acceleration of vibration to the control unit 13. Further, the piezoelectric sensor 9 can firmly detect the seating or the like since the piezoelectric sensor 9 is flexible and is difficult to be destructed even when impact continues applying thereto and outputs the detected signal facilitating to differentiate the person and the article.

Further, although according to the above-described embodiment, presence/absence, a heart rate or the like is determined by smoothing the output signal from the piezoelectric sensor 9, there may be constructed a constitution in which the output signal of the piezoelectric sensor 9 is amplified as necessary and thereafter converted into digital data by AD conversion by a microcomputer or the like, presence/absence is determined based on a value constituted by subjecting the digital data to a moving average in the microcomputer, or a heart rate number or the like is calculated by calculating an autocorrelation coefficient of the digital data.

The effect of the embodiment described above will be summarized.

There is constructed the constitution including the pressing means (amplifying means) 27 for amplifying vibration transmitted to the upper lid (rigid body) 6, the projection (amplifying means) 28 and including the piezoelectric sensor (vibration detecting sensor) 9 for detecting amplified vibration and the vibration detection apparatus 34 is formed by the pressing means (amplifying means) 27, the projection (amplifying means) 28, the piezoelectric sensor (vibration detecting sensor) 9.

Thereby, despite that the upper lid (rigid body) 6 is hardly deformed by vibration transmitted to the upper lid (rigid body) 6, vibration transmitted to the upper lid (rigid body) 6 is amplified by the amplifying means and detected by the vibration detecting sensor 34 and therefore, vibration transmitted to the upper lid (rigid body) 6 can accurately be detected.

Further, as amplifying means, there is constructed the constitution including the pressing means 27 having elasticity for pressing the piezoelectric sensor (vibration detecting sensor) 9.

Thereby, the pressing means 27 is vibrated by vibration of the upper lid (rigid body) 6, since the pressing means 27 is provided with elasticity, the piezoelectric sensor (vibration detecting sensor) 9 can be pressed by vibration amplified more than vibration of the upper lid (rigid body) 6.

Further, as the amplifying means, there is constructed the constitution including the pressing means 27 for pressing the piezoelectric sensor (vibration detecting sensor) 9 by a face thereof opposed thereto having a shape different from that of the piezoelectric sensor (vibration detecting sensor) 9.

Thereby, since the shapes of the faces of the piezoelectric sensor (vibration detecting sensor) 9 and the pressing means 27 opposed to each other differ from each other, at the piezoelectric sensor (vibration detecting sensor) 9, there are brought about a portion which is strongly pressed by the pressing means 27 by vibration of the upper lid (rigid body) 6 and the portion which is not pressed so much thereby and in comparison with the case in which the piezoelectric sensor (vibration detecting sensor) 9 is pressed uniformly from all the face, the piezoelectric sensor (vibration detecting sensor) 9 can be pressed by amplified vibration.

Further, the vibration detecting sensor is constituted by the piezoelectric sensor 9 having flexibility.

Thereby, when the piezoelectric sensor 9 receives vibration, the piezoelectric sensor 9 is easily deformed, the output in accordance with the deformation is produced and therefore, the output in accordance with vibration can accurately be outputted.

Further, according to the toilet seat apparatus 5 having the vibration detecting apparatus 34, there is constructed the constitution in which the rigid bodies are the upper lid (rigid body) 6, the base plate 7 and body movement of the user transmitted to the toilet is detected.

Thereby, even when the toilet seat is hardly deformed by body movement of the user transmitted to the toilet seat, body movement of the user transmitted to the toilet seat is amplified by the amplifying means and is detected by the piezoelectric sensor (vibration detecting sensor) 9 and therefore, body movement of the user transmitted to the toilet seat can accurately be detected.

Further, according to the toilet seat apparatus 5, there is constructed the constitution for detecting at least one of the seating, the heart beat, the respiration from the body movement of the user.

Thereby, it is easy to detect the seating, that is, motion information whether the user is seated thereon or biologic information of the heart rate or the respiration of the user based on the accurately detected body movement and the multifunction toilet seat apparatus 5 making full use of detected information can be realized.

Further, an explanation will be given of other example using particularly pressing means having an elasticity and having a different shape of a face thereof opposed to the vibration detecting sensor to the amplifying means in comparison with that of Fig.11.

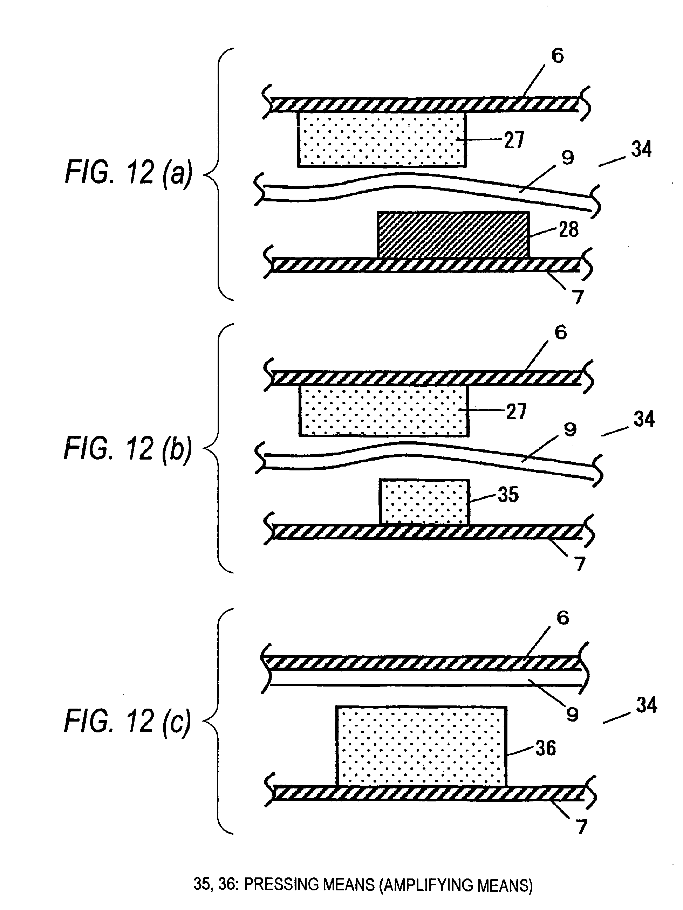

First, Figs.12(a) to 12(c) show examples having different arrangements. Fig.12(a) shows a constitution of arranging the pressing means (amplifying means) 27 and the projection (amplifying means) 28 at respective positions. Fig.12(b) shows a constitution of using the pressing means 35 having an elasticity for the amplifying means in place of a projection of a rigid body, in which positions of right ends of the pressing means 27 and the pressing means 35 are aligned and positions of left ends thereof are shifted from each other. In Fig.12(c), the piezoelectric sensor (vibration detecting sensor) 9 is disposed on a side of the upper lid (rigid body) 6 relative to the pressing means 36 and is particularly fixed directly to the upper lid (rigid body) 6.

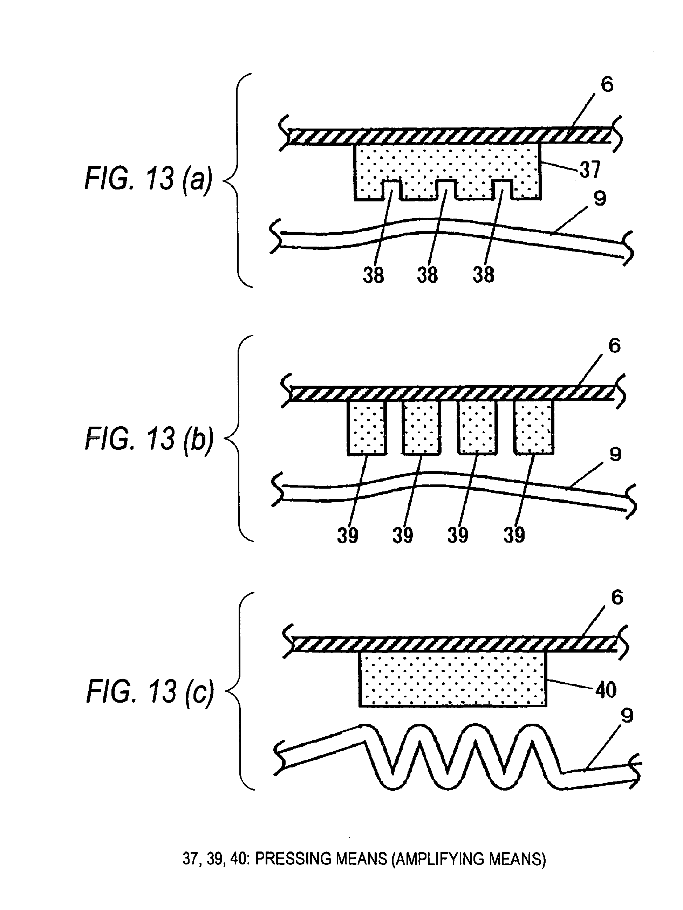

Figs.13(a) to 13(c) show examples in which shapes of faces of the pressing means (amplifying means) and the piezoelectric sensor (vibration detecting sensor) opposed to each other differ from each other. In Fig.13(a), a recess portion 38 is formed at the pressing means (amplifying means) 37. Naturally, a projected portion may be provided. In Fig.13(b), a plurality of the pressing means (amplifying means) 39 are provided. In Fig.13(c), the pressing means 40 is opposed to the piezoelectric sensor 9 by a flat face and the piezoelectric sensor (vibration detecting sensor) 9 is bent.

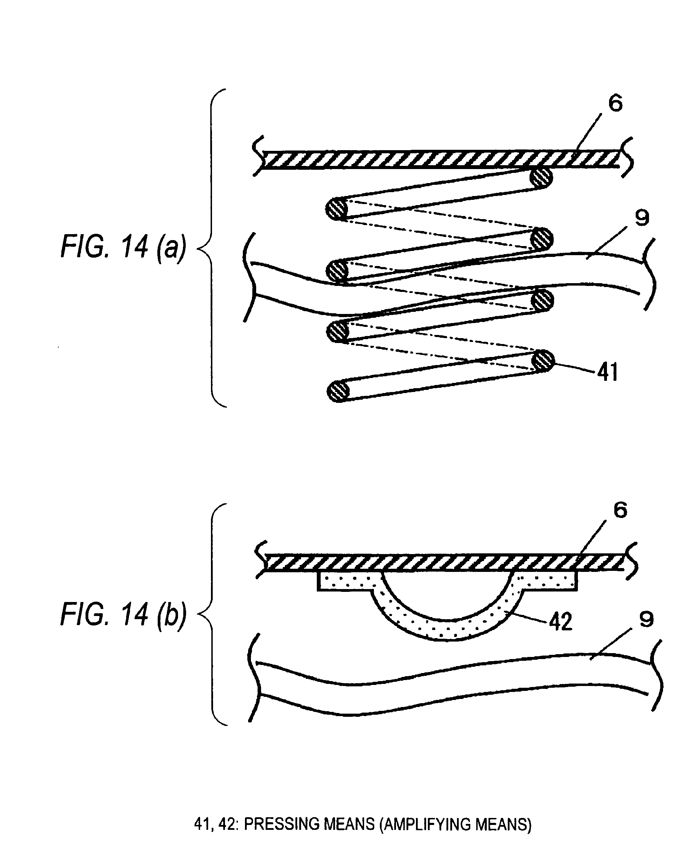

Figs.14(a) and 14(b) show other example of pressing means (amplifying means) having an elasticity. In Fig.14(a), a spring is used as the pressing means (amplifying means) 41. The pressing means (amplifying means) 42 of Fig. 14(b) is provided with an elasticity by bending and fixing a thin spring member made of a metal.

### (Embodiment 5)

According to the embodiment, an explanation will be given on an example of using particularly pressing means which is not provided with an elasticity and is provided with a different shape of a face thereof opposed to the vibration detecting sensor for amplifying means.

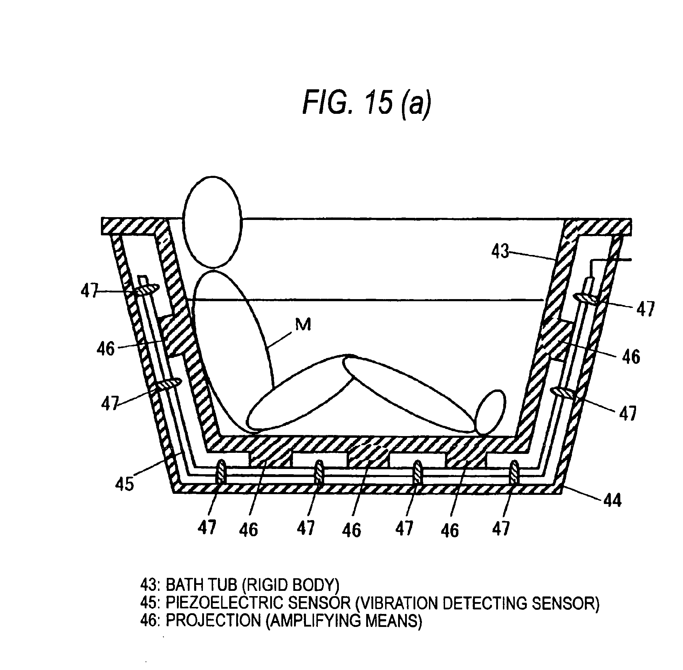

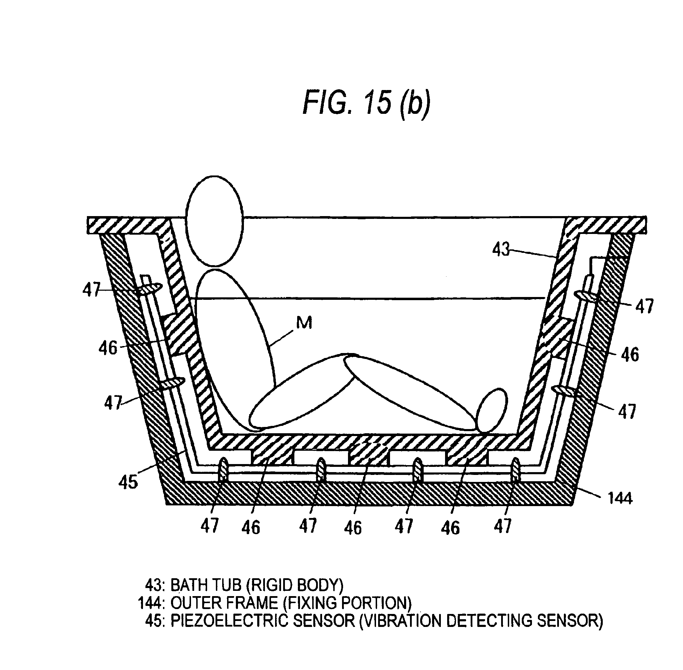

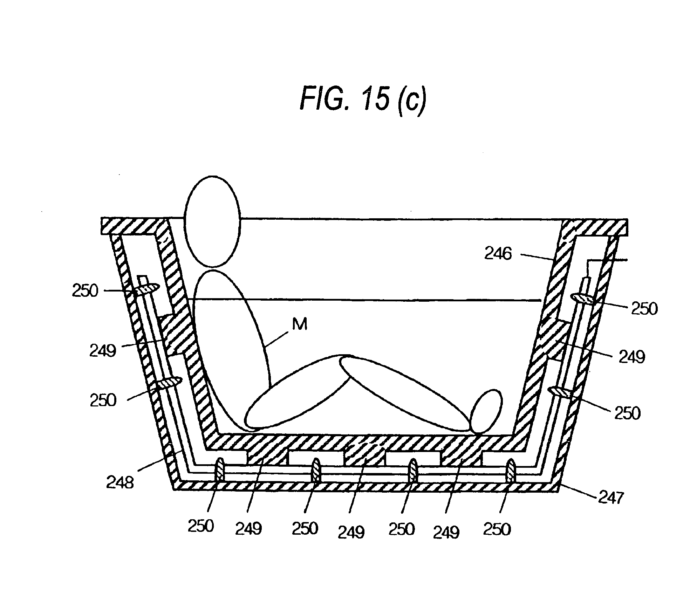

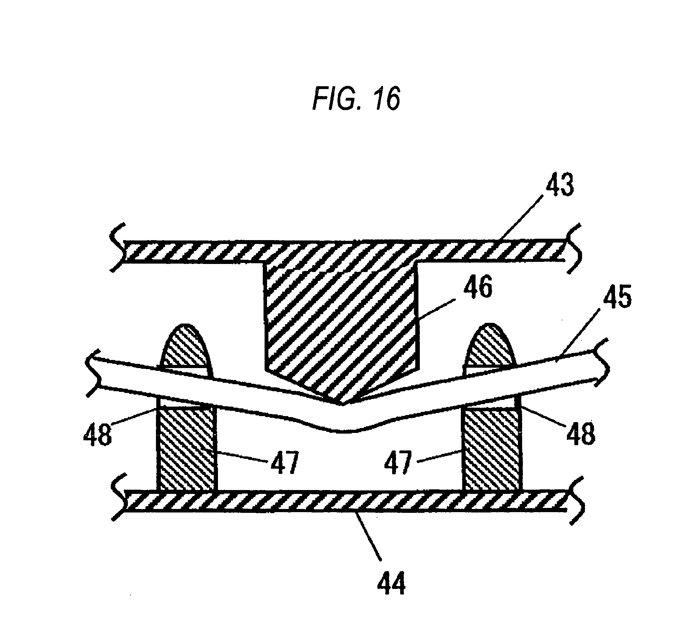

Fig.15(a) is a constitution view of a section of a bath tub apparatus showing a fifth embodiment according to the invention, and Fig.16 is a sectional view of an essential portion of Fig.15(a).

As shown by Fib.15(a), the bath tub apparatus according to the embodiment of the invention is arranged with the piezoelectric sensor 45 in the cord-like shape having a flexibility as the vibration detecting sensor between a bath tub 43 of a rigid body and a cover 44. Further, the bath tub apparatus includes the projection 46 projected from the bath tub 43 to the piezoelectric sensor 45, and a holder 47 for supporting the piezoelectric sensor 45 at the cover 44 and the piezoelectric sensor 45 is positioned by way of a hole 48 of the holder 47.

Here, the projection 46 and the piezoelectric sensor 45 are opposed to each other by shapes thereof different from each other. There is constructed a constitution in which the piezoelectric sensor 9 is longer in a left and right direction of Fig.16 and the projection 46 is longer in a depth direction of Fig.16, whereas the piezoelectric sensor 9 is provided with a curved face owing to the cable-like shape and is opposed thereto by a constant shape in the left and right direction, the projection 46 is constituted by a shape in which a center portion thereof in the left and right direction is further projected. Therefrom, the piezoelectric sensor 45 is provided with a portion which is not brought into contact with the projection 46 and a portion which is brought into contact with the projection 46, further, in the portion brought into contact with the projection 46, there are present various portions having different pressed states from a portion (center) which is strongly pressed to a portion which is not pressed so much.

In addition thereto, although the piezoelectric sensor 45 is attached to the cover 44 via the holder 47, a situation is changed by whether the cover 44 is a rigid body or an elastic body, further, whether the holder 47 is a rigid body or an elastic body.

First, when the cover 44 is not integrally fixed to the bath tub 43, regardless of a material of the cover 44 or a material of the holder 47, although the bath tub 43 is vibrated by body movement of the user, the cover 44 is not vibrated. Vibration of the bath tub 43 is transmitted to the piezoelectric sensor 45 only from the projection 46 and therefore, the piezoelectric sensor 45 receives vibration only from a portion thereof brought into contact with the projection 46 and a portion thereof which is not brought into contact with the projection 46 does not receive vibration. That a total of the piezoelectric sensor 45 does not receive the same vibration but the piezoelectric sensor 45 receives vibration which differs by the portions is equal to that the piezoelectric sensor 45 is partially deformed and therefore, the output of the piezoelectric sensor 45 can be increased. As a result, the piezoelectric sensor 45 can be deformed by vibration of the bath tub (rigid body) 43 which is hardly deformed and therefore, vibration of the bath tub (rigid body) 43 is amplified. Therefrom, a difference between the shapes of the faces of the projection 46 and the piezoelectric sensor 45 opposed to each other can be regarded as a kind of amplifying means.

Next, when the cover 44 is integrally fixed to the bath tub 43 and at least one of the cover 44 and the holder 47 is constituted by an elastic body, the bath tub 43 is vibrated by body movement of the user and vibration by way of the cover 44 and the holder 47 is constituted by vibration which differs from that of the bath tub 43 owing to vibration by way of the elastic member. Although vibration of the bath tub 43 is transmitted to the piezoelectric sensor 45 from the projection 46, also vibration by way of the cover 44 and the holder 47 is transmitted to the piezoelectric sensor 46 and therefore, the piezoelectric sensor 45 receives vibration which differs by the portion brought into contact with the projection 46 and the portion brought into contact with the holder 47. That a total of the piezoelectric sensor 45 does not receive the same vibration but receives vibration which differs by the portions is equal to that the piezoelectric sensor 45 is partially deformed and therefore, the output of the piezoelectric sensor 45 can be increased. As a result, the piezoelectric sensor 45 can be deformed from vibration of the bath tub (rigid body) 43 which is hardly deformed and therefore, vibration of the bath tub (rigid body) 43 is amplified. Therefrom, the projection 46, the cover 44, the holder 47 can be regarded to constitute a kind of amplifying means.

Finally, in the case in which the cover 44 is integrally fixed to the bath tub 43 and both of the cover 44 and the holder 47 are constituted by rigid bodies, when the bath tub 43 is vibrated by body movement of the user, it seems that vibration transmitted from the projection 46 to the piezoelectric sensor 45 and vibration by way of the cover 44 and the holder 47 are constituted by the same vibration. However, microscopically, there is a slight time difference therebetween to a degree which cannot be determined by the human being. That is, when the bath tub 43 is vibrated, the projection 46 is immediately vibrated, however, the holder 47 is not vibrated immediately. After the bath tub 43 is vibrated, the cover 47 is vibrated by way of a connecting portion at a surrounding of the bath tub 43 and the holder 47 starts to be vibrated further thereafter. That is, a length of a path of transmitting vibration is longer. Therefore, the piezoelectric sensor 45 receives vibration which differs slightly by the portion brought into contact with the projection 46 and the portion brought into contact with the holder 47. That a total of the piezoelectric sensor 45 does not receive the same vibration but receives vibration which differs by the portions is equal to that the piezoelectric sensor 45 is partially deformed and therefore, the output of the piezoelectric sensor 45 can be increased. As a result, the piezoelectric sensor 45 can be deformed from vibration of the bath tub (rigid body) 43 which is hardly deformed and therefore, vibration of the bath tub (rigid body) 43 is amplified. Therefrom, the projection 46, the cover 44, the holder 47 can be regarded to constitute a kind of amplifying means. However, in this case, the amplifying function is smaller than those in the above-described cases and therefore, a circuit of processing the sensor output needs to be devised to increase an amplification factor or the like.

Now, the embodiment includes other constitution having the amplifying function.

First, the piezoelectric sensor 45 is elastic. The piezoelectric sensor 45 having a flexibility (flexible performance) is also provided with an elasticity and therefore, the piezoelectric sensor 45 per se can be vibrated by vibration from the projection 46 brought into contact with the piezoelectric sensor 45 and vibration from the holder 47, particularly, by the elasticity of the piezoelectric sensor 45, vibration which differs from original vibration can be produced. That is, this is a mechanism in which while generating an output with regard to the provided original vibration, still other output is generated by producing different vibration by the piezoelectric sensor 45 per se thereby.

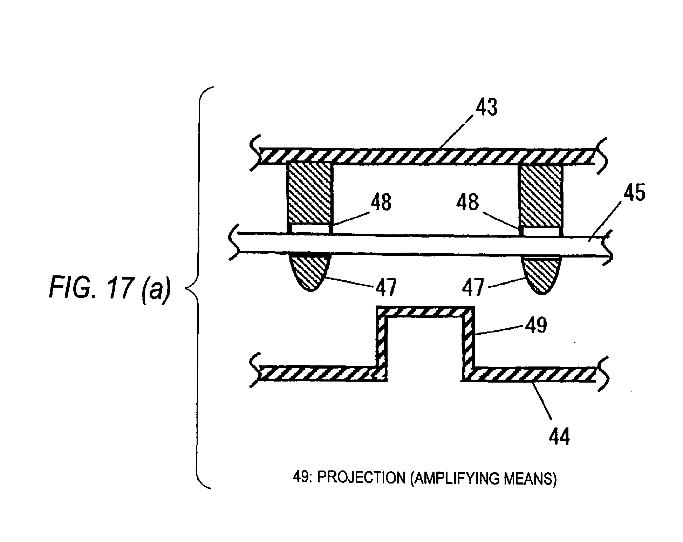

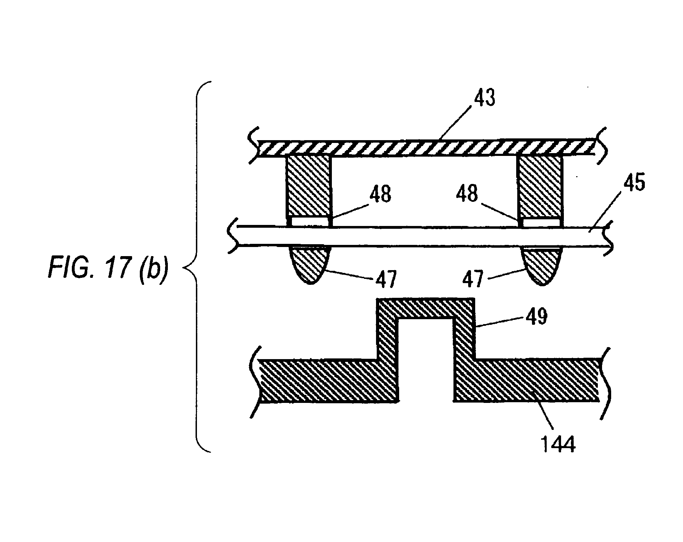

Next, the piezoelectric sensor 45 is mounted in a state of being applied with a tension. In Fig.16, the piezoelectric sensor 45 is supported in a state of being applied with the tension, that is, in a state of being pulled tightly. At this occasion, vibration is propagated in the piezoelectric sensor 45 more remotely than in a case in which the tension is not applied thereto. When the piezoelectric sensor 45 is applied with the tension to some degree, vibrations from the projections 46, the holder 47 brought into contact with the piezoelectric sensor 45 are propagated in the piezoelectric sensor 45 more remotely, as a result, the output can be generated from various locations in the piezoelectric sensor 45. According thereto, it is conceived that there is achieved an effect the same as that in increasing a sensitivity of the sensor, that is, amplifying vibration. Further, with regard to what degree of a tension is preferably applied thereto, it is preferable to constitute a range in which at least a mechanical strength of the piezoelectric sensor 45 is not deteriorated, particularly a range of capable of maintaining the elasticity. Further, Fig.17(a) shows other example of using pressing means which is not provided with elasticity and in which a shape of a face opposed to the vibration detecting sensor differs for amplifying means.

In Fig.17(a), a projection (amplifying means) 49 is constituted on a side of the cover 44 and the holder 47 is constituted on a side of the bath tub 43. Further, Fig.17(a) shows other example of using pressing means which is not provided with elasticity and in which a shape of a face opposed to the vibration detecting sensor differs for amplifying means.

Further, although according to the embodiment, the bath tub is shown as the rigid body, the bath tub is constituted by various materials when viewed historically. The materials are constituted by wood, resin, metals of stainless steel and the like, marble, rock, tile and the like. The rigid body for the bath tub can be adopted so far as the rigid body is constituted by a material which is not deformed considerably to be sensed by the user in normal bathing and in which vibration is conducted.

### (Embodiment 6)

According to the embodiment, an explanation will be given of an example of using particularly pressing means which is provided with an elasticity and in which a shape of a face thereof opposed to the vibration detecting sensor is equal for amplifying means.

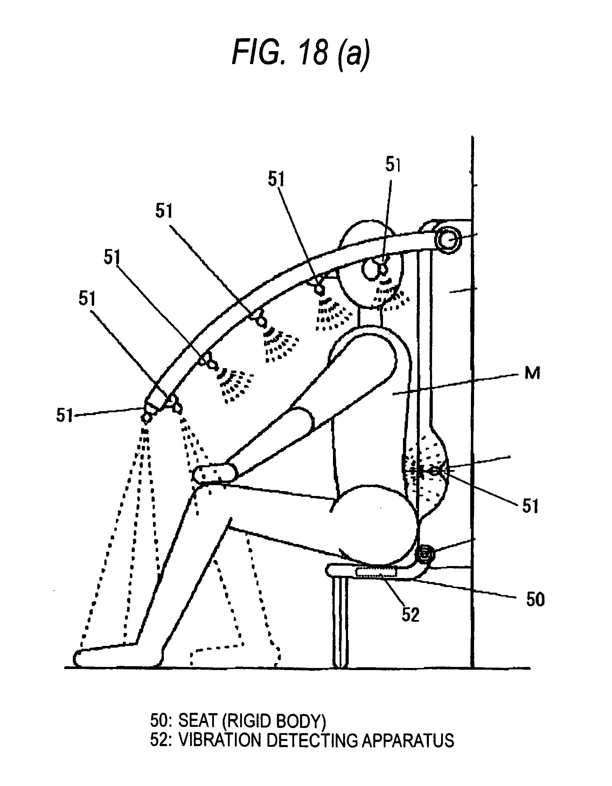

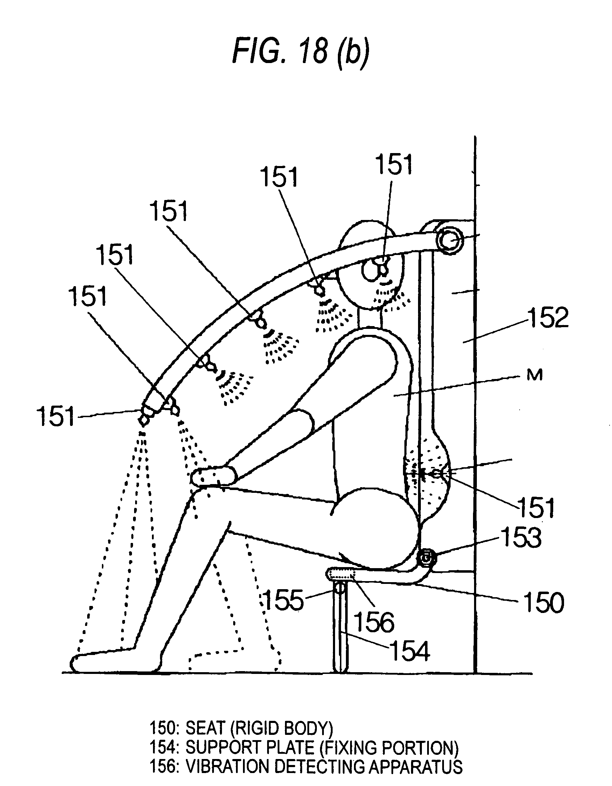

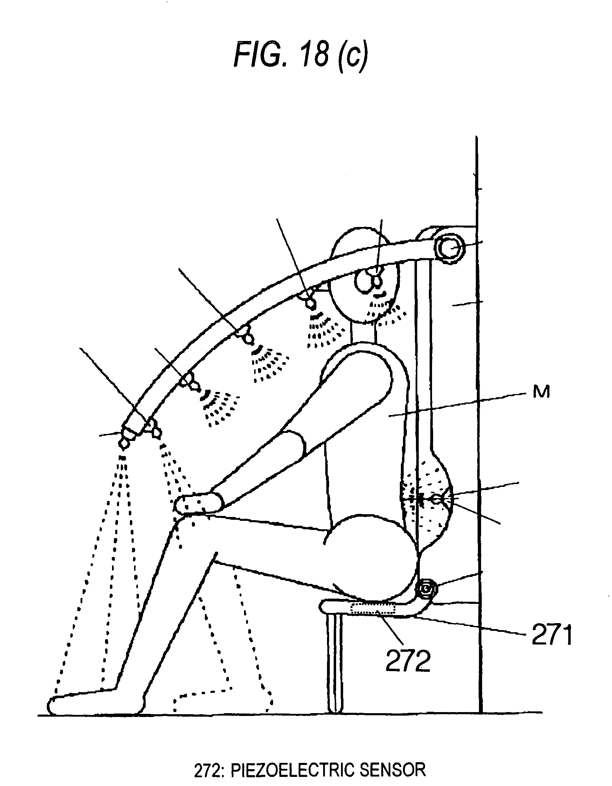

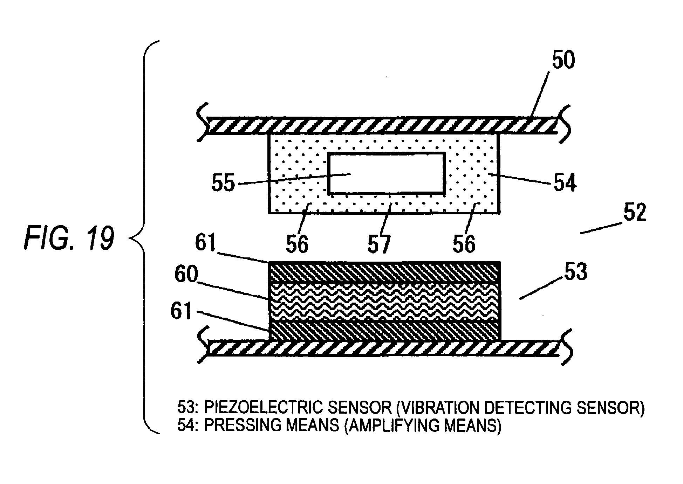

Fig.18(a) is a constitution view of a shower apparatus showing a sixth embodiment of the invention and Fig.19 is a sectional view of an essential portion of Fig.18(a).

According to the shower apparatus of the embodiment of the invention, as shown by Fig.18(a), the shower can be used in a seated attitude and a person can be flushed with hot water injected from a plurality of shower nozzles 51 in a state of being seated on the seat 50. Inside of the seat 50 of the rigid body is integrated with the vibration detecting apparatus 52 and the vibration detecting apparatus 52 includes the piezoelectric sensor 53 having an elasticity in a sheet-like shape as the vibration detecting sensor, and pressing means 54 having an elasticity as amplifying means. As shown by Fig.19, even when shapes of faces of the pressing means 54 and the piezoelectric sensor 53 opposed to each other are flat to be equal to each other, by constructing a constitution having a cavity 55 at a center of the pressing means 54, the effect of amplification is enlarged. In considering a pressing portion 56 for pressing the piezoelectric sensor 53 without interposing the cavity 55 therebetween and a pressing portion 57 for pressing the piezoelectric sensor 53 by interposing the cavity 55 therebetween, although the piezoelectric sensor 53 is pressed by the pressing portion 56 more strongly, the piezoelectric sensor 53 is pressed from the pressing portion 57 more weakly, which can be regarded as a constitution having a function similar to those of Figs.13(a) (b).

That is, in the piezoelectric sensor 53, there are present a portion which receives strong vibration by being strongly pressed by the pressing portion 56 and a portion which does not receive vibration so much by being pressed not so much by the pressing portion 57. That a total of the piezoelectric sensor 53 does not receive the same vibration but receives vibration which differs by the portions is equal to that the piezoelectric sensor 53 is partially deformed and therefore, the output of the piezoelectric sensor 53 can be increased. As a result, the piezoelectric sensor 53 can be deformed from vibration of the rigid body (seat 50) which is hardly deformed and therefore, vibration of the rigid body is amplified. As a result, the pressing means 54 can further amplify vibration by the cavity 55, the pressing portions 56, 57.

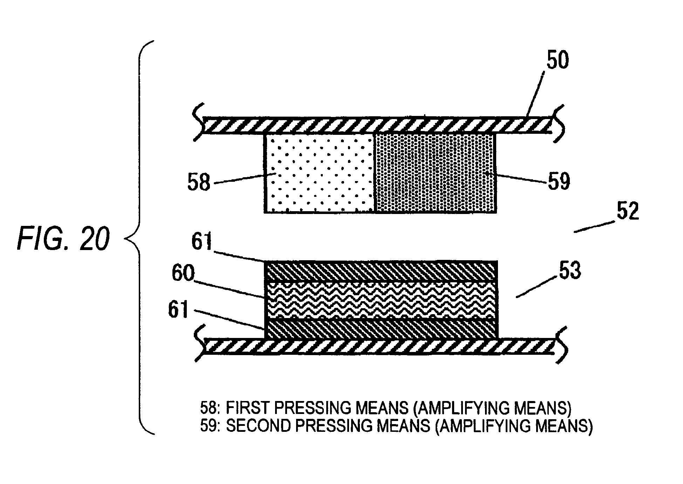

Further, Fig.20 shows other example of using particularly pressing means which is provided with an elasticity and in which a shape of a face thereof opposed to the vibration detecting sensor is equal for amplifying means.

In Fig.20, there are provided the first pressing means (amplifying means) 58 and the second pressing means (amplifying means) 59 having different elasticities as pressing means and different vibrations are transmitted to faces thereof opposed to the piezoelectric sensor 53 by a difference between the respective elascticities. That a total of the piezoelectric sensor 53 does not receive the same vibration but receives vibrations which differ by the portions is equal to that the piezoelectric sensor 53 is partially formed and therefore, the output of the piezoelectric sensor 53 can be increased. The piezoelectric sensor 53 is deformed from vibration of the rigid body (seat 50) which is hardly deformed and therefore, vibration of the rigid body is amplified. As a result, the difference between the elasticities of the first pressing means (amplifying means) 58 and the second pressing means (amplifying means) 59 can be regarded as amplifying means for amplifying vibration of the rigid body (seat 50).

Further, the piezoelectric sensor 53 of the embodiment is constituted not by a cable-like shape but by a sheet-like shape by attaching a piezoelectric element material to a piezoelectric sheet 60 and attaching conductive rubber 61 as electrodes to both faces of the piezoelectric sheet 60.

Further, although in Fig.12, Fig.13, Fig.14, Fig.17(a), Fig.19, Fig.20, there is a gap between the pressing means and the projection and the piezoelectric sensor, similar to Fig.11 (a), a gap is provided for simplifying explanation and it is added that the both members are brought into contact with each other when actually used.

### (Embodiment 7)

According to the embodiment, an explanation will be given of an example of mounting the vibration detecting sensor at inside of a rigid body (toilet seat) and constituting a fixing portion at outside of the rigid body.

Fig. 1 is the disassembled perspective view of the toilet seat apparatus showing a seventh embodiment according to the invention, Fig. 2 is the perspective view of the outlook of the vibration detecting sensor used in the toilet seat apparatus of Fig. 1, Fig.3 is the perspective view of the total of the toilet to which the toilet seat apparatus shown in Fig.1 is applied, Fig.11 is the sectional view of the essential portion of the toilet seat apparatus of Fig.1, Fig.5 is the side view of the state of using the toilet shown in Fig.3, Fig. 6 is the block constitution diagram of the control unit in the toilet seat apparatus, and Fig.7 is the time chart of the sensor output and the operation of the toilet seat apparatus.

As shown by Fig.1, the toilet seat apparatus 5 according to the embodiment of the invention is constituted by arranging the piezoelectric sensor 9 in the cord-like shape having the flexibility as the vibration detecting sensor to the base plate 7 of the case 8 comprising the upper lid 6 of the rigid body and the base plate 7 molded by resin.

The upper lid 6 forms the upper portion of the case 8 by including the main body portion 10 in the semicircular shape in the sectional view. The lower face of the ceiling plate 11 of the main body portion 10 is attached with the heater 12 for heating. Further, the heater 12 is connected to the control unit 13, mentioned later, and is set to a desired temperature by manual operation.

The base plate 7 forms the lower portion of the case 8 by including the main body portion 14 in the channel-like shape in the sectional view. The piezoelectric sensor 9 is attached to the upper face of the bottom plate 15 of the main body portion 14.

The upper lid 6 and the base plate 7 are integrally integrated by fitting a screw (not illustrated) from the through hole 16 formed at the base plate 7 to the locking portion 17 formed at the upper lid 6. Further, the piezoelectric sensor 9 is connected to the control unit 13 similar to the heater 12.

Here, simply describing the piezoelectric sensor 9 in the cord-like shape used in the embodiment, as shown by Fig.2, the sensor 9 is the sensor in the cable-like shape using the piezoelectric element material and is constituted by the core line (center electrode) 18 arranged at the center in the axial direction, the piezoelectric element material 19 covered at the surrounding of the core line 18, the outer side electrode 20 arranged at the surrounding of the piezoelectric element material 19, and PVC (polyvinyl chloride resin) 21 for covering the outermost periphery.

The piezoelectric sensor 9 uses the piezoelectric element material 19 having the heat resistance capable of withstanding the surrounding temperature of about 120 °C, further, is provided with the flexibility (flexible performance) comparable to that of a normal vinyl cord by using the piezoelectric element material 19 constituted by the resin having the flexibility (flexible performance) and the piezoelectric ceramic and the flexible electrodes.

Further, the piezoelectric sensor 9 is provided with a high sensitivity comparable to that of a polymer piezoelectric element material and achieves a particularly high sensitivity in a low frequency region (10 Hz or lower) for detecting the heart rate of the human body. This is because a reduction in the sensitivity is low even in the low frequency region since the specific inductive capacitance (about 55) of the piezoelectric material 19 is larger than the specific inductive capacitance (about 10) of the polymer piezoelectric element material.

The piezoelectric sensor 9 in the cord-like shape provided in this way as being molded with the piezoelectric element material 19 is not provided with the piezoelectric function and therefore, it is necessary to execute the processing (polarizing processing) for providing the piezoelectric function to the piezoelectric element material 19 by applying several KV/mm of direct current high voltage to the piezoelectric element material 19. The polarizing processing is carried out by applying a direct current voltage between the two electrodes 18, 20 after forming the core line 18 and the outer side electrode 20 at the piezoelectric element material 19.

As shown by Fig.3, the base plate 7 is mounted with 4 pieces of the pads 23 for absorbing impact attached to the rear face of the base plate 15 and having an elastic force for absorbing impact with the toilet main body 22 by being disposed between the toilet seat apparatus 5 and the toilet main body 22 when the toilet seat is used similar to the background art apparatus. Although the pad 23 is a leg portion of the toilet seat apparatus 5, the pad 23 is also a kind of a fixing portion for fixing the toilet seat apparatus 5 of the toilet main body 22, when the user is seated thereon, the body weight is applied thereon and the pad 23 is fixed by being brought into a state of being pressed to the toilet main body 22 harder than the toilet seat. Therefore, when the toilet seat is vibrated by receiving body movement of the user, a vicinity of the pad 23 is maintained in an environment of being vibrated hardly by being fixed to the toilet main body 22.

Further, the toilet seat apparatus 5 is mounted with the lid member 25 lifted up to a side of the water tank 24 along with the toilet seat apparatus 5.

According to the embodiment, the piezoelectric sensor 9 in the cord-like shape is mounted onto the base plate 7 by being positioned and supported by the plurality of holders 26 arranged separately from each other.

When a vicinity of one of the four pads 23 enlarged, as shown by Fig.11, there is constructed the constitution including the pressing means 27 attached to the inner face of the upper lid (rigid body) 6 and having the elasticity, and the projection 28 of the rigid body attached onto the base plate (rigid body) 7 for pinching the piezoelectric sensor 9 by the pressing means 27 and the projection 28. Fig. 11(a) shows a state before integrating the upper lid 6 and the base plate 7 and Fig.11(b) shows a state of integrating the upper lid 6 and the base plate 7 by screwing the upper lid 6 and the base plate 7. When the user is seated on the toilet seat apparatus 5, naturally, the state of Fig.11(b) is brought about, the upper lid 6 and the base plate 7 are constituted by the rigid bodies such that the toilet seat apparatus 5 is not destructed even when body weight is applied thereon.

Here, the rigid body is defined as a member which is not deformed to exceed strength even when at least body weight of a person is applied thereon, particularly a member in which when the person is seated thereon, the person does not feel deformation by which the hip portion sinks, that is, the member by which the user does not feel anxiety for the strength. Although a material is not particularly limited but may be an insulating member of resin, ceramic or the like or may be a conductor of a metal or the like, it is added that the toilet seat of the background art is generally made of resin.

Now, in Fig.11 (b), when the user is seated thereon, the pad 23 constitutes the fixing portion which is hardly vibrated by being fixed to the toilet main body 22, both of the base plate 7 connected to the pad 23 and the projection 28 are constituted by the rigid bodies and therefore, the base plate 7 and the projection 28 are difficult to be vibrated and can support the piezoelectric sensor 9 in a state of being difficult to vibrate the piezoelectric sensor 9 from a lower face thereof. However, since the shape of the base plate 7 is large, the remoter from the pad 23, the more liable to be vibrated. On the other hand, it seems that since the upper lid (rigid body) 6 is brought into direct contact with the user, vibration is easy to be transmitted in the upper lid (rigid body) 6 and the upper lid 6 is vibrated in accordance with body movement and is constructed by a constitution of transmitting vibration to the upper face of the piezoelectric sensor 9 via the pressing means 27. Therefore, the piezoelectric sensor 9 can accurately detect vibration since the piezoelectric sensor 9 receives vibration of the upper lid (rigid body) 6 from the upper face while maintaining the lower face in a state of being difficult to be vibrated. Particularly, the piezoelectric sensor 9 used in the embodiment generates a signal in accordance with acceleration of deformation provided to the piezoelectric element material and therefore, by vibrating only the upper face without vibrating the lower face, acceleration of deformation of effectively contracting and elongating the piezoelectric element material can be received and the large output signal can be generated.

Now, in the state in which the user is seated on the toilet seat, although total body weight of the user is applied on the toilet seat, the upper lid 6 is hardly deformed partially since the upper lid 6 is constituted by the rigid body. However, it seems that a total of the toilet seat is vibrated by body movement of the user although vibration is small. With regard to how vibration of the toilet seat is amplified and transmitted to the piezoelectric sensor 9, a detailed explanation will be given as follows.

First, since the pressing means 27 is disposed between the upper lid 6 and the piezoelectric sensor 9, the pressing means 27 is vibrated by vibrating the upper lid 6, a behavior of the vibration becomes considerably complicated. Whereas the upper lid 6 is constituted by the rigid body, the pressing means 27 is constituted by a member having an elasticity and therefore, whereas the upper portion of the pressing means 27 (vicinity of a portion thereof connected to the upper lid 6) is vibrated similar to the upper lid 6, a lower portion (vicinity of a portion thereof connected to the piezoelectric sensor 9) of the pressing means 27 is vibrated slightly delayedly to repeat vibration different from that of the upper lid 6. Therefore, the piezoelectric sensor 9 is transmitted not only with the vibration of the upper lid 6 as the rigid body but also different vibration by the pressing means 27 and the pressing means 27 amplifies so-to-speak vibration of the upper lid 6. Therefrom, the pressing means 27 can be regarded as a kind of amplifying means.

Next, the pressing means 27 and the piezoelectric sensor 9 are opposed to each other by shapes different from each other. There is constructed the constitution in which the piezoelectric sensor 9 is longer in the left and right direction of Fig.11 and the pressing means 27 is larger in the depth direction of Fig.11 and there is constructed a constitution in which whereas the piezoelectric sensor 9 is opposed thereto by the curved face owing to the cable-like shape, the pressing means 27 is opposed thereto by the plane. Therefrom, the piezoelectric sensor 9 is provided with a portion which is not brought into contact with the pressing means 27 and a portion which is brought into contact with the pressing means 27, further, in the portion brought into contact with the pressing means 27, there are present various portions having different states of being pressed from a portion which is strongly pressed to a portion which is not pressed so much. Therefore, according to the piezoelectric sensor 9, vibration differs by the portions such that the piezoelectric sensor 9 does not receive so much vibration at the portion which is not brought into contact with the pressing means 27 and the piezoelectric sensor 9 receives vibration strongly at the portion of being strongly pressed by the pressing means 27. That a total of the piezoelectric sensor does not receive the same vibration but receives vibration which differs by the portions is equal to that the piezoelectric sensor 9 is partially deformed and therefore, the output of the piezoelectric sensor can be increased. As a result, since the piezoelectric sensor 9 can be deformed from vibration of the rigid body which is hardly deformed, vibration of the rigid body is amplified. Therefrom, the difference of the shapes of the faces of the pressing means 27 and the piezoelectric sensor 9 opposed to each other can be regarded as a kind of amplifying means.

Next, the projection 28 and the piezoelectric sensor 9 are opposed to each other by shapes different from each other. Therefrom, the piezoelectric sensor 9 is provided with a portion which is not brought into contact with the projection 28 and a portion which is brought into contact with the projection 28. Therefore, the piezoelectric sensor 9 is not so much fixed at the portion brought into contact with the projection 28 and is liable to receive vibration from the pressing means 27 and is fixed to the pad 23 via the base plate 7 at the portion brought into contact with the projection 28 and is difficult to receive vibration from the pressing means 27. That is, the piezoelectric sensor 9 receives and does not receive vibration depending on the portions. That a total of the piezoelectric sensor does not receive the same vibration but receive vibration or does not receive vibration depending on the portions is equal to that the piezoelectric sensor 9 is partially deformed and therefore, the output of the piezoelectric sensor can be increased. As a result, the piezoelectric sensor 9 can be deformed from vibration of the rigid body which is hardly deformed although the vibration is small and therefore, vibration of the rigid body is amplified. Therefrom, the difference between the shapes of the faces of the projection 28 and the piezoelectric sensor 9 opposed to each other can be regarded as a kind of amplifying means. However, it seems that an effect of amplification by the projection 28 is smaller than an effect of amplification of the above-described pressing means 27.

Next, the pressing means 27 and the projection 28 are opposed to each other by the different shapes and particularly, the area of the pressing means 27 is larger. Since the projection 28 is constituted by the rigid body, only the center portion of the pressing means 27 is compressed, at the compressed center portion, the elasticity of the pressing means 27 is hampered and therefore, vibration near to that of the upper lid 6 is carried out. Further, a force is exerted to the piezoelectric sensor 9 by the pressing means 27 and the projection 28 and the piezoelectric sensor 9 is fixed to some degree. The magnitude of the force is changed by the elasticity of the pressing means 27 and the distance between the upper lid 6 and the base plate 7 when the upper lid 6 and the base plate 7 are integrated. When the elasticity of the pressing means 17 is low and when the distance between the upper lid 6 and the base plate 6 is short, the piezoelectric sensor 9 is further exerted with the force and is fixed firmly. On the other hand, on the side of the surrounding of the pressing means 27 (a portion to which the projection 28 is not opposed), the elasticity is maintained and therefore, vibration different from that of the upper lid 6 can be carried out. Therefore, the piezoelectric sensor 9 executes vibration near to that of the upper lid 6 at the center portion of the pressing means 27 and executes vibration different from that of the upper lid 6 only by being pressed by the pressing means 27 at a side of surrounding (a portion to which the projection 28 is not opposed) of the pressing means 27. At this occasion, a point which requires caution is that a portion executing vibration near to that of the upper lid 6 and the portion executing vibration different from that of the upper lid 6 are disposed at positions extremely proximate to each other in the piezoelectric sensor 9. That the piezoelectric sensor 9 receives vibrations different from each other at positions extremely proximate to each other is equal to that the piezoelectric sensor 9 is locally deformed and therefore, the output of the piezoelectric sensor can be increased. As a result, the piezoelectric sensor 9 can be deformed from vibration of the rigid body which is hardly deformed although the deformation is small and therefore, vibration of the rigid body is amplified. Therefrom, a difference between the shapes of the faces of the pressing means 27 and the projection 28 opposed to each other can be regarded as a kind of amplifying means.

In this case, the projection 28 may not be the rigid body. Because even when the projection 28 is constituted by an elastic body, by increasing the height of the pressing means 27 or the projection 28, or shortening the distance between the upper lid 6 and the base plate 7, by strongly compressing the pressing means 27 at the center portion, the elasticity can be hampered.

Here, with regard to an elasticity of the pressing means 27, the elasticity may preferably be higher than that of the upper lid 6, the above-described effect is achieved as amplifying means. For example, a representative cushion material may be used or cover, sponge or the like may be used.

According to the toilet seat apparatus 5 having the above-described constitution, as shown by Fig.5, when the toilet seat apparatus 5 is mounted on the toilet main body 22 and weight of the human body M is applied thereon by being seated in using the toilet seat apparatus 5, as described above, the piezoelectric sensor 9 is pressed. As a result, vibration in accordance with movement of the human body M is amplified and applied on the piezoelectric sensor 9 and the electric signal is firmly outputted.

The electric signal provided in accordance with the acceleration of vibration is supplied to the control unit 13.

However, the electric signal outputted from the piezoelectric sensor 9 is masked by the control unit 13 such that vibration produced by driving a cleaning nozzle or operating a blower, or flushing water or the like in using the toilet seat apparatus 5 does not constitute noise for detecting presence/absence, the heart rate or the like of the human body.

As shown by Fig. 6, the control unit 13 includes a filter circuit, amplifying means, smoothing means, determining means and the like, not illustrated, at inside of the control portion 29 and is mounted with the heart rate calculating means 30 for calculating the heart rate based on the output signal of the smoothing means, the displaying means 31 for displaying the output signal of the heart rate calculating means 30, the comparing means 32 for comparing the calculated output of the heart rate calculating means 30 and the set value and the alarm issuing means 33 for issuing the alarm based on the result of comparison and is inputted with the detected signal of the piezoelectric sensor 9.

When the piezoelectric sensor 9 detects body movement of the human body M and outputs the electric signal, the control unit 13 filters the electric signal by the filter circuit and thereafter, amplifies the electric signal by the amplifying means and smoothes the electric signal by the smoothing means. As shown by Fig.7, a large output waveform is outputted from the smoothing means at an instance at which the human body M is seated on the toilet seat apparatus 5, when an article is mounted thereon, or when the body is moved. On the other hand, in the still state after the human body M is seated thereon, the output waveform of the comparative low level is outputted from the smoothing means by small body movement of the body propagated by action of the heart and the respiratory action.

In contrast thereto, when the human body M is not present or when the article is mounted thereon, the output waveform is not shown in a constant period of time after the smoothing means outputs a large output waveform.

Hence, the determining means compares to determine as follows by the output V of the smoothing means and the previously determined two set values Va, Vb. That is, when V < Va, it is determined that the human body M of the article is not present (unpresent output Hi). When Va ≦ V < Vb, it is determined that the human body M is present in the still state (present output Hi). Further, when Vb < V, it is determined that body movement is brought about by the human body M (body movement output Hi). When the article is mounted thereon in place of the human body M, although determination of seating or body movement is temporarily carried out, unpresence of the human body M is determined by determining a state of placing the article since vibration of low level propagated by action of the heart or the respiratory action of the human body M does not appear.

When the determining means determines seating of the person, the deodorizing means and the heating means start to be operated. The operation is stopped when unpresence of the person is determined.

Further, the heating means governs temperature control of the heater 12.

Further, when the human body M is seated thereon in the still state, a small signal propagated by action of the heart is outputted from the smoothing means. Based on the signal, the heart rate calculating means 30 calculates to output the heart rate. A result of the calculation is displayed by the displaying means 31 which is an outside monitor. Further, as shown by Fig.6, the control unit 13 is mounted with the comparing means 32 for comparing the output signal of the heart rate calculating means 30 and the previously determining set value, and the alarm issuing means 33 for issuing the alarm by the output of the comparing means 32 and can issue the alarm when the heart rate becomes equal to or larger than the set value. Particularly, when a person strains in evacuation, the heart rate is increased and there is a concern of bringing about occurrence of the cerebral hemorrhage, the control unit 13 can contribute to health control by foreseeing the attack. At this occasion, when the control unit 13 is connected by a network in a hospital or the like, not only the toilet seat apparatus 5 used in the hospital can centrally be monitored summarizingly but also a behavior in a rest room which cannot directly be diagnosed can always be monitored.

Further, a signal to the displaying means 31 can be transmitted via communicating means by wired means or wireless means.

According to the above-described toilet seat apparatus 5, the piezoelectric sensor 9 can achieve high reliability by easily detecting even slight movement of the human body by supplying the electric signal in accordance with the acceleration of vibration firmly to the control unit 13. Further, the piezoelectric sensor 9 is provided with the flexibility and is difficult to be destructed even when impact continues applying thereto, further, outputs the detected signal facilitating to differentiate the person and the article and therefore, the piezoelectric sensor 9 can firmly detect seating of the person or the like.

Further, although according to the above-described embodiment, presence/absence, the heart rate or the like is determined by smoothing the output signal from the piezoelectric sensor 9, there may be constructed a constitution in which the output signal of the piezoelectric sensor 9 is amplified as necessary and thereafter, converted into digital data by AD conversion by a microcomputer or the like, presence/absence is determined based on a value constituted by subjecting the digital data to a moving average in the microcomputer, or the heart rate or the like is calculated by calculating the autocorrelation coefficient of the above-described digital data.

The effect of the embodiment described above will be summarized. The vibration detecting apparatus 34 is formed by constructing the constitution of supporting the piezoelectric sensor (vibration detecting sensor) 9 for detecting vibration transmitted to the upper lid (rigid body) 6 or the base plate (rigid body) 7 of the toilet seat apparatus 5 at the vicinity of the pad (fixing portion) 23 for fixing the toilet seat apparatus 5 to the toilet main body.

Thereby, vibration is difficult to be brought about the most at a vicinity of the pad (leg portion (fixing portion)) 23 and therefore, the piezoelectric sensor (vibration detecting sensor) 9 supported at the vicinity of the pad (leg portion (fixing portion)) 23 via the projection 28 can be prevented from being vibrated at at least the supported portion. Therefore, vibration transmitted to the upper lid (rigid body) 6 can accurately be detected in an environment in which the piezoelectric sensor (vibration detecting sensor) 9 per se is not vibrated.

Further, there is constructed the constitution including four of the pads (leg portions (fixing portions) 23 and supporting the piezoelectric sensor (vibration detecting sensor) 9 by the vicinities of the respective pads.

Thereby, even when the function of restraining vibration differs in four of the pads (leg portions (fixing portions)) 23, the piezoelectric sensor (vibration detecting sensor) 9 is supported also by the vicinity of the pad (leg portion (fixing portion)) having the highest function of restraining vibration and therefore, vibration transmitted to the upper lid (rigid body) 6 can accurately be detected. For example, when the user stoops and a gravitational center thereof is inclined forward, it is conceivable that vibration is difficult to be restrained since the pad on the rear side is not so much applied with the body weight, also in this case, the body weight is applied on the pad of the front side and therefore, vibration can be restrained. As a result, when the sensors are supported by all the pads, regardless of where the gravitational center is disposed, there is present necessarily at least one or more of the pads capable of restraining vibration by being applied with the body weight and therefore, at the pad, vibration of the piezoelectric sensor (vibration detecting sensor) 9 can be restrained and vibration can accurately be detected. Incidentally, when the piezoelectric sensor in the cable-like shape is used as in the embodiment, even when a plurality of fixing portions of pads or the like are present, the sensor can be constituted by a single one thereof.

Further, the piezoelectric sensor (vibration detecting sensor) 9 is constituted on a side of a vibration source (on a side of the upper lid) 6 of the pad (fixing portion) 23.

Thereby, vibration of the upper lid 6 can be detected on the side of the vibration source (side of the upper lid 6) while preventing vibration on the side of the pad (leg portion (fixing portion)) 23 of the piezoelectric sensor (vibration detecting sensor) 9 and therefore, vibration transmitted to the upper lid 6 can accurately be detected by a simple constitution to a degree of having the pressing means 27 and the projection 28.

Further, the vibration detecting sensor is constituted by the piezoelectric sensor 9 having the flexibility.

Thereby, the piezoelectric sensor 9 is easily deformed when the piezoelectric sensor 9 receives vibration and generates the output in accordance with the deformation and therefore, the output in accordance with vibration can be outputted accurately.

Further, in the toilet seat apparatus 5 having the vibration detecting apparatus 34, there is constructed the constitution in which the rigid body is constituted by the toilet seat (upper lid 6, base plate 7), the fixing portion is constituted by the pad (leg portion (fixing portion)) 23 attached to the lower face (base plate) 7 of the toilet seat and capable of fixing the toilet seat by being brought into contact with the upper face of the toilet main body 22 and the body movement of the user transmitted to the toilet seat is detected.

Thereby, the base plate 7 of the toilet seat apparatus 5 is fixed to the toilet main body 22 at a vicinity of the pad 23 and is difficult to be vibrated the most and therefore, the piezoelectric sensor (vibration detecting sensor) 9 supported by the vicinity of the pad 23 can be prevented from being vibrated at at least the supported portion. Therefore, the body movement of the user transmitted to the upper lid 6 of the toilet seat apparatus 5 in an embodiment in which the piezoelectric sensor (vibration detecting sensor) 9 per se is not vibrated can accurately be detected.

Further, in the toilet seat apparatus 5, there is constructed the constitution of detecting at least one of the seating, the heart beat, the respiration from the body movement of the user.

Thereby, based on body movement which is detected accurately, it is easy to detect motion information of the seating, that is, whether the user is seated thereon, or biologic information of the heart beat or the respiration of the user and the multifunction toilet seat apparatus 5 can be realized by making full use of detected information.

Further, an explanation will be given of other example of mounting the vibration detecting sensor at inside of the rigid body (toilet seat) and constituting the fixing portion at outside of the rigid body while comparing with that in Fig.11.

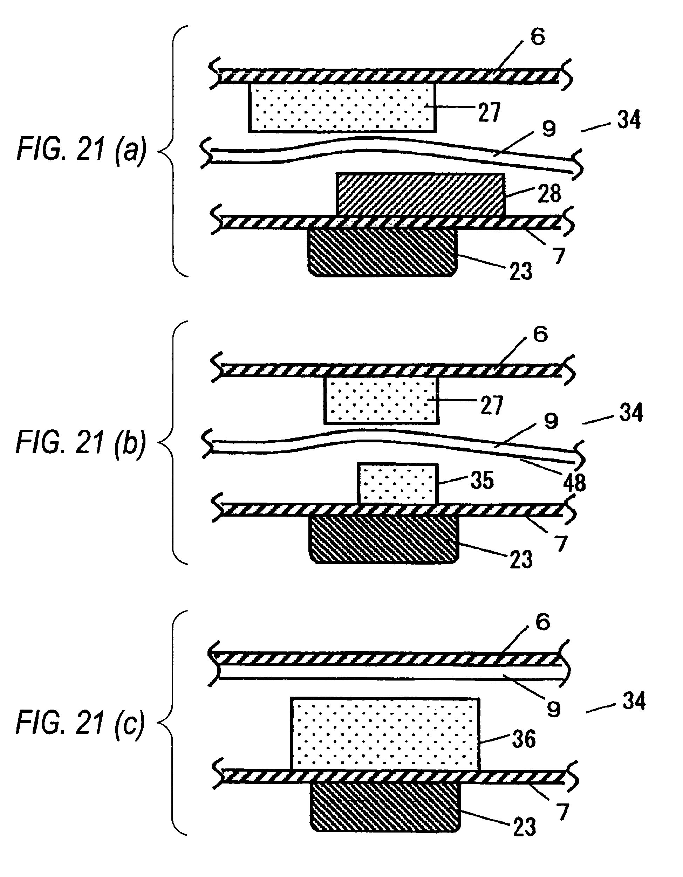

First, Fig.21 shows examples of a different arrangements. Fig.21 (a) shows a constitution of arranging the pressing means 27 and the projection 28 at respective positions, the pressing means 27 is arranged to be shifted to the left side of the pad 23 and the projection 28 is arranged to be shifted to the right side of the pad 23, respectively. Fig.21(b) shows a constitution of using the pressing means 35 having an elasticity in place of the projection of the rigid body and positions of right ends of the pressing means 27 and the pressing means 35 are aligned and positions of left ends thereof are shifted from each other. Further, the pad 23 is constituted by a shape larger than those of the pressing means 27, the pressing means 35 at this occasion. In Fig.21(c), the piezoelectric sensor (vibration detecting sensor) 9 is disposed on a side of the upper lid (rigid body) 6 near to the pressing means 36 and is particularly fixed directly to the upper lid (rigid body) 6. In this case, there is constructed the constitution in which a total of the upper face of the piezoelectric sensor 9 is vibrated in accordance with the upper lid 6 and vibration of only a portion (portion pressed by the pressing means 36) of the lower face of the piezoelectric sensor 9 is hampered.

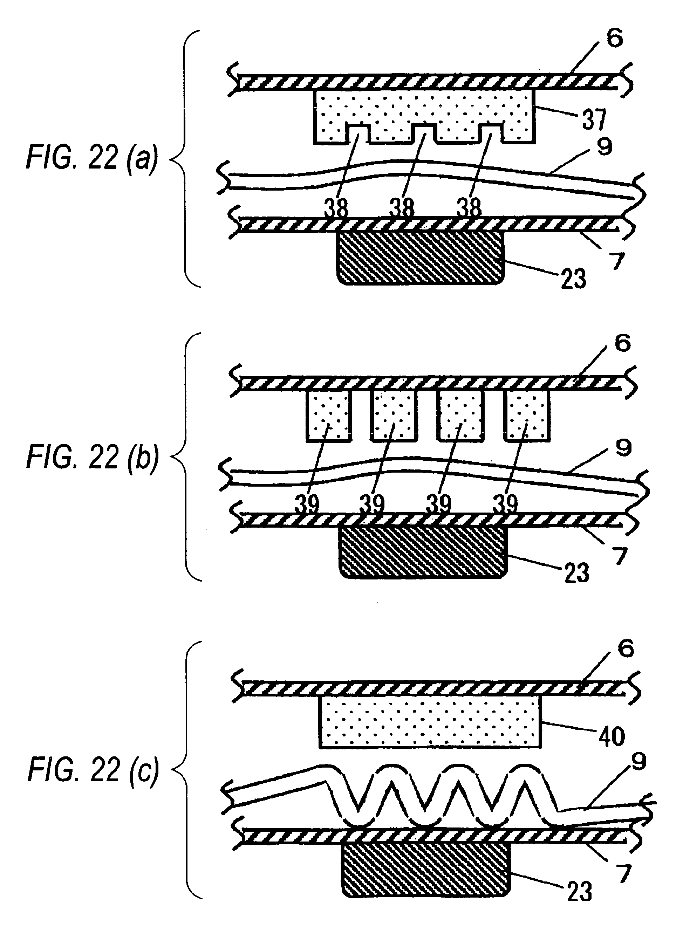

Fig.22 shows examples in which the shapes of faces of the pressing means of the piezoelectric sensor (vibration detecting sensor) opposed to each other differ from each other. In Fig.22(a), the recess portion 38 is formed at the pressing means 37. Naturally, the projected portion may be provided. In Fig.22(b), a plurality of pressing means 39 are provided. In Fig.22(c), the pressing means 40 is opposed thereto by the flat face and the piezoelectric sensor (vibration detecting sensor) 9 is bent.

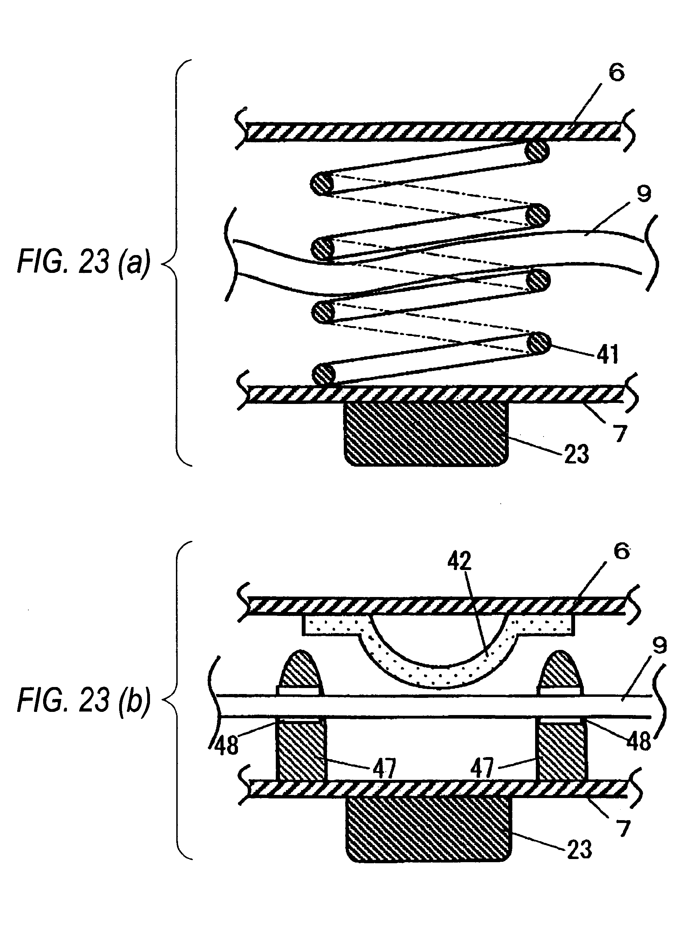

Fig.23 shows other examples of pressing means having an elasticity. In Fig.23(a), a spring is used as the pressing means 41. The pressing means 42 of Fig.23(b) is provided with an elasticity by bending and fixing a thin spring material made of a metal.

### (Embodiment 8)

According to the embodiment, an explanation will be given of an example of mounting a vibration detecting sensor between a rigid body (bath tub) and a fixing portion.

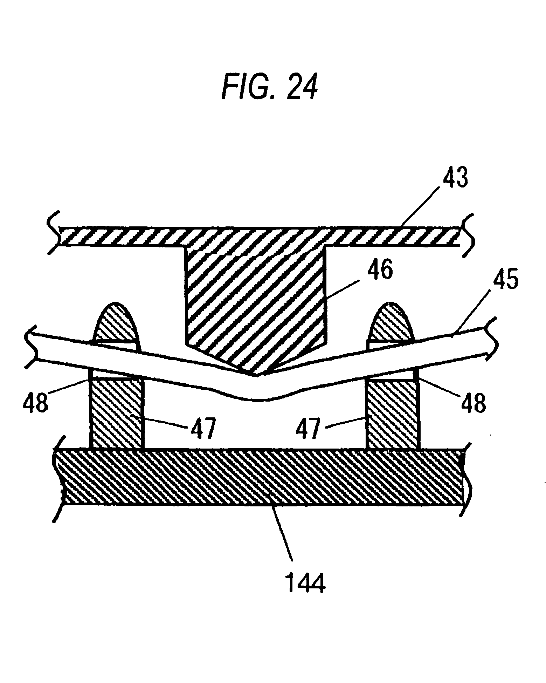

Fig.15(b) is a constitution view of a section of a bath tub apparatus showing an eighth embodiment according to the invention and Fig.24 is a sectional view of an essential portion of Fig.15(b).

As shown by Fig.15(b), the bath tub apparatus according to the embodiment of the invention is arranged with the piezoelectric sensor 45 in the cord-like shape having a flexibility as a vibration detecting sensor between the bath tub 43 of the rigid body and an outer frame 144 as a fixing portion which is heavier and thicker than the bath tub 43 and is difficult to be transmitted with vibration. Further, there is provided the holder 47 for supporting the projection 46 projected from the bath tub 43 to the piezoelectric sensor 45, the piezoelectric sensor 45 at the outer frame (fixing portion) 144 and the piezoelectric sensor 45 is positioned via the hole 48 of the holder 47.

Here, the projection 46 and the piezoelectric sensor 45 are opposed to each other by shapes different from each other. There is constructed the constitution in which the piezoelectric sensor 9 is longer in the left and right direction of Fig.24 and the projection 46 is larger in the depth direction of Fig.24, further, whereas although the piezoelectric sensor 9 is provided with a curved face owing to the cable-like shape, the piezoelectric sensor 9 is opposed thereto by the constant shape in the left and right direction, the projection 46 is constituted by the shape in which a center portion in the left and right direction is further projected. Therefrom, the piezoelectric sensor 45 is provided with the portion which is not brought into contact with the projection 46 and the portion which is brought into contact with the projection 46, further, in the portion brought into contact with the projection 46, there are present various portions having different states of being pressed from a portion (center) which is pressed strongly to a portion which is not pressed so much. From the above-described, vibration of the bath tub 43 is transmitted to the piezoelectric sensor 45 via the projection 46.

On the other hand, the piezoelectric sensor 45 is attached to the outer frame (fixing portion) 144 via the holder 47. There is constructed a constitution in which the outer frame (fixing portion) 144 is brought into contact with the bath tub 43 only at an edge thereof and even when vibration is brought about at inside of the bath tub 43, vibration is not transmitted to the outer frame (fixing portion) 144. Therefore, a portion of the piezoelectric sensor 45 supported by the holder 47 is difficult to be vibrated since the outer frame (fixing portion) 144 is not vibrated. Therefore, vibration can accurately be detected since the piezoelectric sensor 45 receives vibration of the bath tub 43 from a face thereof brought into contact with the projection 46 while maintaining a face thereof brought into contact with the holder 47 in a state of being difficult to be vibrated. Particularly, the piezoelectric sensor 9 used in the embodiment generates a signal in accordance with acceleration of deformation provided to the piezoelectric element material and therefore, by vibrating only the projection 46 without vibrating the face brought into contact with the holder 47, acceleration of deformation of effectively contracting or elongating the piezoelectric element material can be received and a large output signal can be generated.

Now, the embodiment includes other constitution having a function of amplifying vibration.

First, there is provided the elasticity of the piezoelectric sensor 45. The piezoelectric sensor 45 having flexibility (flexible performance) is also provided with the elasticity and therefore, vibration from the projection 46 brought into contact with the piezoelectric sensor 45 can vibrate the piezoelectric sensor 45 per se and particularly, vibration different from the original vibration can be produced by elasticity of the piezoelectric sensor 45. That is, there is constituted a mechanism in which while generating an output of the provided original vibration, the other output is generated by producing different vibration by the piezoelectric sensor 45 per se.

Next, the piezoelectric sensor 45 is mounted in a state of being applied with the tension. In Fig.24, the piezoelectric sensor 45 is supported in a state of applying the tension, that is, pulling the piezoelectric sensor 45 tightly. At this occasion, in comparison with the case in which the piezoelectric sensor 45 is not applied with the tension, vibration is propagated remotely in the piezoelectric sensor 45. Vibration from the projection 46 brought into contact with the piezoelectric sensor 45 is propagated remotely in the piezoelectric sensor 45 when the piezoelectric sensor 45 is applied with the tension to some degree, as a result, the output can be generated from various locations in the piezoelectric sensor 45. According thereto, it is conceived that an effect the same as that in increasing the sensitivity of the sensor, that is, amplifying the vibration is achieved. Further, with regard to what degree of the tension is preferably applied, it is preferable to constitute a range in which at least the mechanical strength of the piezoelectric sensor 45 is not deteriorated, particularly, in a range of capable of maintaining the elasticity.

Fig.17(b) shows other example of mounting the vibration detecting sensor between the rigid body (bath tub) and the fixing portion.

In Fig.17(b), the projection 49 is constituted on the side of the outer frame 144 and the holder 47 is constituted on the side of the bath tub 43.

Further, although according to the embodiment, the bath tub is shown as the rigid body, the bath tub is constituted by various materials when viewed historically. There are materials comprising wood, resin, a metal of stainless steel and the like, marble, rock, tile and the like. The rigid body for the bath tub can be adopted so far as the rigid body is constituted by a material which does not produce large deformation which can be sensed by the user in normal bathing and in which vibration is transmitted.

Further, with regard to the outer frame as the fixing portion, although the outer frame does not transmit vibration since the outer frame comprises the rigid body heavier and thicker than the bath tub, it is conceivable to constitute the outer frame which is not brought into contact with the bath tub. When the outer frame is not brought into contact with the bath tub, vibration is not transmitted and therefore, it is not necessary to restrict the material to be heavy and thick.

### (Embodiment 9)

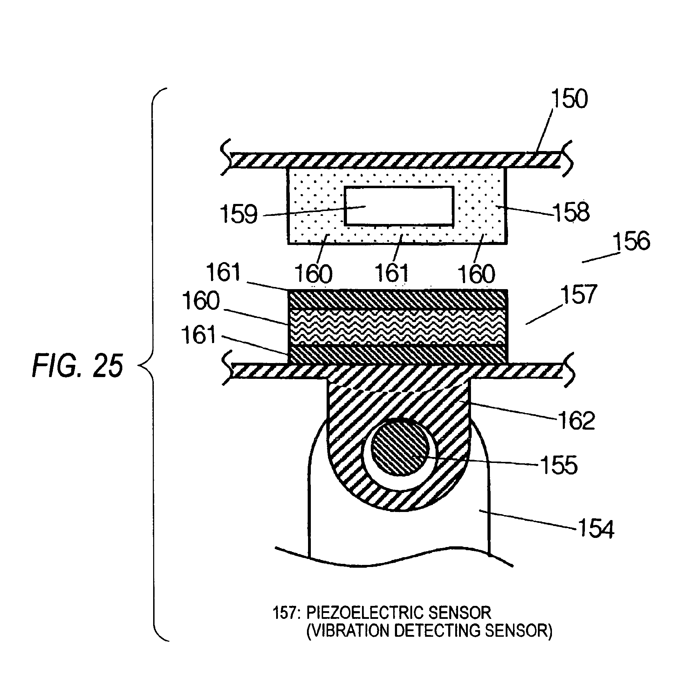

According to the embodiment, an explanation will be given of an example of mounting the vibration detecting sensor to inside of a rigid body (seat) and constituting a fixing portion of a movable type at outside of the rigid body. Fig.18(b) is a constitution view of a shower apparatus showing a ninth embodiment according to the invention and Fig.25 is a sectional view of an essential portion of Fig.18(b).

According to the shower apparatus of the embodiment according to the invention, as shown by Fig. 18(b), shower can be used in a seated attitude and a person can be splashed with hot water injected from a plurality of shower nozzles 151 in a state of being seated on a seat 150. The seat 150 is constituted to be rotatable centering on a shaft 153 relative to a shower apparatus main body 152, a support plate (fixing portion) 154 is constituted to be rotatable centering on a shaft 155 relative to the seat 150 and is constituted to support the seat 150 by the support plate (fixing portion) 154 as shown by Fig.18(b) when used and fold the support plate 154 by lifting up the seat 150 to put away to be in parallel with the shower apparatus main body 152 when not used.

A vibration detecting apparatus 156 is integrated to inside of the seat 150 of the rigid body, and the vibration detection apparatus 156 includes a piezoelectric sensor 157 in a sheet-like shape having an elasticity as a vibration detecting sensor and pressing means 158 having an elasticity as amplifying means. As shown by Fig.25, even when shapes of faces of the pressing means 158 and the piezoelectric sensor 157 opposed to each other are flat to be equal to each other, an effect of amplification is enlarged by constructing a constitution having a cavity 159 at a center of the pressing means 158. Considering the pressing portion 160 for pressing the piezoelectric sensor 157 without interposing the cavity 159 and the pressing portion 161 for pressing the piezoelectric sensor 157 by interposing the cavity 159, although the piezoelectric sensor 157 is pressed strongly from the pressing portion 160, the piezoelectric sensor 157 is weakly pressed by the pressing portion 161, which can be conceived to construct a constitution having a function similar to that in Figs.22(a) (b).

That is, at the piezoelectric sensor 157, there are a portion of receiving strong vibration by being pressed strongly by the pressing portion 160 and a portion which does not receive vibration so much without being pressed so much by the pressing portion 161. That a total of the piezoelectric sensor 157 does not receive the same vibration but receives different vibration depending on the portions is equal to that the piezoelectric sensor 157 is partially deformed and therefore, the output of the piezoelectric sensor 157 can be increased. As a result, the piezoelectric sensor 157 can be deformed from vibration of the rigid body (seat 150) which is hardly deformed and therefore, vibration of the rigid body is amplified. As a result, the pressing means 158 can amplify vibration further by the cavity 159, the pressing portions 160, 161.

Further, the support plate 154 is transmitted with a load applied on the seat 150 via the shaft 155 and therefore, there is constructed a constitution in which the support plate 154 is firmly fixed between the seat 150 and a floor face and is hardly vibrated. Further, there is constructed a constitution in which also a bearing 162 connected to the shaft 155 is strongly pressed to the shaft 155 and therefore, the bearing 162 is hardly vibrated, as a result, also the floor face of the piezoelectric sensor 157 is hardly vibrated.

Therefore, the piezoelectric sensor 157 can accurately detect vibration since vibration of the seat 150 is received from a face thereof brought into contact with the pressing means 158 while maintaining a face on a side of the bearing 152 in a state of being difficult to be vibrated.

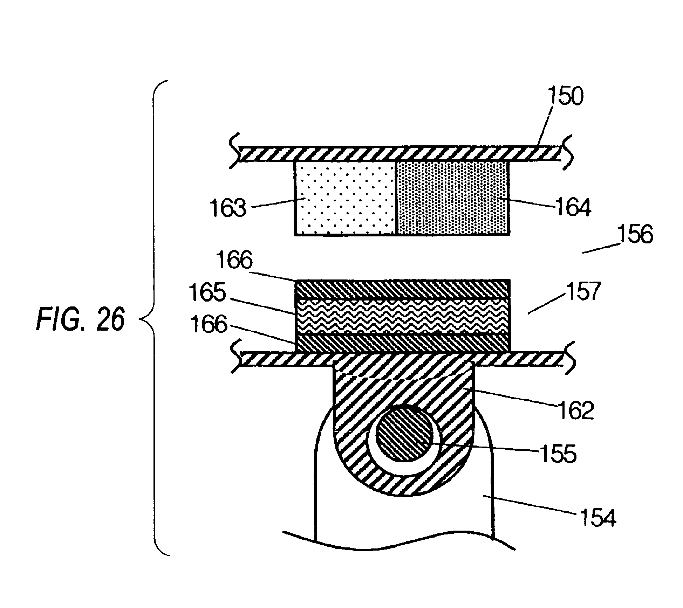

Further, Fig.26 shows other example of mounting the vibration detecting sensor at inside of the rigid body (seat) and constituting a fixing portion of a movable type at outside of the rigid body.

In Fig.26, there are provided first pressing means 163 and second pressing means 164 having different elasticities as pressing means for transmitting different vibrations to a face of the piezoelectric sensor 157 opposed thereto by a difference between the respective elasticities. That a total of the piezoelectric sensor 157 does not receive the same vibration but receives different vibrations depending on the portions is equal to that the piezoelectric sensor 157 is partially deformed and therefore, the output of the piezoelectric sensor 157 can be increased. The piezoelectric sensor 157 can be deformed from vibration of the rigid body (seat 150) which is hardly deformed and therefore, vibration of the rigid body is amplified. As a result, the difference between the elasticities of the pressing means 163 and the second pressing means 164 can be regarded as amplifying means for amplifying vibration of the rigid body (seat 150). Further, the piezoelectric sensor 157 of the embodiment is not constituted by a cable-like shape but is constituted in a sheet-like shape by attaching conductive rubber 166 as electrodes to both faces of the piezoelectric sheet 165 molded by the piezoelectric element material in a sheet-like shape.

### (Embodiment 10)

According to the embodiment, an explanation will be given of an example of mounting a vibration detecting sensor to inside of a rigid body (toilet seat), constituting a fixing portion at outside of the rigid body and supporting the vibration detecting sensor not only at a vicinity of the fixing portion but also at other portion.

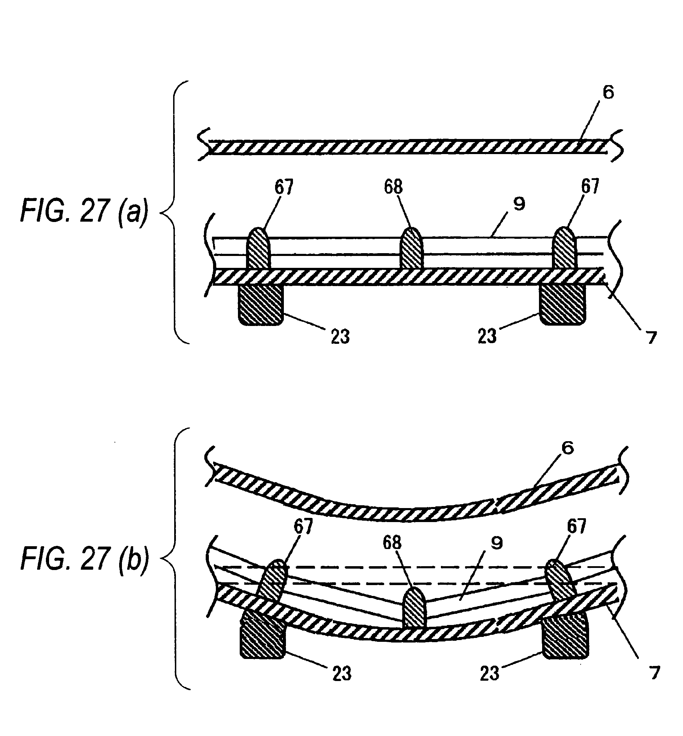

Fig.27 is a constitution view of a section of a toilet seat apparatus in which the piezoelectric sensor (vibration detecting sensor) 9 is fixedly attached onto the pad (fixing portion) 23 and holders 67, 68 formed at positions between the pads. Fig.27(a) shows a state of not being vibrated and Fig.17(b) shows a state in which the upper lid (rigid body) 6, the base plate (rigid body) 7 are bent downward by vibration. Although both of the upper lid 6 and the base plate 7 are constituted by the rigid bodies, it is conceived that microscopically, the upper lid 6 and the base plate 7 are bent as shown by the drawing by body movement of the user. However, the drawing is illustrated exaggeratingly to facilitate to understand and actually, bending is small bending to a degree which the user cannot feel actually.

Now, whereas in Fig.27(b), the holder 68 is pulled down in comparison with that in Fig.27(a) by downwardly bending the base plate (rigid body) 7, the holder 67 is fixed by the pad 23 and therefore, the holder 67 is hardly moved. Therefore, the piezoelectric sensor 9 is pulled to downwardly deform by an amount of moving down the holder 68. Conversely, when the base plate 7 is bent upwardly by vibration, the holder 68 is lifted up, and the piezoelectric sensor 9 is pulled upwardly to deform. When up and down movement is produced by body movement of the user, the piezoelectric sensor 9 repeats deformation of being pulled and loosened at respective times and generates a signal in accordance therewith. It seems that generation of the signal can be realized because the sensor is fixed not only at the vicinity of the pad (fixing portion) 23 but also at the position between the pads. Further, in this case, even when there is not the pressing means from the side of the upper lid 6, vibration can be detected and the vibration detecting apparatus can be constituted independently from the upper lid 6.

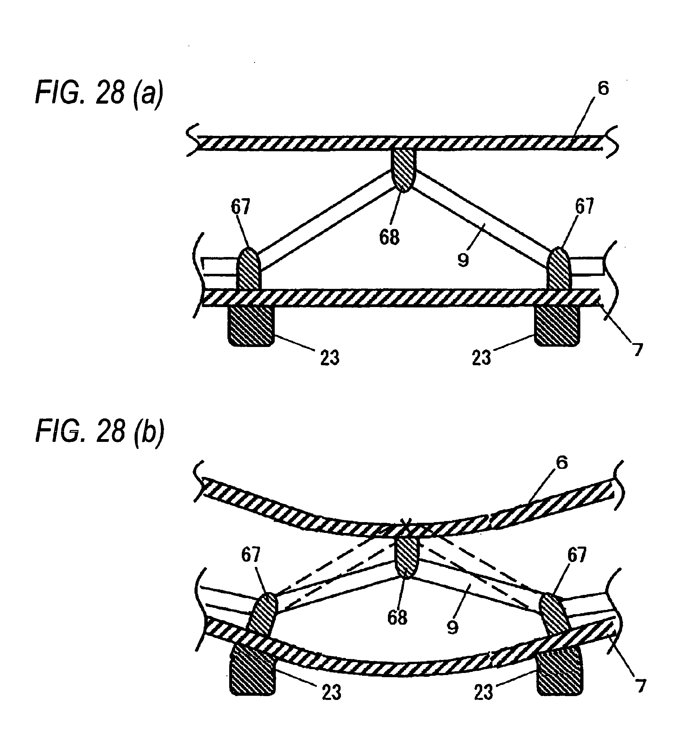

Fig.28 shows an example of constituting the holder 68 to the side of the upper lid (rigid body) 6 in comparison with that in Fig.27 and when bent downwardly as in Fig.28(b), the holder 68 is pulled down, however, the piezoelectric sensor 9 is not only pulled but also added with deformation in a direction of being contracted. That is, there is constructed a constitution in which a direction of deformation is reversed to that in Fig.27.

Further, although in Fig.21, Fig.22, Fig.23, Fig.17(b), Fig.25, Fig.26, there is a gap between the pressing means or the projection and the piezoelectric sensor, similar to Fig. 11(a), a gap is provided for simplifying explanation and it is added that the both members are brought into contact with each other when used actually.

### (Embodiment 11)

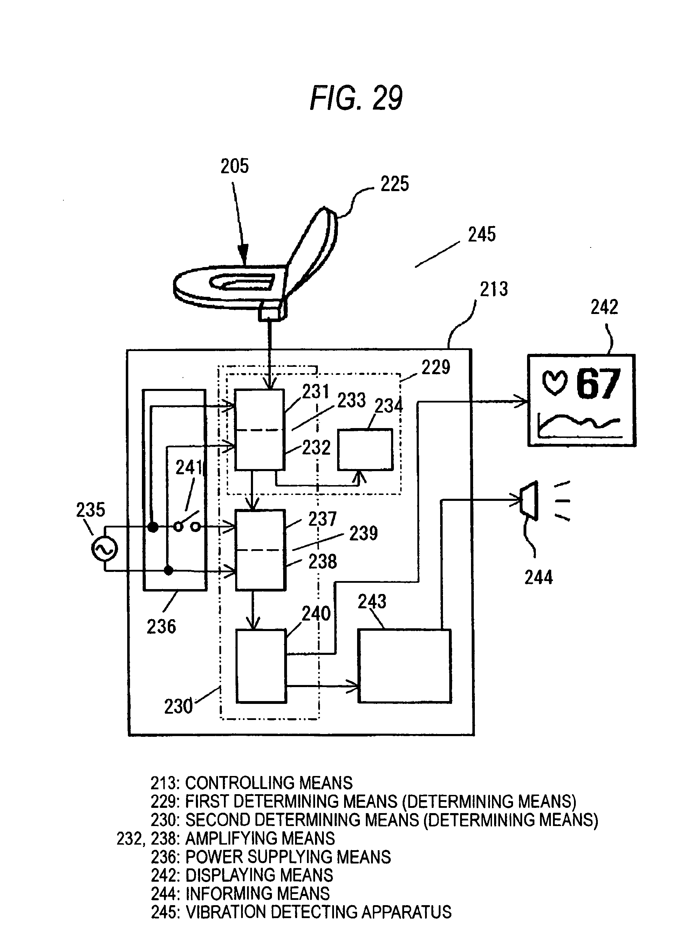

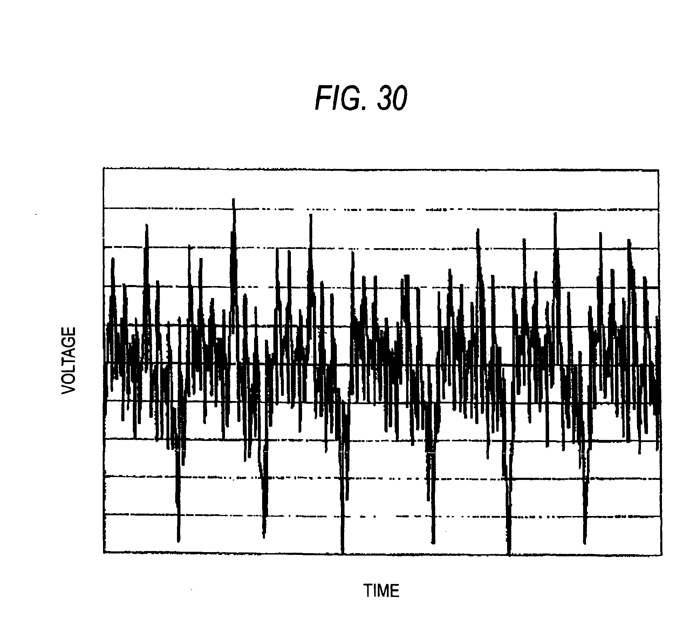

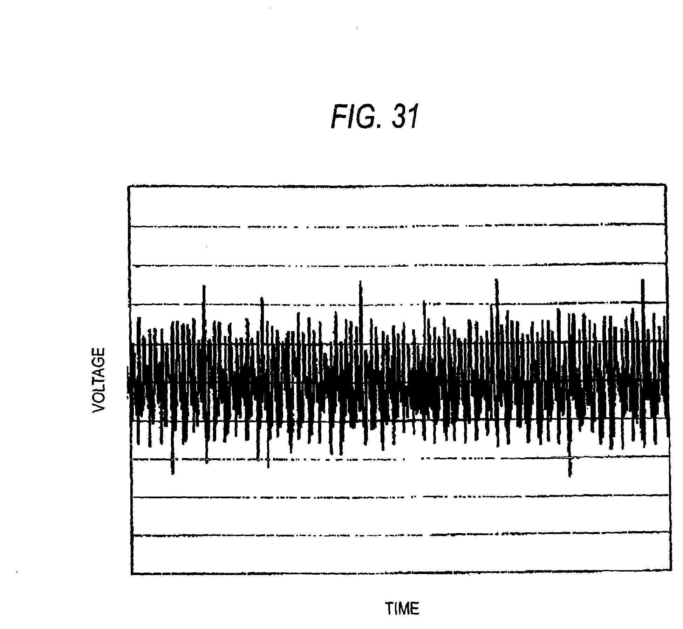

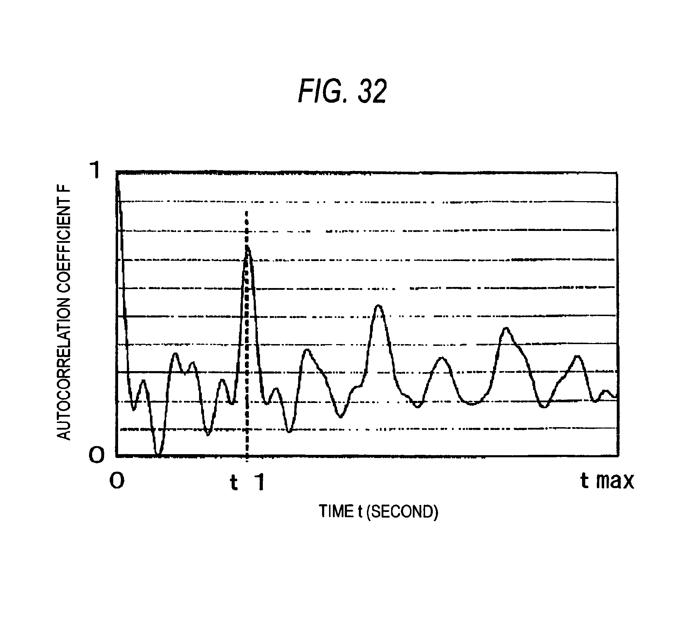

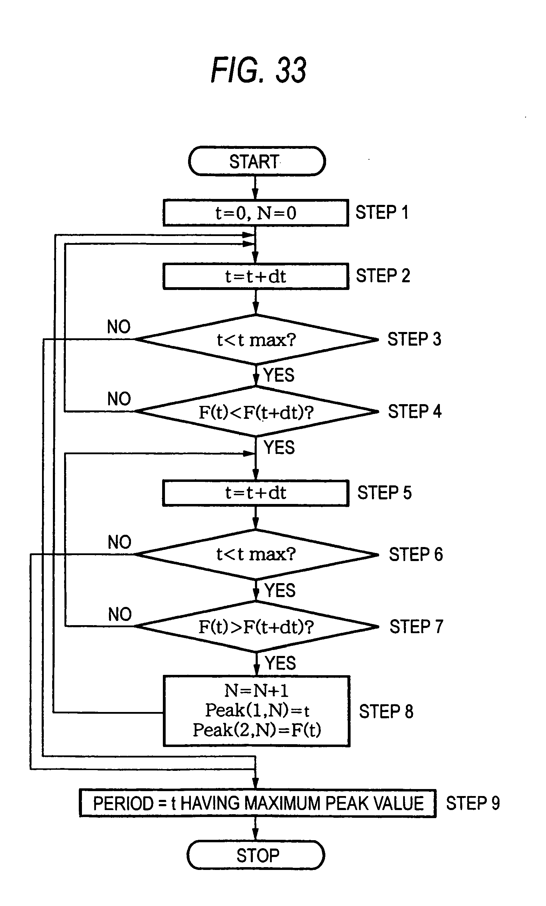

Fig. 1 is a disassembled perspective view of a toilet seat apparatus showing an eleventh embodiment according to the invention, Fig. 2 is a perspective view of an outlook of a vibration detecting sensor used in the toilet seat apparatus, Fig.3 is a perspective view of a total of a toilet to which the toilet seat apparatus shown in Fig.1 is applied, Fig.11 is a sectional view of an essential portion of the toilet seat apparatus of Fig.1, Fig.5 is a side view of a state of using the toilet shown in Fig.3, Fig.29 is a block constitution diagram of a control apparatus in the toilet seat apparatus, Fig.7 is a time chart of a sensor output and operation in the toilet seat apparatus, Fig.30 shows a sensor output in still time, Fig.31 shows a signal constituted by processing a sensor output by a filter, Fig.32 is a characteristic diagram of an autocorrelation coefficient of a signal processed by a filter, Fig.33 is a flowchart for calculating a period of heart rate.

As shown by Fig.1, the toilet seat apparatus 5 of the embodiment according to the invention is constituted by arranging the piezoelectric sensor 9 in the cord-like shape having the elasticity as the vibration detecting sensor to the base plate 7 of the case 8 comprising the upper lid 6 of the rigid body and the base plate 7 molded by resin.

The upper lid 6 forms the upper portion of the case 8 by including the main body portion 10 in the semicircular shape in the sectional view. The heater 12 for heating is attached to the lower face of the ceiling plate 11 of the main body portion 10. Further, the heater 12 is connected to the control means 213, mentioned later, and is set to a desired temperature by manual operation.

The base plate 7 forms the lower portion of the case 8 by including the main body portion 14 in the channel-like shape in the sectional view. The piezoelectric sensor 9 is attached to the upper face of the bottom plate 15 of the main body portion 14.

The upper lid 6 and base plate 7 are integrally integrated by fitting a screw (not illustrated) from the through hole 16 formed at the base plate 7 to the locking portion 17 formed at the upper lid 16. Further, the piezoelectric sensor 9 is connected to the controlling means 213 similar to the heater 12.

Here, simply explaining of the piezoelectric sensor 9 in the cord-like shape used in the embodiment, as shown by Fig.2, the sensor 9 is the sensor in the cable-like shape using the piezoelectric element material and is constituted by the core line (center electrode) 18 arranged at the center in the axial direction, the piezoelectric element material 19 covered to the surrounding of the core line 18, the outer side electrode 20 arranged at the surrounding of the piezoelectric material 19, and PVC (polyvinyl chloride resin) 21 for covering the outermost periphery.

The piezoelectric sensor 9 uses the piezoelectric element material 19 having the heat resistance capable of withstanding the surrounding temperature of about 120 °C, and includes the flexibility (flexible performance) comparable to that of a normal vinyl cord by using the piezoelectric element material 19 constituted by a resin having the flexibility (flexible performance) and the flexible electrodes.

Further, the piezoelectric sensor 9 is provided with a high sensitivity comparable to that of a polymer piezoelectric element material and achieves particularly high sensitivity in the low frequency region (10 Hz or lower) for detecting the heart rate of the human body. This is because a reduction in the sensitivity is small even in the low frequency region since the specific inductive capacity (about 55) of the piezoelectric element material 19 is larger than the specific inductive capacity (about 10) of the polymer piezoelectric element material.

The piezoelectric sensor 9 in the cord-like shape provided in this way as being molded with the piezoelectric element material 19 is not provided with the piezoelectric function and therefore, it is necessary to carry out the processing (polarizing processing) for providing the piezoelectric function to the piezoelectric element material 19 by applying the direct current high voltage of several KV/mm to the piezoelectric element material 19. The polarizing processing is carried out by applying the direct current voltage between the two electrodes 18, 20 after forming the core line 18 and the outer side electrode 20 at the piezoelectric element material 19.

As shown by Fig.3, the base plate 7 is mounted with 4 pieces of the pads 23 for absorbing impact attached to the rear face of the bottom plate 15 and having the elastic force for absorbing impact with the toilet main body 22 by being disposed between the toilet seat apparatus 5 and the toilet main body 22 when the toilet seat is used similar to the background art apparatus. Further, the toilet seat apparatus 5 is mounted with the lid member 25 lifted up to the side of the water tank 24 along with the toilet seat apparatus 5.

According to the embodiment, the piezoelectric sensor 9 in the cord-like shape is mounted onto the base plate 7 by being positioned and supported by the plurality of holders 26 arranged separately from each other.

Enlarging the pad 23, as shown by Fig.11, there is constructed the constitution including the pressing means 27 having an elasticity attached to the inner face of the upper lid as the rigid body and the projection 28 of the rigid body attached onto the base plate 7 and pinching the piezoelectric sensor 9 by the pressing means 27 and the projection 28. Fig.11(a) shows a state before integrating the upper lid 6 and the base plate 7 and Fig.11 (b) shows a state of screwing the upper lid 6 and the base plate 7 to integrate. When the user is seated on the toilet seat apparatus 205, naturally, there is brought about a state of Fig.11 (b), the upper lid 6 and the base plate 7 are constituted by the rigid bodies such that the toilet seat apparatus 5 is not destructed even when body weight is applied thereon.

Here, the rigid body is defined as a member which is not deformed to exceed strength even when at least body weight of the person is mounted thereon, particularly, a member by which when the person is seated thereon, the person does not feel deformation by which the hip portion sinks, that is, the member by which the user does not feel anxiety of the strength. Although the material is not particularly limited but may be the insulating member of resin, ceramic or the like, may be the conductor of a metal or the like, it is added that the background art toilet seat is generally made of resin.

Now, in the state in which the user is seated on the toilet, total body weight of the user is applied on the toilet seat, however, the upper lid 6 is hardly deformed partially owing to the rigid body. However, it seems that a total of the toilet seat is vibrated by body movement of the user although vibration is slight. With regard to how to amplify vibration of the toilet seat to transmit to the piezoelectric sensor 9, a detailed explanation will be given as follows.

First, since the pressing means 27 is disposed between the upper lid 6 and the piezoelectric sensor 9, the pressing means 27 is vibrated by vibration of the upper lid 6, however, a behavior of the vibration is considerably complicated. Whereas the upper lid 6 is constituted by the rigid body, the pressing means 27 is the member having the elasticity and therefore, whereas the upper portion (vicinity of the portion connected to the upper lid 6) of the pressing means 27 is vibrated similar to the upper lid 6, the lower portion (vicinity of the portion connected to the piezoelectric sensor 9) of the pressing means 27 is vibrated slightly delayedly to repeat vibration different from that of the upper lid 6. Therefore, the piezoelectric sensor 9 is transmitted not only with vibration of the upper lid 6 and the base plate 7 as the rigid bodies but also different vibration by the pressing means 27 and the pressing means 27 amplifies so-to-speak vibration of the upper lid 6. Therefrom, the pressing means 27 can be regarded as a kind of amplifying means.

Next, the pressing means 27 and the piezoelectric sensor 9 are opposed to each other by shapes different from each other. There is constructed the constitution in which in the left and right direction of Fig.11, the piezoelectric sensor 9 is longer and in the depth direction of Fig.11, the pressing means 27 is larger and whereas the piezoelectric sensor 9 is opposed thereto by the curved face owing to the cable-like shape, the pressing means 27 is opposed thereto by the plane. Therefrom, in the piezoelectric sensor 9, there are portions which is not brought into contact with the pressing means 27 and the portion which is brought into contact with the pressing means 27 and in the portion brought into contact with the pressing means 27, there are present various portions having different states of being pressed from the portion which is strongly pressed to the portion which is not pressed so much. Therefore, the piezoelectric sensor 9 receives vibration which differs depending on the portions such that at the portion which is not brought into contact with the pressing means 27, the piezoelectric sensor 9 receives vibration as the rigid body, at the portion which is strongly pressed by the pressing means 27, the piezoelectric sensor 9 receives mainly vibration different from that of the rigid body by the pressing means 27. That a total of the piezoelectric sensor does not receive the same vibration but receives vibrations which differ depending on the portions is equal to that the piezoelectric sensor 9 is partially deformed and therefore, the output of the piezoelectric sensor can be increased. As a result, the piezoelectric sensor 9 can be deformed from vibration of the rigid body which is hardly deformed and therefore, vibration of the rigid body is amplified. Therefrom, the difference between the shapes of the faces of the pressing means 27 and the piezoelectric sensor 9 opposed to each other can be regarded as a kind of amplifying means.

Next, the projection 28 and the piezoelectric sensor 9 are opposed to each other by shapes different from each other. Therefrom, in the piezoelectric sensor 9, there are a portion which is not brought into contact with the projection 28 and the portion which is brought into contact with the projection 28. Therefore, the piezoelectric sensor 9 receives or does not receive vibration depending on portions such that at the portion which is not brought into contact with the projection 28, the piezoelectric sensor 9 does not receive as the rigid body so much and at the portion brought into contact with the projection 28, the piezoelectric sensor 9 receives vibration as the rigid body by the projection 28, that is, vibration similar to the base plate 7 or the upper lid (rigid body) 6. That a total of the piezoelectric sensor cannot receive the same vibration but the piezoelectric sensor receives or does not receive vibration depending on the portions is equal to that the piezoelectric sensor 9 is partially deformed and therefore, the output of the piezoelectric sensor can be increased. As a result, the piezoelectric sensor 9 can be deformed from vibration of the rigid body which is hardly deformed although deformation is small and therefore, vibration of the rigid body is amplified. Therefrom, the difference between the shapes of the faces of the projection 28 and the piezoelectric sensor 9 opposed to each other can be regarded as a kind of amplifying means. However, the effect of amplification by the projection 28 is smaller than the effect of amplification of the above-described pressing means 27.

Next, the pressing means 27 and the projection 28 are opposed to each other by the shapes different from each other and particularly, the area of the pressing means 27 is larger. Since the projection 28 is the rigid body, only the center portion of the pressing means 27 is compressed, the elasticity of the pressing means 27 is hampered at the compressed center portion and therefore, vibration near to that of the rigid body is executed. Further, a force is applied to the piezoelectric sensor 9 by the pressing means 27 and the projection 28 and the piezoelectric sensor 9 is fixed to some degree. The magnitude of the force is changed by the elasticity of the pressing means 27 and also by the distance between the upper lid 6 and the base plate 7 when integrated. The piezoelectric sensor 9 is exerted with the force and is firmly fixed when the elasticity of the pressing means 27 is lower and when the distance between the upper lid 6 and the base plate 7 is short. On the other hand, at the side of the surrounding of the pressing means (portion to which the projection 28 is not opposed), the elasticity is maintained and therefore, the side can be vibrated different from that of the rigid body. Therefore, at the center portion of the pressing means 27, the piezoelectric sensor 9 is fixed and executes vibration near to that of the rigid body and on the side of the surrounding of the pressing means 27 (portion to which the projection 28 is not opposed), the piezoelectric sensor 9 executes vibration different from that of the rigid body by only being pressed by the pressing means 27. At this occasion, a point requiring caution is that the portion of executing vibration near to that of the rigid body and the portion executing vibration different from that of the rigid body are disposed at positions extremely proximate to each other in the piezoelectric sensor 9. That the piezoelectric sensor 9 receives vibrations different from each other at the extremely proximate positions is equal to that the piezoelectric sensor 9 is locally deformed and therefore, output of the piezoelectric sensor can be increased. As a result, the piezoelectric sensor 9 can be deformed from vibration of the rigid body which is hardly deformed although deformation is small and therefore, vibration of the rigid body is amplified. Therefrom, the difference between the shapes of the pressing means 27 and the projection 28 opposed to each other can be regarded as a kind of amplifying means. Here, the projection 28 may not be the rigid body. Even when the projection 28 is constituted by an elastic body, the elasticity can be hampered by strongly compressing the pressing means 27 at the center portion by increasing the height of the pressing means or the projection 28 or shortening the distance between the upper lid 6 and the base plate 7.

Here, with regard to the elasticity of the pressing means 27, the elasticity may be higher than that of the upper lid 6 and the above-described effect as the amplifying means is achieved. For example, a representative cushion member may be used therefor or rubber or sponge may be used therefor. As shown by Fig.5, when the toilet seat apparatus 205 having the above-described constitution is arranged on the toilet main body 22 and weight of the human body M is applied thereon by seating in using the toilet seat apparatus, as described above, the piezoelectric sensor 9 is pressed. As a result, vibration in accordance with movement of the human body M is amplified and applied to the piezoelectric sensor 9 and the electric signal is firmly outputted.

The electric signal provided in accordance with acceleration or vibration is supplied to the controlling means 213.

However, when the toilet seat apparatus 5 is used, the electric signal outputted from the piezoelectric sensor 9 is masked by the controlling means 213 such that vibration produced by driving a cleaning nozzle as cleaning means, operating a blower as drying means, flushing water or the like does not constitute noise for detecting presence/absence, the heart rate or the like of the human body.

As shown by Fig.29, inside of the controlling means 213 includes first determining means 229 for determining motion information of the human body and second determining means 230 for determining biologic information.

The first determining means 229 is constituted to receive the output of the piezoelectric sensor, process the output signal of the piezoelectric sensor by signal processing means 233 having filtering means 231 and amplifying means 232 and calculate motion information of the person (how the person is operated) by motion information calculating means 234 based on the signal. Here, an outsider power source portion 235 supplies power to the signal processing means 235 via power supplying means 236 and always awaits for vibration generated by body movement of the human body.

The second determining means 230 is constituted to receive a signal from the signal processing means 233, process the signal by signal processing means 239 including filtering means 237 and amplifying means 238 and calculate biologic information (heart beat, respiration or the like) of the person by biologic information calculating means 240 based on the signal. Here, the outside power source portion 235 supplies power to the signal processing means 235 via the power supplying means 236 and when a switch 241 of the power supplying means 236 is made OFF, power supply to the signal processing means 239 is cut and biologic information cannot be determined.

When not used, the switch 241 is made OFF to await for vibration generated by body movement of the human body only by the first determining means 229. Further, when used by the person, the person necessarily executes motion of "lifting up the lid member 225" and motion of "being seated on the toilet seat apparatus 205" and therefore, vibration of lifting up the lid member 225 and large vibration of being seated thereon are generated at the toilet seat apparatus 205. Thereby, the piezoelectric sensor 9 is dynamically changed from a state in which vibration is not present to a state of receiving large vibration and therefore, acceleration of displacement is large and a large output is generated therefrom. The large output of the piezoelectric sensor 9 is transmitted to the motion information calculating means 234 as a signal based on motion information by the filtering means 231 and the amplifying means 232, as a result, the first determining means 229 determines that there are brought about motions of "lifting up the lid member 225" and "being seated on the toilet seat apparatus 205" or the like. Further, after determination of "being seated on the toilet seat apparatus 205" by the first determining means 229, the controlling means 213 makes the switch 241 ON by the power supplying means 236. Then, power is supplied also to the signal processing means 239 and therefore, the signal processing means 239 receives a signal from the signal processing means 233, further processes the signal by the filtering means 237 and the amplifying means 238 or the like and transmits the signal to the biologic information calculating means 240. Further, when the user is still, a large vibration by previous motions of "lifting up the lid member 225" and "being seated on the toilet seat apparatus 205" is stopped in a short period of time and thereafter, only a weak vibration in accordance with biologic information of the user, that is, hear beat, respiration or the like is continued. Although the vibration in accordance with the biologic information is weak and therefore, the output of the piezoelectric sensor 9 is small, the output is transmitted to the biologic information calculating means 240 as a signal amplified in two stages by the amplifying means 232, 238 and therefore, the output can be processed by the biologic information calculating means 240 as a signal having a pertinent magnitude. Further, even in the same biologic information, with regard to vibration by heart beat and vibration by respiration, a frequency of heart beat is high and a frequency of respiration is low and therefore, the both can be separated by the filter 237. In this case, as the constitution of detecting heart beat, at inside of the biologic information calculating means 240, a period of heart rate is calculated by calculating an autocorrelation coefficient to calculate heart rate. Further, there are mounted displaying means 242 for displaying the calculated heart rate, comparing means 243 for calculating the heart rate with a previously set value of the heart rate and informing means 244 for issuing an alarm based on a result of comparison. The informing means 244 can issue an alarm when the heart rate becomes equal to or larger than the set value. Particularly, when the person strains in evacuation, the hear rate is increased and there is a concern of bringing about occurrence of the cerebral hemorrhage, however, the controlling means 293 can contribute to health control by foreseeing the attack from a change in the heart rate. At this occasion, when the controlling means 293 is connected by a network in, for example, a hospital or the like, not only the toilet seat apparatus 205 used in the hospital can centrally be monitored summarizingly but also a behavior in a rest room which cannot be diagnosed directly can always be monitored.

Further, the signal to the displaying means 242 can be transmitted via communicating means by wired means or wireless means.

Here, with regard to the first determining means for determining motion information, when attention is paid to the magnitude of the output of the signal processing means 233, as shown by Fig. 7, a large output waveform is outputted at an instance at which the human body M is seated on the toilet seat apparatus 205 (or at an instance at which the human body M stands up), or when an article of the lid member or the like is mounted thereon (or when the article is removed), or when the body is moved even in a state in which the human body M stays to be seated thereon. On the other hand, when a still state is brought about after the human body M is seated thereon, an output waveform with comparatively low level is outputted by small body movement of the body propagated by action of the heart or the respiratory action.

In contrast thereto, when the human body M is unpresent, or when an article is mounted thereon, an output waveform is not shown in a constant period of time after output the large output waveform.

Hence, an output V of the signal processing means 233 and a previously determined two set values Va, Vb can be compared and determined as follows. That is, when V < Va, it is determined that the human body M or the article is not present (unpresent output Hi), when Va ≦ V < Vb, it is determined that the human body M is present in a still state (present output Hi). Further, when Vb < V, it is determined that the human body M produces body movement (body movement output Hi). When the article is mounted thereon in place of the human body M, although determination of seating, body movement is executed temporarily, unpresence of the human body M is determined by determining a state of placing the article since vibration of low level propagated by action of the heart or the respiratory action as in the human body M does not appear.

Further, for example, when it is determined that the person is present by the first determining means, deodorizing means and toilet seat heating means may be started to operate and the operation may be stopped when unpresence of the person is determined. Further, here, the toilet seat heating means is the heater 12.

On the other hand, with regard to the second determining means for determining biologic information, heart beat is to be extracted from a variation of the output in the so-to-speak still time of Fig.7. Actually, an output waveform of the piezoelectric sensor 9 in the still time is as shown by Fig.30.

Now, in the signal processing means 239, the filtering means 237 is a low pass filter having a cutoff frequency of 30 Hz for particularly removing noise component of 60 Hz and is a high pass filter having a cutoff frequency of 0.5 Hz for removing a vibration component by respiration. Fig.31 shows an output waveform after passing the filtering means 237.

Next, in the biologic information calculating means 240, first, an autocorrelation coefficient between moving time of 0 to t max second is calculated. Fig.32 shows an example of the calculated autocorrelation coefficient.

Successively, a period is determined based on the calculated autocorrelation coefficient F(t). Fig.33 shows operation of calculating the period by a flowchart. Operation is constituted by peak detection.

While increasing t from 0 by small time period dt, from step 2 to step 4, a reduction in F(t) is confirmed and an increase in F(t) is confirmed from step 5 to step 7. t and F(t) after finishing the increase are stored to at step 8. A peak is detected by repeating the steps until the moving time t becomes t max and after finishing detection, at step 9, t indicating a maximum value in the peaks is defined as the period. According to the data example of Fig.32, the period is t1 second. Next, the heart rate is calculated. The heart rate is calculated from the calculated period and the heart rate is S = 60/t1. In the data example, S = 60/t1. The heart rate S is calculated in the biologic information calculating means 240 as described above.

Incidentally, according to the embodiment, an amplification factor of the amplifying means 232 can be set to 10 and an amplification factor of the amplifying means 238 can be set to 200.

Further, the first determining means can be used intentionally by the user as inputting means for advancing steps of control of the toilet seat apparatus 205.

For example, as a general function of the toilet seat apparatus 205, after evacuation, water is flushed, or drying wind is blown. However, in this case, the operation is not necessarily carried out by pertinent time period or amount. For example, it is considerably difficult to stop cleaning by detecting a degree of cleaning the hip portion or detecting a drying degree and therefore, start and stop thereof is frequently controlled by driving the toilet seat apparatus 205 by a previously set average time period, or depressing a switch by the user.

Hence, it is conceivable to control start and stop by detecting motion information of, for example, "rocking the hip portion" by the first determining means. It is possible that when the hip portion is rocked by the user staying to be seated on the toilet after finishing evacuation, cleaning water is started to flush, when the hip portion is rocked successively, cleaning water is stopped to flush, drying wind is started to be blown, next, when the hip portion is rocked, drying wind is stopped to be blown. According to the method, the user can clean and dry the hip portion by preferable time period and amount and therefore, unpleasant feeling by a deficiency in time period and amount can be eliminated and time period and amount can be prevented from being excessively large to be wasted. Further, in comparison with the operation of touching a switch by the hand, the hand may not touch anywhere and therefore, the operation is the most clean.

According to the above-described toilet seat apparatus 205, the piezoelectric sensor 9 can achieve high reliability by easily detecting even slight movement of the human body by supplying the electric signal in accordance with acceleration of vibration firmly to the controlling means 213. Further, the piezoelectric sensor 9 is provided with the flexibility and is difficult to be destructed even when impact continues to be applied thereto, further, the piezoelectric sensor 9 outputs the detected signal facilitating to differentiate the person and the article and therefore, detection of the seating or the like can be ensured.

Further, although according to the above-described embodiment, an explanation has been given of the constitution of determining presence/absence by smoothing the output signal from the piezoelectric sensor 9 and calculating the heart rate by calculating the autocorrelation coefficient, it is also conceivable to determine presence/absence by converting the output signal into digital data by AD conversion by a microcomputer or the like and based on the value constituted by subjecting the digital data to the moving average in the microcomputer.

The effect of the embodiment described above will be summarized.

The vibration detecting apparatus 245 is constituted as shown by Fig.29 by combining the piezoelectric sensor 9 having the flexibility for detecting vibration and the controlling means 251 for determining biologic information after determining motion information based on the output of the piezoelectric sensor 9.

Thereby, it is not necessary to await for biologic information until determining motion information and therefore, consumption of power necessary for awaiting for biologic information can be prevented to achieve efficient formation, or electric noise generated by useless current can similarly be prevented and accuracy of determination is promoted.

Further, vibration is derived from body movement of the human body and the determining means is constituted to determine heart beat, respiration or the like as biologic information after determining presence of the human body as motion information.

Thereby, since biologic information of heart beat, respiration or the like is not generated when the human body is not present and therefore, by only determining biologic information after determining presence of the human body, biologic information can be determined sufficiently accurately. It is not necessary to await for biologic information until determining presence of the human body and the effect of efficient formation and promotion of accuracy is similarly achieved.

Further, there is constructed the constitution including the first determining means 229 for determining motion information and the second determining means 230 for determining biologic information.

Thereby, respectives of motion information and biologic information can pertinently be determined and accuracy of determination is promoted.

Further, there is constructed the constitution including the power supplying means 236 for supplying power to the first determining means 229 and the second determining means 230 in which the power supplying means 236 does not supply power to the second determining means 230 in determining motion information.

Thereby, until determining motion information by the first determining means 229, consumption of power to the second determining means 230 can be reduced and efficient formation of power is achieved. At this occasion, electric noise generated by current consumed by the second determining means 230 can similarly be prevented and accuracy of determination is promoted.

Further, there is constructed the constitution in which the determining means 229, 230 include the amplifying means 232, 238 for amplifying the output of the piezoelectric sensor 9 and the amplification factor (2000) in determining biologic information is made to be larger than the amplification factor (10) in determining motion information.

Thereby, since vibration caused by biologic information is considerably smaller than vibration caused by motion information, by increasing the amplification factor in determining biologic information, biologic information can accurately be determined.

Further, there is constructed the constitution of determining motion information and biologic information by detecting body movement of the user transmitted to the toilet seat.

Thereby, it is not necessary to await for biologic information of heart beat, respiration or the like of the seated user until determining motion information in which the user lifts up the lid member 205 or is seated on the toilet seat and therefore, the effect of efficient formation and accuracy promotion is achieved.

Further, there is constructed the constitution of including the controlling means for controlling at least one of the displaying means 242, the informing means 244, communicating means, cleaning means, drying means, the toilet seat heating means (heater) 12, water feeding and flushing means, room air conditioning means, ventilating means, deodorizing means and the like based on motion information and biologic information.

Thereby, it is easy to execute various controls by motion information and biologic information which are efficiently and accurately determined and the multifunction toilet seat apparatus 205 making full use of motion information and biologic information can be realized.

### (Embodiment 12)

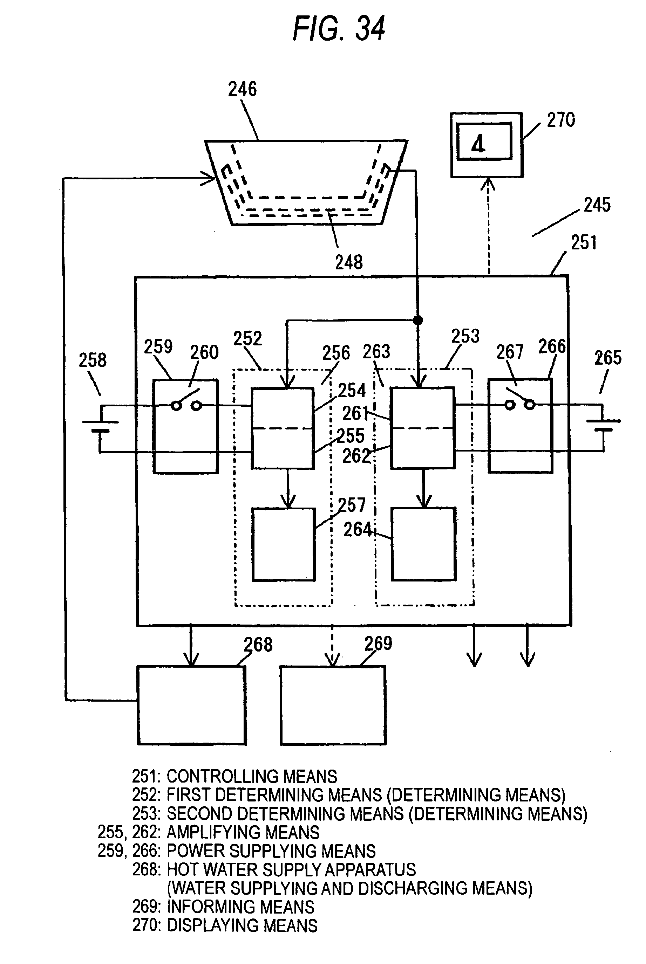

Fig.15(c) is a constitution view of a section of a bath tub apparatus showing a twelfth embodiment according to the invention and Fig.34 is a block constitution diagram of a control apparatus in the bath tub apparatus.

As shown by Fig.15(c), the bath tub apparatus of the embodiment according to the invention is arranged with a piezoelectric sensor 248 in a cord-like shape having a flexibility between a bath tub 246 of a rigid body and a cover 247. Further, the bath tub apparatus includes a projection 249 projected from the bath tub 246 to the piezoelectric sensor 248, and a holder 250 for supporting the piezoelectric sensor 248 by the cover 247 and the piezoelectric sensor 248 is positioned via the holder 250.

Here, the projection 249 and the piezoelectric sensor 248 are opposed to each other by shapes different from each other. There is constructed a constitution in which the piezoelectric sensor 248 is longer in a direction in parallel with paper face of Fig.15(c) and the projection 29 is larger in a depth direction of Fig.15(c). Therefrom, the piezoelectric sensor 248 is provided with a portion which is not brought into contact with the projection 249 and a portion which is brought into contact with the projection 249 and in the portion brought into contact with the projection 249, there are present various portions having different states of being pressed from a portion (center) which is strongly pressed to a portion which is not pressed so much.

In addition thereto, although the piezoelectric sensor 248 is attached to the cover 247 via the holder 250, a situation is changed by whether the cover 247 is integrally fixed to the bath tub 246, whether the cover 247 is constituted by a rigid body or an elastic body, and whether the holder 250 is constituted by a rigid body or an elastic body.

First, when the cover 247 is not integrally fixed to the bath tub 246, regardless of a material of the cover 247 and a material of the holder 250, although the bath tub 246 is vibrated by body movement of the user, the cover 247 is not vibrated. Vibration of the bath tub 246 is transmitted to the piezoelectric sensor 248 only from the projection 249 and therefore, only a portion of the piezoelectric sensor 258 brought into contact with the projection 249 is vibrated and a portion thereof which is not brought into contact with the projection 249 is not vibrated. That a total of the piezoelectric sensor 248 does not receive the same vibration but receives vibration which differs by the portions is equal to that the piezoelectric sensor 248 is partially deformed and therefore, an output of the piezoelectric sensor 248 can be increased. As a result, the piezoelectric sensor 248 can be deformed from vibration of the bath tub (rigid body) 246 which is hardly deformed and therefore, vibration of the bath tub (rigid body) 246 is amplified. Therefrom, a difference between shapes of faces of the projection 249 and the piezoelectric sensor 248 opposed to each other can be regarded as a kind of amplifying means.

Next, when the cover 247 is integrally fixed to the bath tub 246 and at least one of the cover 247 and the holder 250 is constituted by an elastic body, the bath tub 246 is vibrated by body movement of the user, and vibration by way of the cover 247 and the holder 250 executes vibration different from that of the bath tub 246 because the vibration is brought about by way of the elastic body. Although vibration of the bath tub 246 is transmitted to the piezoelectric sensor 248 from the projection 249, also the vibration by way of the cover 247 and the holder 250 is transmitted to the piezoelectric sensor 248 and therefore, the piezoelectric sensor 248 receives vibrations which differ by the portion brought into contact with the projection 249 and the portion brought into contact with the holder 250. That a total of the piezoelectric sensor 248 does not receive the same vibration but receives vibrations which differ by the portions is equal to that the piezoelectric sensor 248 is partially deformed and therefore, the output of the piezoelectric sensor 248 can be increased. As a result, the piezoelectric sensor 248 can be deformed from vibration of the bath tub (rigid body) 246 which is hardly deformed and therefore, vibration of the bath tub (rigid body) 246 is amplified. Therefrom, the projection 249, the cover 247, the holder 250 can be regarded to constitute a kind of amplifying means.

Finally, in the case in which the cover 247 is integrally fixed to the bath tub 246 and both of the cover 247 and the holder 250 are rigid bodies, when the bath tub 246 is vibrated by body movement of the user, it seems that vibration conducted to the piezoelectric sensor 248 from the projection 249 and vibration by way of the cover 247 and the holder 250 are constituted by the same vibration. However, microscopically, there is a slight time difference to a degree which cannot be determined by the human being therebetween. That is, when the bath tub 246 is vibrated, the projection 249 is immediately vibrated, however, the holder 250 is not vibrated immediately. After vibrating the bath tub 246, the cover 247 is vibrated by way of a portion thereof connected to a surrounding of the bath tub 246 and the holder 250 is started to vibrate further thereafter. That is, a length of a path of transmitting vibration is longer. Therefore, the piezoelectric sensor 248 receives vibrations which differ slightly by the portion which is brought into contact with the projection 249 and the portion which is brought into contact with the holder 250. That a total of the piezoelectric sensor 248 does not receive the same vibration but receives vibrations which differ by the portions is equal to that the piezoelectric sensor 248 is partially deformed and therefore, the output of the piezoelectric sensor 248 can be increased. As a result, the piezoelectric sensor 248 can be deformed from vibration of the bath tub (rigid body) 246 which is hardly deformed and therefore, vibration of the bath tub (rigid body) 246 is amplified. Therefrom, the projection 249, the cover 247, the holder 250 can be regarded to constitute a kind of amplifying means. However, in this case, an amplifying function is smaller than those in the above-described cases and therefore, a circuit of processing the sensor output needs to be devised for increasing the amplification factor.

Now, the embodiment includes other constitution having the amplifying function.

First, the constitution includes an elasticity of the piezoelectric sensor 248. The piezoelectric sensor 248 having a flexibility (flexible performance) is also provided with the elasticity and therefore, vibration from the projection 249 brought into contact with the piezoelectric sensor 248 and vibration from the holder 250 can vibrate the piezoelectric sensor 248 per se, particularly, can execute vibration which differs from the original vibration by the elasticity of the piezoelectric sensor 248. That is, there is constituted a mechanism in which while generating an output with regard to the provided original vibration, still other output is generated by executing different vibration by the piezoelectric sensor 248 per se.

Next, the piezoelectric sensor 248 is mounted thereto in a state of being exerted with a tension. In Fig.15(c), the piezoelectric sensor 248 is supported in a state of being exerted with a tension, that is, of being pulled tightly. In this case, in comparison with the case in which the piezoelectric sensor 248 is not exerted with the tension, vibration in the piezoelectric sensor 248 is propagated remotely. Vibration from the projection 249, the holder 250 brought into contact with the piezoelectric sensor 248 is more remotely propagated in the piezoelectric sensor 248 when a tension to some degree is exerted thereto, as a result, the output can be generated from various locations in the piezoelectric sensor 248. It seems that an effect similar to that in increasing a sensitivity of the sensor, that is, amplifying vibration is achieved thereby. Further, with regard to what degree of tension is preferably applied, it is preferable to constitute a range in which at least a mechanical strength of the piezoelectric sensor 248 is not deteriorated, particularly in a range of capable of maintaining the elasticity.

As shown by Fig.34, inside of the controlling means 251 includes first determining means 252 for determining motion information of the human body and second determining means 253 for determining biologic information.

The first determining means 252 is constituted to receive the output of the piezoelectric sensor 248, process the output signal of the piezoelectric sensor 248 by signal processing means 256 having filtering means 254 and amplifying means 255 and calculate motion information (what operation is executed) by the person by motion information calculating means 257 based on the signal. Further, an outside power source portion 258 supplies power to the signal processing means 256 via power supplying means 259 and can control ON/OFF of power supply by a switch 260. When the switch 260 of the power supplying means 259 is made ON, the signal can be supplied by supplying power to the signal processing means 256 and motion information can be determined by the motion information calculating means 257. When the switch 260 of the power supplying means 259 is made OFF, power supply to the signal processing means 256 is cut and motion information cannot be determined.

The second determining means 253 is constituted to receive the output of the piezoelectric sensor 248, process the output signal of the piezoelectric sensor 248 by signal processing means 263 having filtering means 261 and amplifying means 262 and calculate biologic information (heart beat, respiration or the like) of the person by biologic information calculating means 264 based on the signal. Here, the outside power source portion 265 supplies power to the signal processing means 263 via power supplying means 266 and can control ON/OFF of power supply by a switch 267. When the switch 267 of the power supplying means 266 is made ON, the signal can be processed by supplying power to the signal processing means 263 and biologic information can be determined by the biologic information calculating means 264. When the switch 267 of the power supplying means 266 is made OFF, power supply to the signal processing means 263 is cut and biologic information cannot be determined.

The following control is conceivable by the controlling means 251 based thereon. Although when not used, both of the switches 260, 267 are made OFF and motion information and biologic information are not awaited for, there is brought about a state of awaiting for motion information by switching only the switch 260 ON in corporation with finishing to automatically fill hot water to the bath tub, or finishing to heat the bath tub. When the person is moved into the bath tub 246, large vibration based on motion of "moving into the bath tub 246" is generated at the bath tub 246. Thereby, the piezoelectric sensor 248 is dynamically changed from a state in which vibration is not present to a state of receiving large vibration and therefore, acceleration of deformation is large and large output is generated. Large output of the piezoelectric sensor 248 is transmitted to the operation information calculating means 257 as a signal based on motion information by the filtering means 254 and the amplifying means 255, as a result, the first determining means 252 determines that there is brought about operation of "moving into the bath tub 246". Further, after determining "moving into the bath tub 246" by the first determining means 252, the controlling means 251 makes the switch 267 ON by the power supplying means 266. Then, power is supplied also to the signal processing means 263 and therefore, the signal processing means 263 receives a signal form the piezoelectric sensor 248, processes further the signal by the filtering means 261, the amplifying means 262 and the like and transmits the signal to the biologic information calculating means 264. Further, when the user remains still, the large vibration by the previous motion of "moving into the bath tub 246" is stopped in a short period of time, thereafter, only weak vibration in accordance with biologic information of the user, that is, heart beat, respiration or the like is continued. Since the vibration in accordance with the biologic information is weak, the output of the piezoelectric sensor 248 is small, however, the output is transmitted to the biologic information calculating means 264 as a signal amplified by the amplifying means 262 and therefore, the output can be processed as a signal having a pertinent magnitude by the biologic information calculating means 264. Further, despite the same biologic information, with regard to vibration by heart beat and vibration by respiration, a frequency of heart beat is higher and a frequency of respiration is lower and therefore, the both can be separated by the filter 261 or the like. Here, when, for example, an increase in a blood pressure or a degree of a rush of blood to the head of the bathing person can be determined by biologic information of heart beat, respiration and the like, the water supply apparatus 268 can be controlled to the lower temperature of hot water by pouring water after determination. Further, when a doze of the bathing person can be determined from biologic information, a danger of drowning can be avoided beforehand by informing a danger to the family by the informing means 269 disposed at other room by carrying out wireless communication.

Further, the first determining means can be used intentionally by the user as inputting means of a control of the bath tub apparatus.

For example, as general functions of the bath tub apparatus, there is carried out automatic hot water filling, additional heating, pouring hot water, pouring water, producing bubbles having a relaxation effect, or ventilating, drying, cooling and heating a bathing room or the like. Further, generally, the bathing person controls the functions by switch operation.

Hence, it is conceivable to execute the controls by detecting motion information of "knocking at the bath tub 246" by the first determining means 252 of the invention. The bath tub 246 is the integrally constituted rigid body and vibration larger than that of biologic information can be provided by only knocking at an edge thereof lightly. Further, the motion of "knocking at the bath tub 246" can be made to correspond to switches of various functions by factors of time and number of times. For example, there can be constituted a way of use such that when the bath tub 246 is knocked at by one time in unit time, hot water is poured and when the bath tub 246 is knocked at twice, bubbles are produced. Further, it is also possible that the displaying means 270 for displaying a set value of a holding temperature at inside of the bathing room and numerical values of rise and fall of the set temperature are respectively changed by a number of times of knocking at the bath tub 246 such that when the bath tub 246 is knocked at strongly, the set temperature is in a direction of rising and when the bath tub 246 is knocked at weakly, the set temperature is in a direction of falling. In this case, the holding temperature can be changed by the bathing person while confirming display by the displaying means 270.

As described above, when the piezoelectric sensor is used as the inputting means of the bath tub apparatus, it is not necessary to separately provide a switch in the bathing room and a waterproof measure for the switch is not needed. When all the switches can be replaced by the piezoelectric sensor, it is not necessary to attach a controller of a remote controller or the like on a wall of the bathing room and wiring or construction therefor is not needed.

According to the embodiment, the second determining means 252 is connected in parallel with the first determining means 253 and therefore, the first and second determining means can determine independently from each other. That is, by combinations of ON/OFF of the switches 260, 261, determination of motion information by the first determining means 253 and determination of biologic information by the second determining means 252 can be executed simultaneously or one of the means can be executed. When the determinations are executed simultaneously, an outside apparatus can be controlled by generally executing determination by information of the both, and other determination can be controlled by information of one of the determinations. When only one of the determinations is executed, power consumption of other can be prevented and therefore, wasteful power may not be used and therefore, there is achieved an effect of improving the efficiency, and promoting accuracy of determination since noise can be prevented from being brought about by wasteful current.

Further, although according to the embodiment, the bath tub as the rigid body has been shown, the bath tub is constituted by various materials when viewed historically. The materials are constituted by wood, resin, a metal of stainless steel or the like, marble, or rock, tile and the like. The rigid body for the bath tub can be adopted so far as the rigid body is constituted by a material which is not deformed considerably such that the user feels deformation in normal bathing and vibration is transmitted thereby.

### (Embodiment 13)

Fig.18(c) is a constitution view of a shower apparatus showing a thirteenth embodiment according to the invention.

As shown by Fig.18(c), a shower apparatus of the embodiment according to the invention can use shower in a seated attitude and is arranged with a piezoelectric sensor 272 at inside of a seat 271. Similar to Embodiment 11, motion information of presence/absence, body movement of a user and biologic information of heart beat, respiration or the like are determined to be able to be useful for control of shower or the like.

Particularly, when heart beat or respiration is determined after the user is determined to be seated thereon, the shower apparatus is efficient and can promote accuracy of determination.

### (Embodiment 14)

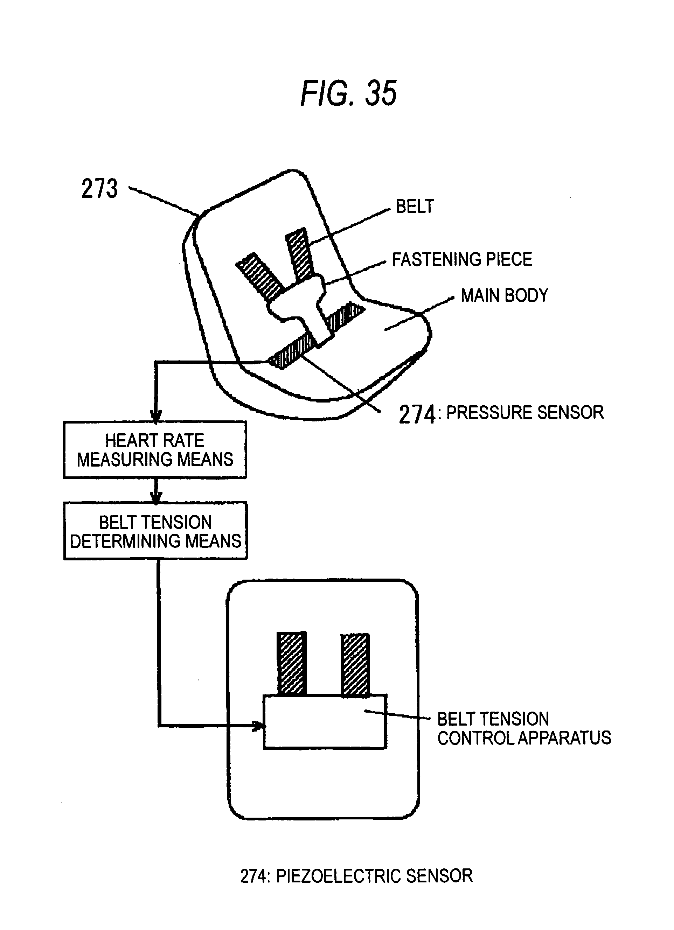

Fig.35 is a constitution view of a child seat showing a fourteenth embodiment according to the invention.

As shown by Fig. 3 5, the child seat according to the embodiment of the invention is arranged with a piezoelectric sensor 274 in a sheet-like shape at a child seat main body 273 having a high cushioning performance. A state of pressing the chest portion of a baby is determined based on motion information of body movement or the like and biologic information of heart beat, respiration of the like of the baby to enable to be useful for control of a tension of a belt or the like.

Particularly, when heart beat or respiration is determined after determining that the baby is seated thereon, the apparatus is efficient and accuracy of determination can be promoted.

Further, in the case of a seat having high cushioning performance, the amplification factor of the determining means can be reduced.

Further, the piezoelectric sensor 274 of the embodiment is constituted not in a cable-like shape but in a sheet-like shape by attaching conductive rubber as electrodes to both faces of a piezoelectric sheet constituted by molding a piezoelectric element material in a sheet-like shape.

### (Embodiment 15)

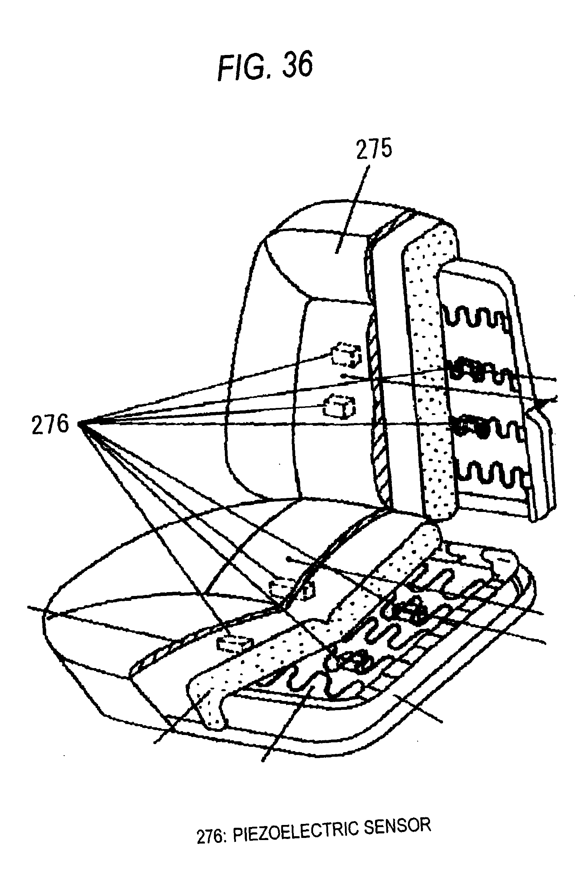

Fig.36 is a constitution view of a car seat showing a fifteenth embodiment according to the invention.

As shown by Fig.36, the car seat of the embodiment according to the invention is arranged with a plurality of piezoelectric sensors 76 at inside of a car seat 275 having a high cushioning performance. A psychological sate or a doze of a driver is determined based on motion information of body movement of the driver or the like and biological information of heart beat, respiration or the like to inform the driver or to enable to be useful for a control of cooling and heating inside of the vehicle.

Particularly, when heart beat or respiration is determined after determining starting of driving, the embodiment is efficient and can promote accuracy of determination.

### (Embodiment 16)

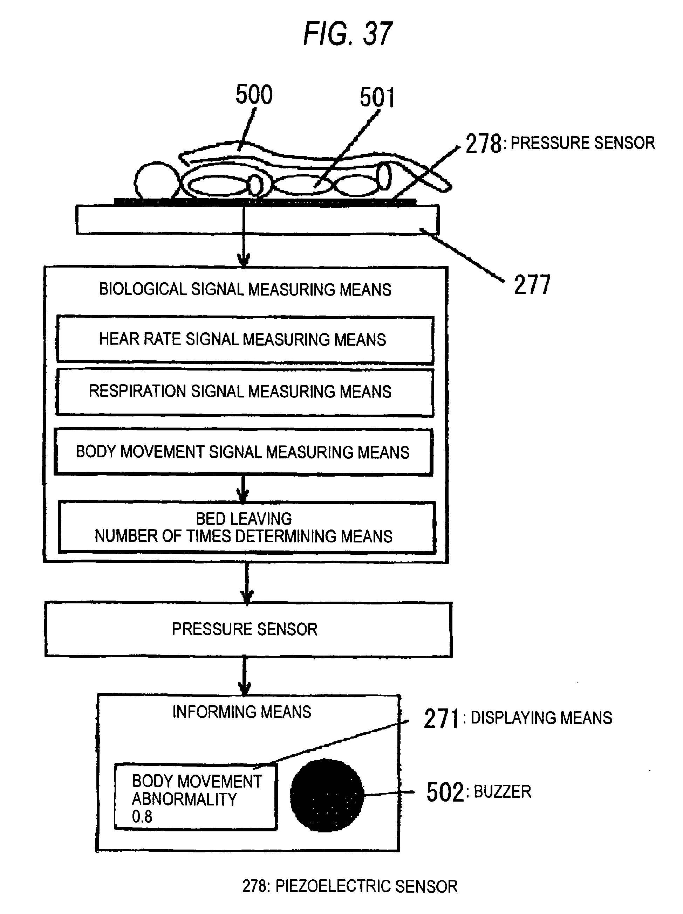

Fig.37 is a constitution view of bedclothes showing a sixteenth embodiment according to the invention.

According to the bedclothes of the embodiment of the invention, as shown by Fig.37, a mattress having a high cushioning performance is mounted with a piezoelectric sensor 278. The embodiment determines sleeping start or physical condition of the person based on motion information of body movement or the like and biologic information of heart beat, respiration or the like of the person to inform the person or the family or can be made to be useful for control of cooling and heating inside of a room.

Further, a nonrespiratory state in sleeping can also be determined by vibration of respiration or snoring as biologic information and when the nonrespiratory state continues for a long period of time, the person can be awakened or informed by lighting illumination or informing.

Particularly, when heart beat or respiration is determined after determining that the person goes to bed or falls asleep, the embodiment is efficient and can promote accuracy of determination.

Further, the sleeping cloth may be a bed or may be a futon or the like and is applicable also to a blanket or a carpet.

Although the invention has been explained in details and in reference to the specific embodiments, it is apparent for a skilled person that the invention can variously be changed or modified without deviating from the spirit and the range of the invention.

The invention is based on Japanese Patent Application No.2003-176677 filed on June 20, 2003, Japanese Patent Application No.2003-346815 filed on October 6, 2003, Japanese Patent Application No.2003-346816 filed on October 6, 2003, and Japanese Patent Application No.2003-348200 filed on October 7, 2003 and content thereof is incorporated herein by reference.

### <Industrial Applicability>

As has been explained above in details, according to the toilet seat apparatus of the invention, the piezoelectric sensor in the cord-like shape can firmly detect vibration applied by the seating or the like by being set with the pressing means and can output the electric signal of a magnitude in accordance with acceleration or vibration. Further, the piezoelectric sensor is provided with the flexibility, easy to be arranged, difficult to be destructed even when impact continues applying thereto, further, can output the electric signal ensuring to differentiate the person and the article to thereby provide high reliability.

Further, according to the vibration detecting apparatus and the toilet seat apparatus of the invention, even when the rigid body is hardly deformed by vibration transmitted to the rigid body, vibration transmitted to the rigid body is amplified by the amplifying means and detected by the vibration detecting sensor and therefore, vibration transmitted to the rigid body can accurately be detected. Therefore, the invention achieves the effect not only for the toilet seat apparatus, the bath tub apparatus, the shower apparatus, but also to a seat having a small elasticity and can be utilized also for a wheel chair. Further, the invention is applicable to a constitution other than a constitution to be seated thereon so far as a user is brought into contact therewith to provide vibration and therefore, the invention is effective to a constitution used when the person stands up, a constitution used when the person is stooped and a constitution used when a person lies down. As an example, a weight meter, a physical length meter, a bed, a stretcher, an operation couch or the like is pointed out.

Further, according to the vibration detecting apparatus and the toilet seat apparatus of the invention, the vibrating rigid body is difficult to be vibrated the most at a vicinity of a fixing portion and therefore, the vibration detecting sensor supported by the vicinity of the pressing portion can be prevented from being vibrated at least at the supported portion. Therefore, vibration transmitted to the rigid body can accurately be detected in an environment in which the vibration detecting sensor per se is not vibrated. Therefore, the invention achieves the effect not only in the toilet seat apparatus, the bath tub apparatus, the shower apparatus, but also to a seat having small elasticity and can be utilized also for a wheel chair. Further, the invention is applicable to a constitution with which a user is brought into contact to provide vibration other than a constitution to be seated thereon and therefore, the invention is effective also for a constitution used when the user stands thereon, a constitution used when the user stoops thereon, or a constitution used when the user lies down thereon. As an example, a weight meter, a physical length meter, a bed, a stretcher, an operation couch or the like is pointed out.

Further, according to the vibration detecting apparatus and the toilet seat apparatus of the invention, the invention is provided with the flexibility and determines biologic information after determining motion vibration based on an output of the piezoelectric sensor for detecting vibration and therefore, it is not necessary to await for biologic information until determining motion information and therefore, consumption of power necessary for awaiting for biologic information can be prevented and efficient formation is achieved, electric noise generated by useless current can similarly be prevented and accuracy of determination is promoted.

Therefore, the invention can be utilized not only for the toilet seat apparatus, the bath tub apparatus, the shower apparatus, the child seat, the bed clothes but also for the wheel chair. Further, the invention is applicable to a constitution other than a constitution to be seated so far as the user is brought into contact therewith to provide vibration and therefore, the invention is effective also to a constitution used when the user stands up thereon, a constitution used when the user stoops thereon, a constitution used when the user lies down thereon. As an example, a weight meter, a physical length meter, a stretcher, an operation couch or the like is pointed out.

## Claims

1. A vibration detecting apparatus including a first amplifying means for amplifying a vibration transmitted to a rigid body and a vibration detecting sensor for detecting the amplified vibration.

2. The vibration detecting apparatus according to Claim 1, wherein the vibration detecting sensor is arranged to a case comprising an upper lid and a base plate and the upper lid comprises a rigid body.

3. The vibration detecting apparatus according to Claim 1 or 2, wherein the first amplifying means is constructed by a constitution including pressing means having an elasticity for pressing the vibration detecting sensor.

4. The vibration detecting apparatus according to any one of Claims 1 through 3, wherein the first amplifying means is constructed by a constitution including the pressing means for pressing the vibration detecting sensor by a face thereof opposed to the vibration detecting sensor by a shape different from a shape of the vibration detecting sensor.

5. The vibration detecting apparatus according to any one of Claims 1 through 4, wherein the vibration detecting sensor is constituted by a piezoelectric sensor having a flexibility.

6. The vibration detecting apparatus according to Claim 1, wherein the vibration detecting sensor is constituted by a constitution of being supported by a vicinity of a fixing portion for fixing the rigid body.

7. The vibration detecting apparatus according to Claim 6, wherein the vibration detecting sensor is constructed by a constitution of being arranged in a case comprising a rigid body and providing a leg portion as a fixing portion at a bottom face of the case for supporting the vibration detecting sensor at a vicinity of the leg portion.

8. The vibration detecting apparatus according to Claim 6 or 7, wherein the vibration detecting apparatus is constructed by a constitution including a plurality of the fixing portions and supporting the vibration detecting sensor at the vicinities of the plurality of fixing portions.

9. The vibration detecting apparatus according to any one of Claims 6 through 8, wherein the vibration detecting sensor is constituted on a side of a vibration source of the fixing portions.

10. The vibration detecting apparatus according to any one of Claims 6 through 9, wherein the vibration detecting sensor is constituted by a piezoelectric sensor having a flexibility.

11. The vibration detecting apparatus according to Claim 1, wherein the vibration detecting sensor is constituted by a piezoelectric sensor having a flexibility, further including determining means for determining biologic information after determining motion information based on an output of the piezoelectric sensor.

12. The vibration detecting apparatus according to Claim 11, wherein the vibration is derived from a body movement of the human body and the determining means is constituted to determine a heart rate, respiration or the like as the biologic information after determining presence of the human body as the motion information.

13. The vibration detecting apparatus according to Claim 11 or 12, wherein the determining means is constructed by a constitution including first determining means for determining the motion information and second determining means for determining the biologic information.

14. The vibration detecting apparatus according to Claim 13, further including power supplying means for supplying a power to the first determining means and the second determining means and the power supplying means is constructed by a constitution which does not supply at least a portion of the power to the second determining means in determining the motion information, or does not supply at least a portion of the power to the first determining means in determining the biologic information.

15. The vibration detecting apparatus according to any one of Claims 11 through 14, wherein the determining means is constructed by a constitution including second amplifying means for amplifying an output of the piezoelectric sensor, an amplification factor in determining the biologic information being larger than an amplification factor in determining the motion information.

16. A toilet seat apparatus **characterized in** a toilet seat apparatus arranged with the vibration detecting sensor of the vibration detecting apparatus according to any one of Claims 1 through 10, at the case comprising the upper lid and the base plate;
wherein the vibration detecting sensor is a piezoelectric sensor in a code-like shape.

17. A toilet seat apparatus **characterized in** a toilet seat apparatus arranged with the vibration detecting sensor of the vibration detecting apparatus according to any one of Claims 11 through 15 at the case comprising the upper lid and the base plate;
wherein the vibration detecting sensor is a piezoelectric sensor in a cord-like shape.

18. The toilet seat apparatus according to Claim 17 constituted to include controlling means for controlling at least one of displaying means, informing means, communicating means, cleansing means, drying means, toilet seat heating means, water feeding and discharging means, room air conditioning means, ventilating means, deodorizing means and the like based on the motion information and the biologic information.

19. The toilet seat apparatus according to any one of Claims 16 through 18, wherein the piezoelectric sensor in the code-like shape outputs an electric signal in accordance with an acceleration of a vibration when the piezoelectric sensor is applied with the vibration.

20. The toilet seat apparatus according to any one of Claims 16 through 19, wherein the piezoelectric sensor in the cord-like shape is attached to one of the upper lid and the base plate and the case includes the pressing means for generating an output by being brought into contact with the piezoelectric sensor in the cord-like shape when the toilet seat apparatus is seated.

21. The toilet seat apparatus according to Claim 20, wherein the pressing means is a projection projected from an inner face of the case to the piezoelectric sensor in the cord-like face arranged in the case.

22. The toilet seat apparatus according to Claim 21, wherein the projection is constituted by a pad for absorbing an impact attached to a lower face of the base plate and brought into elastic contact with an upper face of a toilet main body and the pad is provided to be able to be brought into contact with the piezoelectric sensor in the cord-like shape by penetrating a through hole of the base plate.

23. The toilet seat apparatus according to Claim 21, wherein the piezoelectric sensor in the cord-like shape is supported in a state of being separated from the inner face of the case and the projections are arranged alternately to the upper lid and the base plate along a cable longitudinal direction.

24. The toilet seat apparatus according to Claim 20, wherein an outer face of the upper lid is recessed with a peripheral groove, the pressing means is formed by an elastic body fitted into the peripheral groove and the piezoelectric sensor in the cord-like shape is arranged to be contained in the elastic body.

25. The toilet seat apparatus according to any one of Claims 19 through 24, wherein the electric signal is used for controlling a temperature of hot water of cleaning means, a water pressure, a temperature of a heater in a toilet seat, or detecting a heart rate or the like.

26. The toilet seat apparatus according to Claim 25, wherein the electric signal is used by being outputted to an outside monitor via communicating means.
